# EUROPEAN PATENT SPECIFICATION

(11) **EP 4 596 318 B1**
(45) Date of publication and mention of the grant of the patent: **28.10.2026**
(21) Application number: 23872490.0
(22) Date of filing: 27.09.2023
(51) Int. Cl.: B60N 2/90, A47C 7/62, A47C 31/12, A61B 5/103, A61B 5/00, B60N 2/00, B60N 2/02, B60N 2/66, A61B 5/11

(54) **CONTROL UNIT, POSTURE IMPROVEMENT ASSISTANCE DEVICE, AND CONTROL METHOD**
STEUEREINHEIT, HALTUNGSVERBESSERUNGSUNTERSTÜTZUNGSVORRICHTUNG UND STEUERUNGSVERFAHREN
UNITÉ DE COMMANDE, DISPOSITIF D'AIDE À L'AMÉLIORATION DE POSTURE ET PROCÉDÉ DE COMMANDE

(30) Priority: 28.09.2022 US 202263377372 P; 21.10.2022 US 202263418161 P; 04.11.2022 US 202263422531 P; 29.03.2023 JP 2023053981; 05.09.2023 JP 2023143846; 22.09.2023 JP 2023158403
(43) Date of publication of application: 06.08.2025
(73) Proprietor: TS Tech Co., Ltd., Asaka-shi Saitama 351-0012 (JP)
(72) Inventor: MIZOI, Kensuke, Shioya-gun, Tochigi 329-1217 (JP); YAMAUCHI, Naoto, Shioya-gun, Tochigi 329-1217 (JP); KASHINO, Ryuta, Shioya-gun, Tochigi 329-1217 (JP)
(74) Representative: Grünecker Patent- und Rechtsanwälte PartG mbB
(86) International application number: PCT/JP2023/035306
(87) International publication number: WO 2024/071268

(56) References cited:
- JP-A- 2017 081 194
- JP-A- 2019 077 220
- JP-A- 2019 077 220
- JP-A- 2020 066 404
- JP-A- 2020 066 404
- US-B1- 9 795 322

## Description

### TECHNICAL FIELD

The present invention relates to a control unit, a posture improvement assistance device, and a control method, particularly to a control unit, a posture improvement assistance device, and a control method that performs a notification according to the posture of a seated occupant.

### BACKGROUND ART

Conventionally, a posture notification device that detects the posture or the like of a seated occupant and that outputs a warning to the seated occupant has been known. Specifically, the posture of the seated occupant is detected, and when it is determined that the detection result is not appropriate (for example, the seated occupant is in a hunched posture with a large curve from the back to the lower back), an alarm is output. Accordingly, the seated occupant can correct her or his own posture to an appropriate state, and can improve the seating posture.

PATENT LITERATURE 1 discloses a technique for determining the posture of a seated occupant using pressure sensors. Specifically, a plurality of the pressure sensors are disposed inside a seat, and the distribution of body pressure applied to the seat is obtained based on pressure signals output by the pressure sensors. Then, it is described that by comparing the obtained distribution of body pressure with an appropriate pattern prepared in advance, it is determined whether the posture of the seated occupant is appropriate, and when it is determined that the posture is not appropriate, an alarm is output. Further relevant prior art is described in US 9 795 322 B1 which discloses the preamble of claims 1 and 6, JP 2020 066404 A and JP 2019 077220.

### CITATION LIST

### PATENT LITERATURE

PATENT LITERATURE 1: JP S62-178449 A

### SUMMARY OF INVENTION

### TECHNICAL PROBLEM

According to the technique described in PATENT LITERATURE 1, it can be detected whether the posture of the seated occupant is not appropriate, based on the pressure signals output by the pressure sensors. Therefore, when the seated occupant becomes fatigued and the seating posture collapses, the collapse of the seating posture can be detected, and a warning can be output to a driver. However, the seated occupant cannot know what her or his own posture is. For this reason, it is not possible to specifically determine how to change her or his posture to correct the posture.

The present invention has been made in view of the above-described problems, and an object of the present invention is to provide a control unit, a posture improvement assistance device, and a control method of the posture improvement assistance device that can notify a seated occupant of the posture of the seated occupant.

### SOLUTION TO PROBLEM

In particular, the aforementioned problems are solved by a control unit having the features defined in claim 1 and a control method having the features defined in claim 6, as well as a posture improvement assistance device comprising the inventive control unit, which device has the features defined in claim 5. Further preferred embodiments are defined in the dependent claims.

According to the above-described configuration, the pressure signals output by the plurality of pressure sensors built into the chair are input into the predetermined calculation equation to calculate the lumbar curve value that is an index related to the posture of the seated occupant, and the posture of the seated occupant is determined based on the lumbar curve value. In such a manner, since the posture of the seated occupant is determined based on the lumbar curve value that is an index related to the posture of the seated occupant, it can be quantitatively and easily determined whether the posture of the seated occupant is appropriate. In addition, the output control unit controls the display device to output the determination result of the posture of the seated occupant determined based on the lumbar curve value, as a schematic human body diagram. Therefore, the seated occupant can be notified of the posture of the seated occupant, and the seated occupant can visually perceive her or his own posture.

In addition, it is preferable that the determination unit determines whether the posture of the seated occupant is at least one of a hunched posture, a standard posture, and an arched posture, based on the lumbar curve value, and it is preferable that the output control unit controls the display device to output a text indicating whether the posture of the seated occupant is the hunched posture, the standard posture, or the arched posture, together with the schematic human body diagram.

According to the above-described configuration, whether the posture of the seated occupant is at least one of the hunched posture, the arched posture, and the standard posture is output in text. For this reason, the seated occupant can accurately perceive how to change her or his posture to correct the posture.

In addition, it is preferable that the output control unit controls the display device to output a time-series graph showing a change over time in the lumbar curve value.

According to the above-described configuration, since the time-series graph of the lumbar curve value is output to the display device, the seated occupant can correct her or his posture while checking the change over time in the lumbar curve value.

In accordance with the present invention, the control unit further includes a moveable body control unit. In addition, it is preferable that the control unit further includes a movable body control unit that controls the movable bodies to be displaced. It is preferable that the calculation unit calculates a lumbar curve value variation amount that is a statistical variation amount of the calculated lumbar curve value, it is preferable that the determination unit determines a lower back pain level of the seated occupant based on the lumbar curve value variation amount calculated by the calculation unit, and it is preferable that the movable body control unit controls the movable bodies to be periodically displaced based on a determination result of the lower back pain level by the determination unit.

Thus, in accordance with the present invention, the lower back pain level of the seated occupant is determined based on the lumbar curve value variation amount, and the movable bodies are controlled to be periodically displaced based on the lower back pain level. Therefore, when the lumbar curve value variation amount is large, namely, when the posture of the lumbar is unstable, it can be determined that the seated occupant suffers from lower back pain. Then, when it is determined that the seated occupant suffers from lower back pain, the movable bodies are displaced to apply stimuli to the lumbar, so that the lower back pain of the seated occupant can be relieved.

In addition, it is preferable that the control unit further includes a setting reception unit that receives an input of a set value related to a physique of the seated occupant. It is preferable that the calculation unit calculates the lumbar curve value by inputting the pressure signals and the set value into the predetermined calculation equation.

According to the above-described configuration, the input of the set value related to the physique of the seated occupant is received, and the lumbar curve value is calculated based on the set value. Therefore, an appropriate lumbar curve value can be calculated according to the physique of the seated occupant.

In addition, it is preferable that a posture improvement assistance device includes the control unit; and the chair into which the plurality of movable bodies provided with the pressure sensors are built.

According to the above-described configuration, the seated occupant seated in the chair can be notified of the posture of the seated occupant, and the seated occupant can visually perceive her or his own posture.

In addition, the above-described problems are solved by a control method of the present invention for determining a posture of a seated occupant seated in a chair and controlling a display device to output a determination result, the control method including a computer executing: an acquisition step of acquiring pressure signals output by pressure sensors provided in a plurality of respective movable bodies built into the chair; a calculation step of calculating a lumbar curve value, which is an index related to the posture of the seated occupant, by inputting a plurality of the pressure signals acquired in the acquisition step into a predetermined calculation equation; a determination step of determining the posture of the seated occupant based on the lumbar curve value calculated in the calculation step; and an output control step of controlling the display device to output a schematic human body diagram corresponding to a determination result of the determination step.

According to the above-described configuration, the pressure signals output by the plurality of pressure sensors built into the chair are input into the predetermined calculation equation to calculate the lumbar curve value that is an index related to the posture of the seated occupant, and the posture of the seated occupant is determined based on the lumbar curve value. In such a manner, since the posture of the seated occupant is determined based on the lumbar curve value that is an index related to the posture of the seated occupant, it can be quantitatively and easily determined whether the posture of the seated occupant is appropriate. In addition, the output control unit controls the display device to output the determination result of the posture of the seated occupant determined based on the lumbar curve value, as a schematic human body diagram. Therefore, the seated occupant can be notified of the posture of the seated occupant, and the seated occupant can visually perceive her or his own posture.

### ADVANTAGEOUS EFFECTS OF INVENTION

According to the control unit, the posture improvement assistance device, and the control method of the present invention, the seated occupant can be notified of the posture, and the seated occupant can visually perceive her or his own posture.

In addition, the seated occupant can accurately perceive how to change her or his posture to correct the posture.

In addition, the seated occupant can correct her or his posture while checking a change over time in the lumbar curve value.

In addition, when it is determined that the seated occupant suffers from lower back pain, the movable bodies are displaced to apply stimuli to the lumbar, so that the lower back pain of the seated occupant can be relieved.

In addition, an appropriate lumbar curve value can be calculated according to the physique of the seated occupant.

### BRIEF DESCRIPTION OF DRAWINGS

[FIG. 1] FIG. 1 is a view for describing an overview of a posture improvement assistance device.
[FIG. 2] FIG. 2 is a view schematically showing a spine and a pelvis in a standard posture pattern.
[FIG. 3] FIG. 3 is a view schematically showing the spine and the pelvis in a hunched posture pattern.
[FIG. 4] FIG. 4 is a view schematically showing the spine and the pelvis in an arched posture pattern.
[FIG. 5] FIG. 5 is a view showing a functional configuration of the posture improvement assistance device.
[FIG. 6] FIG. 6 is a view showing the flow of a health mode process.
[FIG. 7] FIG. 7 is a view showing the flow of a posture determination process.
[FIG. 8] FIG. 8 is a view showing the flow of a posture display process.
[FIG. 9] FIG. 9 is a view showing a posture display screen.
[FIG. 10] FIG. 10 is a view showing the flow of a kneading process.
[FIG. 11] FIG. 11 is a view showing a kneading execution screen.
[FIG. 12] FIG. 12 is a perspective view of a back frame of a vehicle seat according to a second example when viewed from the front.
[FIG. 13] FIG. 13 is a rear view of a pressure-receiving member according to the second example.
[FIG. 14] FIG. 14 is a rear view of a pressure-receiving member according to a modification example.
[FIG. 15] FIG. 15 is a front view of a back frame of a vehicle seat according to a third example.
[FIG. 16] FIG. 16 is a rear view of the back frame of the vehicle seat according to the third example.
[FIG. 17] FIG. 17 is a perspective view of a back frame of a vehicle seat according to a fourth example when viewed from the rear.
[FIG. 18] FIG. 18 is a cross-sectional view taken along line A-A of FIG. 17.
[FIG. 19] FIG. 19 is a cross-sectional view taken along line A-A of FIG. 17, and is a view showing a modification example of the attachment position of a control unit.
[FIG. 20] FIG. 20 is a perspective view of the back frame of the vehicle seat according to the fourth example when viewed from the front, and is a view showing a second modification example of the attachment position of the control unit.
[FIG. 21] FIG. 21 is a perspective view of the back frame of the vehicle seat according to the fourth example when viewed from the front, and is a view showing a third modification example of the attachment position of the control unit.
[FIG. 22] FIG. 22 is a side view of a vehicle according to a second embodiment, and is a view showing a state where a sliding door is open.
[FIG. 23] FIG. 23 is a cross-sectional view taken along line A-A of FIG. 22, and is a view showing an interior configuration of the vehicle.
[FIG. 24] FIG. 24 is a view showing a state where right and left sliding doors are moved open and a vehicle seat is moved upward.
[FIG. 25] FIG. 25 is a view showing a state where an airbag provided at a center portion of a roof is deployed.
[FIG. 26] FIG. 26 is a cross-sectional view taken along line B-B of FIG. 22, and is a view showing an interior configuration of the vehicle.
[FIG. 27] FIG. 27 is a view showing a state where an instrument panel is retracted.
[FIG. 28] FIG. 28 is a view schematically showing a structure of the instrument panel.
[FIG. 29] FIG. 29 is a view showing a state where a footrest is pulled out from the instrument panel.
[FIG. 30A] FIG. 30A is a view schematically showing a pull-out structure of the footrest.
[FIG. 30B] FIG. 30B is a view showing another example of a pull-out structure of the footrest.
[FIG. 31] FIG. 31 is a cross-sectional view showing an interior of the vehicle when the instrument panel is moved in an upward direction.
[FIG. 32] FIG. 32 is a view showing an interior configuration of a vehicle in which a center console is provided.
[FIG. 33] FIG. 33 is a descriptive view describing a procedure for supporting an occupant using air cells provided in side supports when the vehicle seat is rotated.
[FIG. 34] FIG. 34 is a descriptive view for describing a procedure for supporting the occupant using air cells provided in armrests when the vehicle seat is rotated.
[FIG. 35] FIG. 35 is a descriptive view describing a procedure for supporting the occupant using the armrests when the vehicle seat is rotated.
[FIG. 36] FIG. 36 is a view schematically showing a configuration of a vehicle according to a first example of a third embodiment, and is a cross-sectional view showing an interior configuration of the vehicle when viewed from the side.
[FIG. 37] FIG. 37 is a cross-sectional view showing a mat state created by storing seat backs.
[FIG. 38] FIG. 38 is a cross-sectional view taken along line A1-A1 of FIG. 36, and is a view showing an interior configuration of the vehicle when viewed from above.
[FIG. 39] FIG. 39 is a cross-sectional view taken along line A2-A2 of FIG. 36, and is a view showing an interior configuration of the vehicle when viewed from the front.
[FIG. 40] FIG. 40 is a view showing another example of the instrument panel.
[FIG. 41] FIG. 41 is a view showing another example of the vehicle, and is a cross-sectional view schematically showing a vehicle including rotating headrests on a ceiling.
[FIG. 42] FIG. 42 is a cross-sectional view taken along line A3-A3 of FIG. 41.
[FIG. 43] FIG. 43 is a view showing another example of the vehicle, and is a cross-sectional view showing a configuration of the vehicle when viewed from the side.
[FIG. 44] FIG. 44 is a cross-sectional view taken along line A4-A4 of FIG. 43.
[FIG. 45] FIG. 45 is a view showing another example of the vehicle, and is a cross-sectional view showing a configuration of the vehicle when viewed from the side.
[FIG. 46] FIG. 46 is a cross-sectional view taken along line A5-A5 of FIG. 45.
[FIG. 47] FIG. 47 is a cross-sectional view taken along line A6-A6 of FIG. 45.
[FIG. 48] FIG. 48 is a view schematically showing a configuration of a vehicle of a second example, and is a cross-sectional view when viewed from the side.
[FIG. 49] FIG. 49 is a cross-sectional view taken along line B1-B1 of FIG. 48.
[FIG. 50] FIG. 50 is a cross-sectional view taken along line B2-B2 of FIG. 48.
[FIG. 51] FIG. 51 is a view showing another example of the vehicle according to the second example, and is a cross-sectional view when viewed from the side.
[FIG. 52] FIG. 52 is a cross-sectional view taken along line B3-B3 of FIG. 51.
[FIG. 53] FIG. 53 is a view showing another example of the vehicle according to the second example, and is a cross-sectional view when viewed from the side.
[FIG. 54] FIG. 54 is a view schematically showing a configuration of a vehicle of a third example, and is a cross-sectional view when viewed from the side.
[FIG. 55] FIG. 55 is a cross-sectional view taken along line C1-C1 of FIG. 19.
[FIG. 56] FIG. 56 is a cross-sectional view taken along line C2-C2 of FIG. 19.
[FIG. 57] FIG. 57 is a view schematically showing a configuration of a vehicle of a fourth example, and is a cross-sectional view when viewed from the side.
[FIG. 58] FIG. 58 is a view showing a state where seats are arranged such that occupants can be seated facing each other.
[FIG. 59] FIG. 59 is a view showing another example of a seat arrangement.
[FIG. 60] FIG. 60 is a view showing another example of a seat arrangement.
[FIG. 61] FIG. 61 is a view showing a modification example of the vehicle of the fourth example, and is a cross-sectional view showing a configuration of a vehicle including three rows of seats.
[FIG. 62] FIG. 62 is a cross-sectional view taken along line D1-D1 of FIG. 61.
[FIG. 63] FIG. 63 is a cross-sectional view showing a state where seat backs are stored and seat cushions are set to a mat state.
[FIG. 64] FIG. 64 is a cross-sectional view taken along line D2-D2 of FIG. 63.
[FIG. 65] FIG. 65 is a view showing another example of the vehicle of the fourth example, and is a cross-sectional view showing a configuration of the vehicle when viewed from the side.
[FIG. 66] FIG. 66 is a view showing another example of a seat arrangement.
[FIG. 67] FIG. 67 is a view showing another example of a seat arrangement.
[FIG. 68A] FIG. 68A is a view schematically showing a vehicle seat installed in a vehicle of a fifth example, and is a side view of the seat with improved holding force for luggage and the like.
[FIG. 68B] FIG. 68B is a view showing another example of the vehicle seat.
[FIG. 68C] FIG. 68C is a view showing another example of the vehicle seat.
[FIG. 68D] FIG. 68D is a view showing another example of the vehicle seat.
[FIG. 68E] FIG. 68E is a view showing another example of the vehicle seat.
[FIG. 68F] FIG. 68F is a view showing another example of the vehicle seat.
[FIG. 68G] FIG. 68G is a view showing another example of the vehicle seat.
[FIG. 68H] FIG. 68H is a view showing another example of the vehicle seat.
[FIG. 68I] FIG. 68I is a view showing another example of the vehicle seat.
[FIG. 68J] FIG. 68J is a view showing another example of the vehicle seat, and is a view when viewed from above.
[FIG. 69] FIG. 69 is a cross-sectional view showing a vehicle in which a luggage net is provided on the instrument panel.
[FIG. 70] FIG. 70 is a view schematically showing a configuration of a vehicle of a sixth example, and is a view of the vehicle when viewed from the side.
[FIG. 71] FIG. 71 is a cross-sectional view taken along line E1-E1 of FIG. 70.
[FIG. 72] FIG. 72 is a view showing an example of a seat arrangement, and is a view of the vehicle when viewed from the side.
[FIG. 73] FIG. 73 is a view showing an example of a seat arrangement, and is a view of the vehicle when viewed from above.
[FIG. 74] FIG. 74 is a view showing a configuration of a vehicle of a seventh example, and is a view of the vehicle when viewed from the side.
[FIG. 75] FIG. 75 is a view showing a configuration of a vehicle of an eighth example, and is a view of the vehicle when viewed from above.
[FIG. 76] FIG. 76 is a view showing an example of a seat arrangement, and is a view of the vehicle when viewed from above.
[FIG. 77] FIG. 77 is a cross-sectional view taken along line F1-F1 of FIG. 76, and is a view of the vehicle when viewed from the front.

### DESCRIPTION OF EMBODIMENTS

### <<First embodiment>>

Hereinafter, a posture improvement assistance device 10, a control unit 30, and a control method executed by the control unit 30 according to a first embodiment of the present invention will be described with reference to the drawings. However, the embodiment to be described below is provided for easy understanding of the present invention, and does not limit the present invention. However, the invention is defined by the appended claims.

In addition, in the following description, the "seat front to rear direction" is a front to rear direction of a vehicle seat S, and is a direction that coincides with a front to rear direction when viewed from a seated occupant seated in the vehicle seat S. The "vehicle front to rear direction" is a direction that coincides with a traveling direction of a vehicle when the vehicle travels. In addition, the "seat width direction" is a lateral width direction of the vehicle seat S, and is a direction that coincides with a right to left direction when viewed from the seated occupant seated in the vehicle seat S. In addition, the "up to down direction" is an up to down direction of the vehicle seat S, and is a direction that coincides with a vertical direction when the vehicle travels on a horizontal surface. In addition, the "outer side" being simply referred to indicates a side closer to the outside in a direction from a center of the vehicle seat S toward the outside, and the "inner side" being referred to means a side closer to the center in a direction from the outside to the center of the vehicle seat S.

### <Overview of posture improvement assistance device 10>

The posture improvement assistance device 10 is used to assist in improving a seating posture by determining the posture of the seated occupant seated in the vehicle seat S, and performing control to output a determination result to a mobile terminal MT used by the seated occupant. The posture improvement assistance device 10 includes the vehicle seat S and the control unit 30 as main configurations.

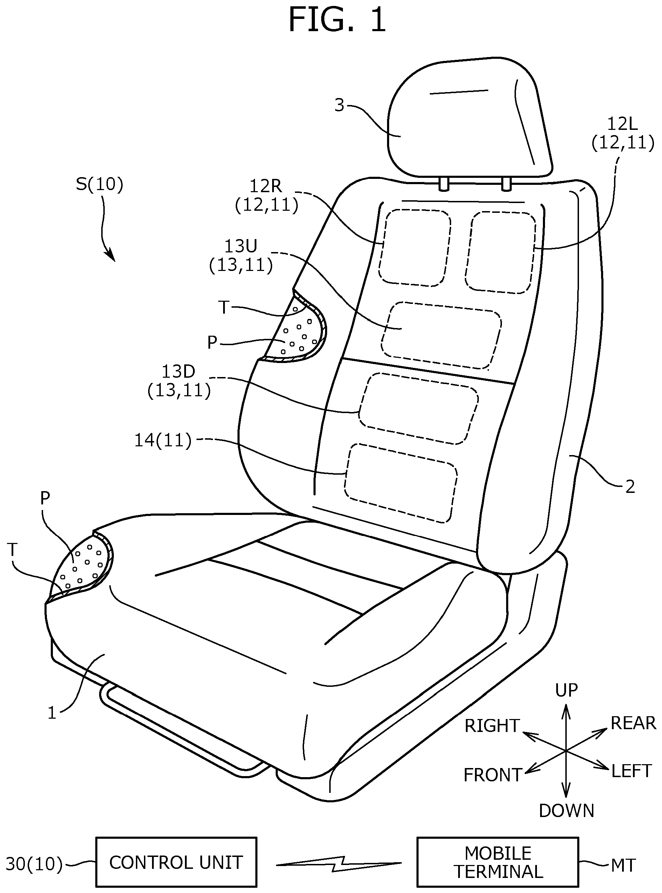

FIG. 1 is a perspective view of the vehicle seat S when viewed diagonally from the front. As shown in FIG. 1, the vehicle seat S includes a seat cushion 1, a seat back 2, and a headrest 3 as main configurations. In FIG. 1, for convenience of illustration, a part of the vehicle seat S is illustrated with a skin material T removed.

The seat cushion 1 serves as a seating portion that supports the buttocks of the seated occupant. The seat cushion 1 is configured by placing a pad material P on a seat cushion frame (not illustrated) constituting the skeleton of the seat cushion 1, and further covering the pad material P with the skin material T.

The seat back 2 serves as a backrest portion that supports the back of the seated occupant. The seat back 2 is configured by placing the pad material P on a seat back frame (not illustrated) constituting the skeleton of the seat back 2, and further covering the pad material P with the skin material T.

A plurality of air cells 11 are built into the seat back 2. The air cells 11 are fluid bags that inflate when compressed air is injected thereinto and, conversely, contract when the compressed air is discharged therefrom. The air cells 11 are built into the seat back 2, and the seat back 2 can be displaced by controlling the compressed air.

The air cells 11 include shoulder air cells 12 disposed at positions facing the shoulder blades of the seated occupant; a lumbar air cell 13 disposed at a position facing the lumbar; and a pelvis air cell 14 disposed at a position facing the pelvis. As will be described later, the air cells 11 repeat inflation and contraction periodically to apply stimuli to the shoulders, the lumbar, and the pelvis of the seated occupant, thereby applying stimuli to the seated occupant. In addition, the air cells 11 may inflate for the purpose of correcting the posture of the seated occupant.

The shoulder air cells 12 are located on an upper side of the seat back 2, and include a right shoulder air cell 12R disposed on a right side in the seat width direction, and a left shoulder air cell 12L disposed on a left side in the seat width direction. The trapezius muscles or levator scapulae muscles of the seated occupant can be massaged by inflating and contracting the shoulder air cells 12.

The lumbar air cell 13 is disposed below the shoulder air cells 12 and at the center in the seat width direction. The lumbar air cell 13 includes a first lumbar air cell 13U and a second lumbar air cell 13D located below the first lumbar air cell 13U. The latissimus dorsi muscles of the seated occupant can be stimulated by inflating and contracting the first lumbar air cell 13U and the second lumbar air cell 13D.

The pelvis air cell 14 is disposed below the lumbar air cell 13 and at the center in the seat width direction. The gluteus maximus muscles of the seated occupant can be massaged by inflating and contracting the pelvis air cell 14.

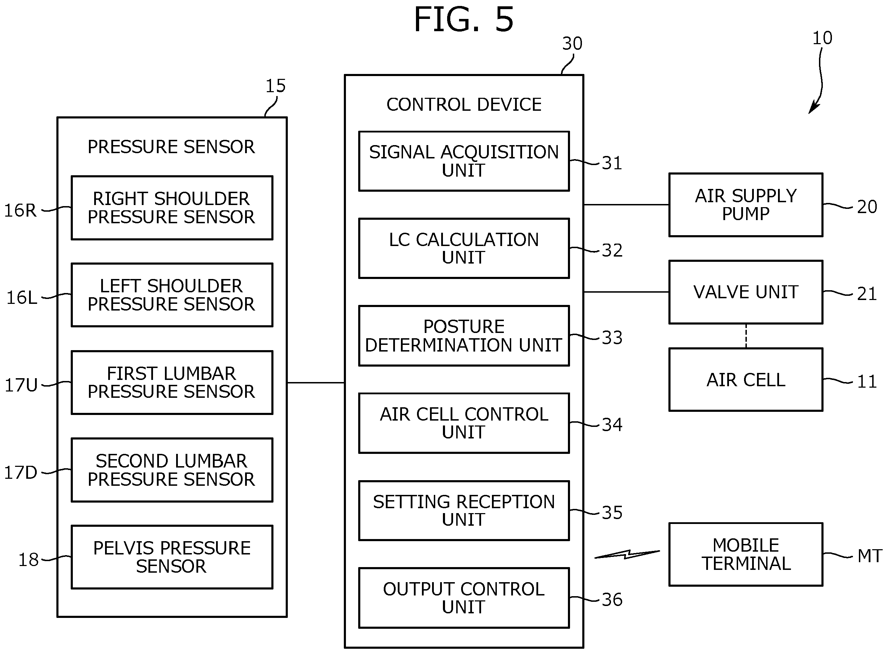

The air cells 11 are provided with pressure sensors 15 (refer to FIG. 5). The pressure sensors 15 are provided in the plurality of respective air cells 11 to detect the internal pressure of the air cells 11. In other words, the right shoulder air cell 12R and the left shoulder air cell 12L described above are provided with shoulder pressure sensors 16 (a right shoulder pressure sensor 16R and a left shoulder pressure sensor 16L). In addition, the first lumbar air cell 13U and the second lumbar air cell 13D are provided with lumbar pressure sensors 17 (a first lumbar pressure sensor 17U and a second lumbar pressure sensor 17D). In addition, the pelvis air cell 14 is provided with a pelvis pressure sensor 18. In such a manner, the pressure sensors 15 are disposed at positions facing the shoulders, the lumbar, and the pelvis of the seated occupant, and detect a load from the seated occupant.

The control unit 30 can determine a posture pattern of the seated occupant based on pressure signals output by the pressure sensors 15.

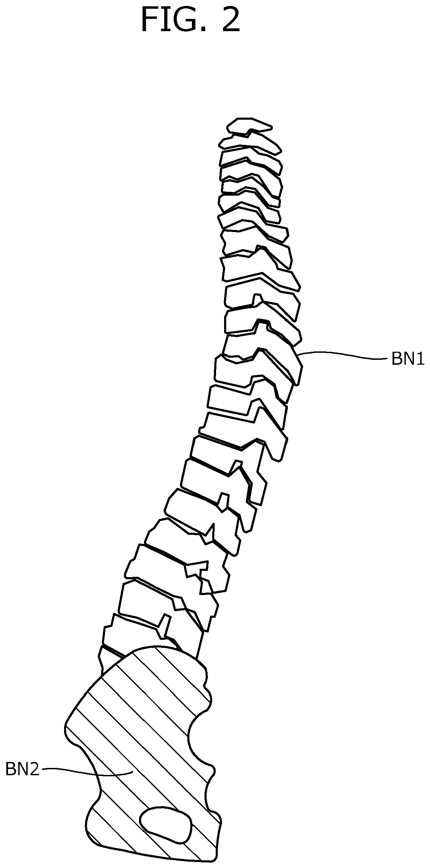

FIGS. 2 to 4 schematically show a spine BN1 and a pelvis BN2 that show posture patterns of seated occupants. More specifically, FIG. 2 shows the state of the standard spine BN1 and the standard pelvis BN2 of a person with a normal posture. As shown in FIG. 2, the standard spine BN1 has an S-shape that gently curves from top to bottom.

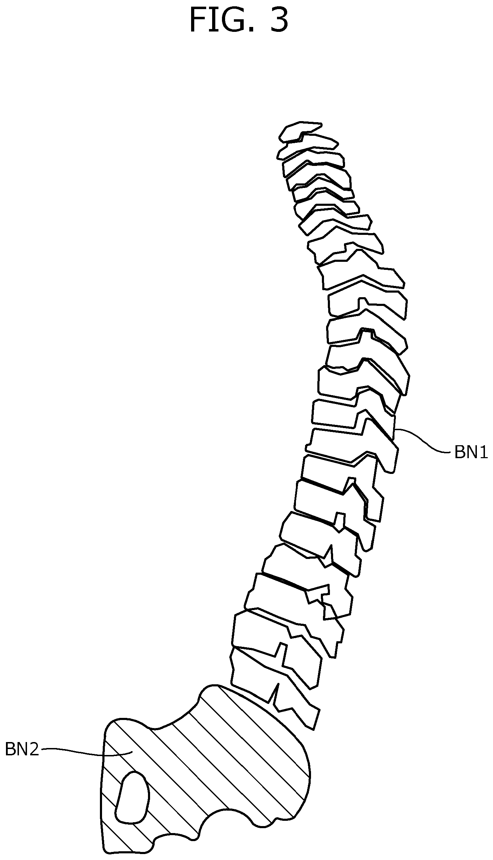

FIG. 3 shows the state of the spine BN1 and the pelvis BN2 of a person with a hunched posture. The hunched posture is also referred to as a round back posture, and the spine BN1 has an arched shape in which the back is rounded and bulges rearward.

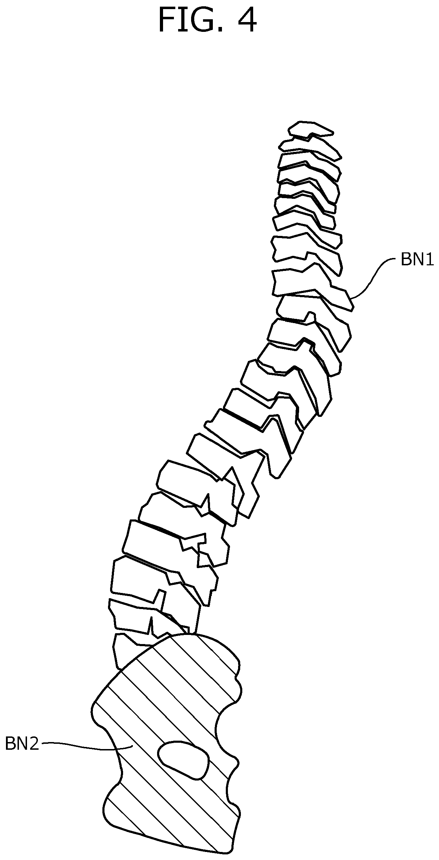

FIG. 4 shows the state of the spine BN1 and the pelvis BN2 of a person with an arched posture. The arched posture is also referred to as a swayback posture or a concave back posture, and has a shape in which the lumbar of the spine BN1 protrudes forward.

The control unit 30 controls the mobile terminal MT used by the seated occupant to display the determined posture of the seated occupant. Accordingly, the seated occupant can perceive her or his own posture, and recognize how to improve the posture. The control unit 30 is built into the seat cushion 1; however, the present invention is not limited thereto. The control unit 30 may be configured separately from the vehicle seat S and provided in the vehicle in which the vehicle seat S is installed.

Returning to FIG. 1, the headrest 3 is attached to an upper portion of the seat back 2 to support the head of the seated occupant. A headrest frame (not illustrated) forming the skeleton of the headrest 3 is provided inside the headrest 3.

A reclining device that is not illustrated in FIG. 1 rotatably couples the seat back 2 to the seat cushion 1. In addition, the reclining device can lock the seat back 2 in a state where the seat back 2 is inclined at a predetermined angle with respect to the seat cushion 1. Furthermore, the reclining device can recline the seat back 2 forward or rearward by releasing the lock of the seat back 2.

### <Regarding functional configurations of posture improvement assistance device 10>

Next, functional configurations of the posture improvement assistance device 10 will be described.

FIG. 5 is a diagram showing the functional configurations of the posture improvement assistance device 10. The posture improvement assistance device 10 includes, as the functional configurations, the control unit 30, the pressure sensors 15, an air supply pump 20, a valve unit 21, and the air cells 11. The control unit 30 is built into the vehicle seat S, and takes charge of control of the entirety of the vehicle seat S. In addition, the control unit 30 can perform wireless communicate with the mobile terminal MT used by the seated occupant, and output a notification signal related to the posture of the seated occupant to the mobile terminal MT.

As described above, the pressure sensors 15 are provided in the air cells 11, and detect the internal pressure of the air cells 11. In other words, the pressure sensors 15 detect loads from the shoulders, the lumbar, and the pelvis of the seated occupant. The pressure sensors 15 are provided inside the air cells 11; however, the present invention is not limited thereto. The pressure sensors 15 may be provided on the surfaces of the air cells 11 on the side facing the seated occupant. The pressure sensors 15 output the pressure signals to the control unit 30.

The air supply pump 20 and the valve unit 21 constitute a compressed air supply mechanism that supplies compressed air to the air cells 11. In more detail, the air supply pump 20 is a small air pump that generates compressed air. The valve unit 21 includes an electromagnetic valve and a discharge port for the compressed air, and controls the discharge of the compressed air by controlling the electromagnetic valve. The air cells 11 inflate when the compressed air is discharged from the valve unit 21, and contract when the compressed air is discharged from the air cells 11.

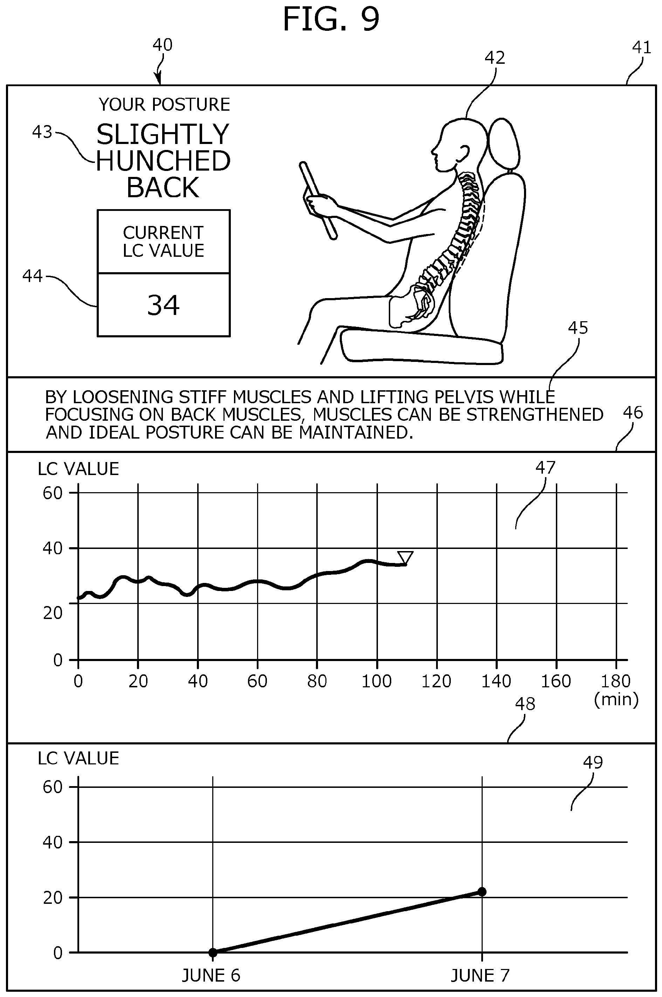

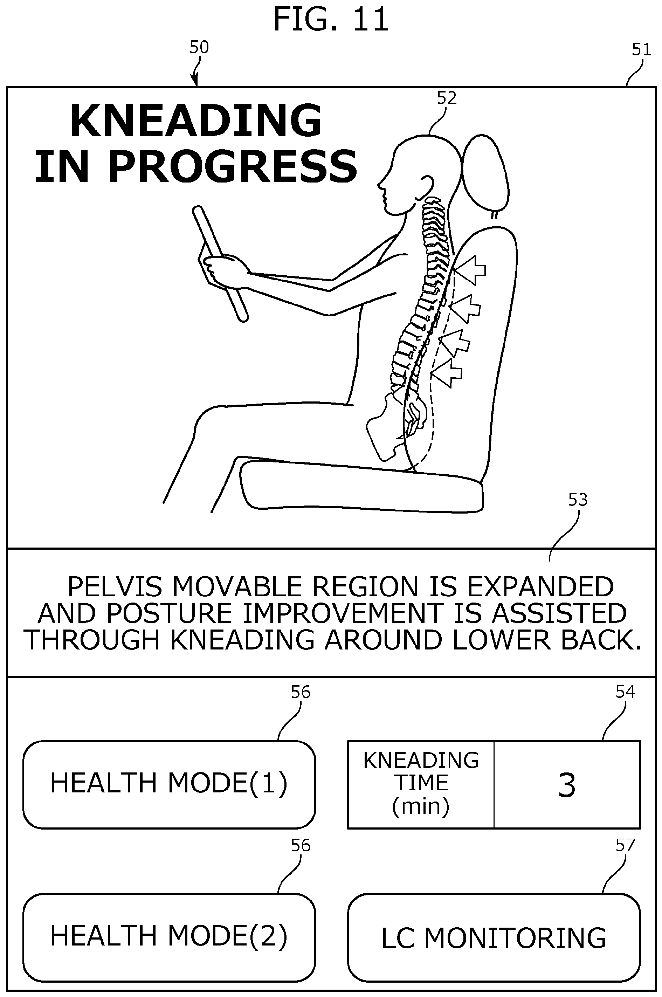

The mobile terminal MT is an information communication terminal including a display unit, and is a smartphone used by the seated occupant. The mobile terminal MT can output various setting information to a setting reception unit 35 of the control unit 30 to be described later upon an input operation by the seated occupant. In addition, the mobile terminal MT can display a posture display screen 40 to be described later with reference to FIG. 9, and a kneading execution screen 50 to be described later with reference to FIG. 11 on the display unit thereof. The mobile terminal MT may have the function of receiving an input operation by the seated occupant and the function of a display device, and may be a tablet terminal. In addition, the mobile terminal MT may be an information communication terminal installed in the vehicle seat S or the vehicle. The mobile terminal MT corresponds to a display device.

The control unit 30 is an electronic control unit (ECU) built into the seat cushion 1. A control circuit is incorporated into the ECU, and the ECU is composed of a processor that executes a program, a non-volatile storage medium, and a volatile storage medium. Furthermore, the processor functions as a signal acquisition unit 31, an LC calculation unit 32, a posture determination unit 33, an air cell control unit 34, the setting reception unit 35, and an output control unit 36 by loading and executing programs stored in the non-volatile storage medium. The processor corresponds to a computer.

The signal acquisition unit 31 includes a communication interface, and acquires the pressure signals (pressure values) output by the pressure sensors 15. More specifically, the signal acquisition unit 31 acquires pressure signals output by the right shoulder pressure sensor 16R, the left shoulder pressure sensor 16L, the first lumbar pressure sensor 17U, the second lumbar pressure sensor 17D, and the pelvis pressure sensor 18. The signal acquisition unit 31 may include an amplifier circuit that amplifies a pressure signal; an analog-to-digital converter (ADC) that converts an analog signal into a digital signal; and a filter circuit that removes noise components. The signal acquisition unit 31 corresponds to an acquisition unit.

The LC calculation unit 32 calculates a lumbar curve value by obtaining a plurality of the pressure signals (pressure values) acquired by the signal acquisition unit 31, and inputting the pressure values to a predetermined calculation equation. Here, the lumbar curve value is an index used to comprehensively evaluate the seating posture of the seated occupant based on the pressure signals from the shoulders, the lower back, and the pelvis. In other words, by comparing the LC value calculated by the LC calculation unit 32 with a threshold value determined in advance, it can be determined whether the posture of the seated occupant is a standard appropriate posture. In addition, by comparing the LC value with the threshold value determined in advance, it can be determined whether the posture of the seated occupant is a hunched back or an arched back. The lumbar curve value is also referred to as the LC value.

When the lumbar curve value is LC, the lumbar curve value can be calculated by the following Calculation Equation (1) . 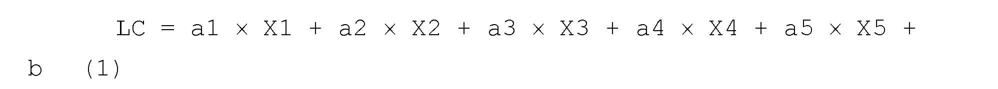 LC = a1 × X1 + a2 × X2 + a3 × X3 + a4 × X4 + a5 × X5 + b (1)

Here, X1, X2, X3, X4, and X5 are pressure values output by the right shoulder pressure sensor 16R, the left shoulder pressure sensor 16L, the first lumbar pressure sensor 17U, the second lumbar pressure sensor 17D, and the pelvis pressure sensor 18, respectively. In addition, a1, a2, a3, a4, a5, and b are coefficients that are set by a seating posture experiment performed on a plurality of subjects in advance. In such a manner, the lumbar curve value can be calculated by Calculation Equation (1) that is a linear first-order polynomial.

Here, the seating posture experiment will be described. In the seating posture experiment, first, posture information of a subject is acquired by an imaging device such as a camera or a motion sensor. Next, a doctor or an expert performs posture evaluation on the acquired posture information, and assigns a lumbar curve value thereto. Subsequently, the subject is seated in an experimental seat into which the pressure sensors 15 are built, and actual measurement values consisting of pressure signals are acquired. The expert can analyze the correlation between the lumbar curve values and the actually measured pressure signals by repeatedly performing the posture evaluation and the acquisition of the actual measurement values described above on the plurality of subjects. Specifically, the coefficients a1, a2, a3, a4, a5, and b can be obtained by performing multiple regression analysis on the lumbar curve values and the actual measurement values.

The equation for calculating the lumbar curve value is not limited to Calculation Equation (1). The lumbar curve value may be calculated based on set values received by the setting reception unit 35 to be described later and the pressure signals may be calculated. At this time, the lumbar curve value can be calculated by the following Calculation Equation (2). 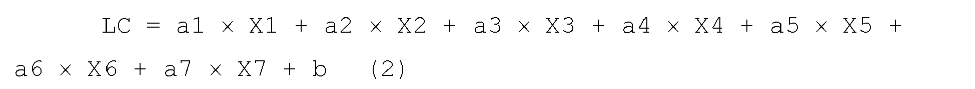 LC = a1 × X1 + a2 × X2 + a3 × X3 + a4 × X4 + a5 × X5 + a6 × X6 + a7 × X7 + b (2)

Here, X6 and X7 are set values related to the physique of the seated occupant, and are the body weight and height of the seated occupant, respectively. In addition, a6 and a7 are coefficients that are set based on the above-described seating posture experiment.

In addition, the LC calculation unit 32 calculates an LC variation amount that is a statistical variation amount of the lumbar curve value. The LC variation amount is a standard deviation of the lumbar curve value when the lumbar curve value is continuously calculated over a predetermined measurement period. In addition, the LC variation amount may be a cumulative squared deviation between the lumbar curve value and an average value during the measurement period.

The measurement period is preferably five minutes. As will be described later, the LC variation amount is used by the posture determination unit 33 to determine a lower back pain level of the seated occupant. Therefore, when the measurement period is shorter than five minutes, the accuracy of determining the lower back pain level decreases. In addition, when the measurement time is longer than five minutes, the time it takes to obtain the determination result of the lower back pain level becomes too long, thereby causing the seated occupant to feel distrustful, which is a risk. However, the measurement period is not limited to five minutes, and may be set by the seated occupant. In addition, the measurement period may be automatically set based on a convergence condition for the LC variation amount. The LC variation amount corresponds to a lumbar curve value variation amount.

The posture determination unit 33 determines the posture of the seated occupant based on the lumbar curve value calculated by the LC calculation unit 32. More specifically, the posture determination unit 33 determines whether the posture of the seated occupant is one of a "hunched posture", a "slightly hunched posture", a "standard posture", a "slightly arched posture", and an "arched posture", based on the lumbar curve value. In addition, the posture determination unit 33 may determine whether the posture of the seated occupant is one of a "hunched posture", a "standard posture", and an "arched posture". The posture determination unit 33 determines the posture of the seated occupant by comparing the lumbar curve value with a predetermined threshold value determined in advance. The threshold value may be set by the seated occupant.

In addition, the posture determination unit 33 determines the lower back pain level of the seated occupant based on the LC variation amount calculated by the LC calculation unit 32. More specifically, the posture determination unit 33 determines whether the seated occupant is in an unstable posture state based on the LC variation amount. As will be described later, when the posture determination unit 33 determines that the seated occupant suffers from lower back pain, a kneading process in which the muscles in the lumbar of the seated occupant are stimulated by controlling the air cells 11 to periodically inflate and contract is performed. The posture determination unit 33 determines the lower back pain level by comparing the LC variation amount with the predetermined threshold value determined in advance. The threshold value may be set by the seated occupant.

The air cell control unit 34 controls the inflation and contraction of the air cells 11 by outputting control signals to the air supply pump 20 and the valve unit 21. When the posture determination unit 33 determines that the seated occupant suffers from lower back pain, the air cell control unit 34 controls the air cells 11 to inflate and contract in an inflation and contraction pattern determined in advance. Here, to describe a specific example of the inflation and contraction pattern, first, the air cells 11 are controlled to inflate for five seconds. Next, the air cells 11 are controlled to contract for five seconds. Then, the air cells 11 are repeatedly controlled to inflate for five seconds again. Such inflation and contraction control of the air cells 11 is repeated for one minute. Accordingly, the muscles in the lumbar of the seated occupant determined to suffer from lower back pain are massaged, so that the burden on the seated occupant can be reduced. The air cell control unit 34 corresponds to a movable body control unit.

The setting reception unit 35 receives input of the set values by the seated occupant. Specifically, the setting reception unit 35 receives input of set values related to the physique of the seated occupant, such as height and body weight. As described above, the LC calculation unit 32 calculates the lumbar curve value by inputting the input height and body weight and the pressure signals (pressure values) output by the pressure sensors 15 into Calculation Equation (2). In addition, the amount of compressed air injected into the air cells 11 may be able to be input to the setting reception unit 35, as a set value. Accordingly, the intensity of a stimulus applied to the muscles in the lumbar of the seated occupant can be appropriately changed in a massage process.

The output control unit 36 controls the mobile terminal MT to output the posture display screen 40 (refer to FIG. 9) on which a schematic human body diagram 42 corresponding to the determination result by the posture determination unit 33 is drawn. Specifically, the output control unit 36 controls the mobile terminal MT to output the posture display screen 40 through communication with the mobile terminal MT via a wireless communication interface (not illustrated) included in the vehicle seat S. As will be described in detail later, the posture display screen 40 displays a text string indicating whether the posture of the seated occupant is one of a "hunched posture", a "slightly hunched posture", a "standard posture", a "slightly arched posture", and an "arched posture", together with the schematic human body diagram 42 corresponding to the posture of the seated occupant. In addition, the posture display screen 40 displays a time-series graph 47 showing a change over time in the lumbar curve value.

In addition, the output control unit 36 controls the mobile terminal MT to output the kneading execution screen 50 (refer to FIG. 11) during execution of the kneading process to be described later.

### <Regarding flow of health mode process>

Next, the flow of a health mode process executed by the control unit 30 will be described. The health mode process is a process that is performed to encourage the seated occupant to improve the seating posture by determining the posture of the seated occupant based on the pressure signals output by the pressure sensors 15, and notifying the determined posture to the seated occupant. The health mode process is started by the control unit 30 when an operation to start the health mode process is performed via the mobile terminal MT used by the seated occupant; however, the present invention is not limited thereto. The health mode process may be started when a condition determined in advance is satisfied.

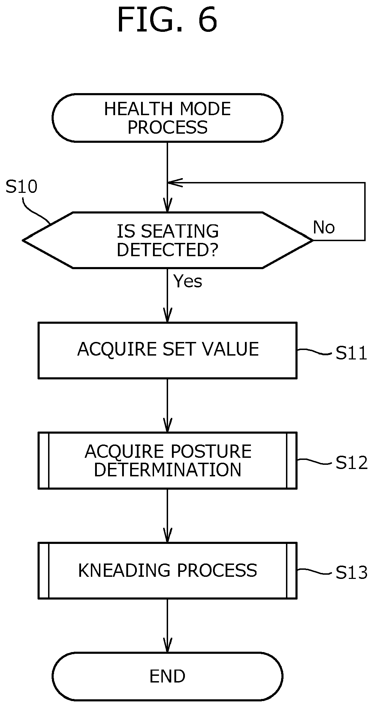

FIG. 6 shows the flow of the health mode process. As shown in FIG. 6, first, the control unit 30 detects a seated occupant by determining whether the seated occupant is seated (step S10). Specifically, the control unit 30 can detect the seated occupant by determining the presence or absence of a load from the seated occupant based on the pressure signals output by the pressure sensors 15. In addition, the control unit 30 may detect the seated occupant based on output signals of seating sensors (not illustrated) built into the vehicle seat S.

When it is determined that the seated occupant is not detected (step S10: No), the control unit 30 stands by until the seated occupant is detected.

On the other hand, when it is determined that the seated occupant is detected (step S10: Yes), the control unit 30 acquires set values (step S11). Specifically, the control unit 30 acquires, via a wireless communication line, set values that the seated occupant inputs by operating the mobile terminal MT used thereby. The set values include the height and body weight of the seated occupant.

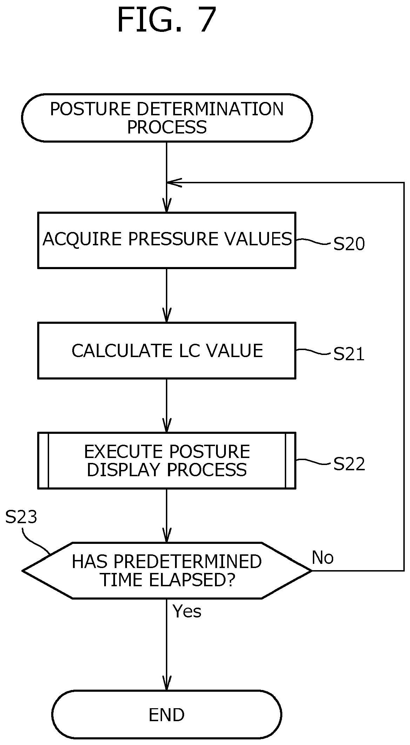

Next, the control unit 30 executes a posture determination process (step S12). FIG. 7 shows the flow of the posture determination process executed by the control unit 30. As shown in FIG. 7, first, the control unit 30 acquires the pressure signals output by the pressure sensors 15 (step S20, acquisition step). Specifically, the control unit 30 acquires pressure signals (pressure values) output by the right shoulder pressure sensor 16R, the left shoulder pressure sensor 16L, the first lumbar pressure sensor 17U, the second lumbar pressure sensor 17D, and the pelvis pressure sensor 18.

Next, the control unit 30 calculates a lumbar curve value (LC value) (step S21, calculation step). Specifically, the control unit 30 calculates the lumbar curve value by inputting the pressure signals (pressure values) acquired in step S20 and the set values acquired in step S11 into Calculation Equation (2) described above.

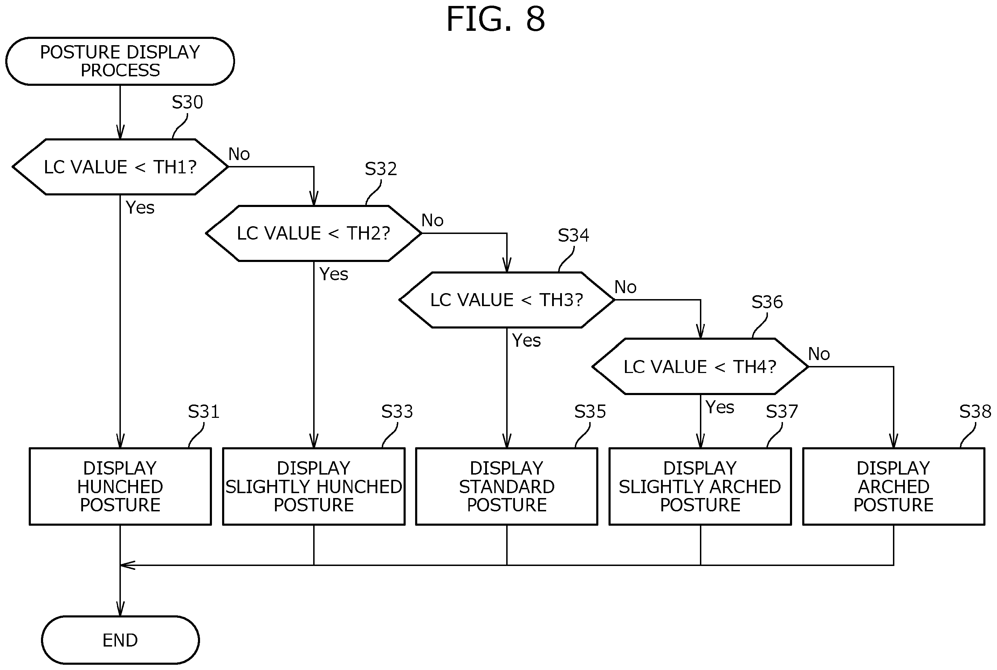

Next, the control unit 30 executes a posture display process (step S22). FIG. 8 shows the flow of the posture display process executed by the control unit 30. As shown in FIG. 8, first, the control unit 30 determines whether the seated occupant is in a hunched posture by comparing the lumbar curve value with a threshold value TH1 set in advance (step S30, determination step). When it is determined that the lumbar curve value is smaller than the threshold value TH1 (step S30: Yes), the control unit 30 determines that the seated occupant is in a hunched posture. Next, the control unit 30 causes the mobile terminal MT used by the seated occupant to display the schematic human body diagram 42 corresponding to a hunched back (step S31, output control step). More specifically, the control unit 30 controls the mobile terminal MT to output the posture display screen 40 on the display unit thereof by performing wireless communication with the mobile terminal MT.

FIG. 9 shows the posture display screen 40 that is output in real time on the display unit of the mobile terminal MT. As shown in FIG. 9, three display regions, namely, a posture display region 41, a graph display region 46, and a history display region 48 are formed in the posture display screen 40.

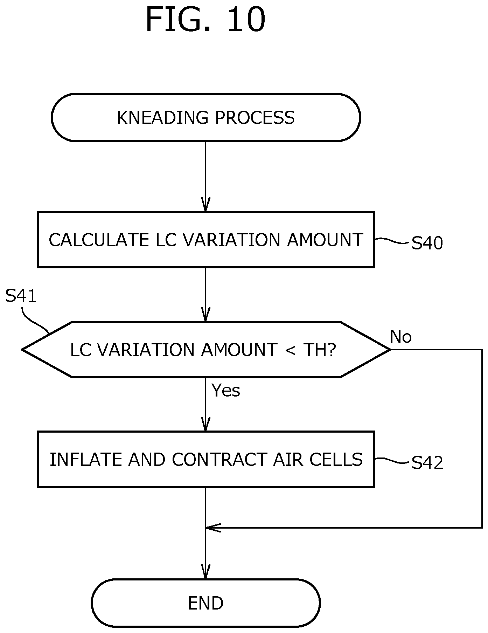

The schematic human body diagram 42 schematically showing the posture of the seated occupant, a posture specifying display 43 capable of specifying the posture of the seated occupant, a lumbar curve value display 44, and posture improvement assistance information 45 are output to the posture display region 41. FIG. 10 shows a screen example when it is determined that the seated occupant is in a "slightly hunched" posture as one example. A display corresponding to the determination result of the seating posture is output to the posture display region 41. Specifically, when it is determined that the seated occupant is in a "hunched posture", the schematic human body diagram 42 corresponding to the "hunched posture" and the posture specifying display 43 consisting of a text string "hunched back" are displayed. Similarly, when it is determined that the seated occupant is in a "standard posture", a "slightly arched posture", or an "arched posture", the schematic human body diagram 42 and the posture specifying display 43 corresponding to each posture are output. In addition, the posture improvement assistance information 45 for assisting the seated occupant in improving the posture is output below the schematic human body diagram 42. The seated occupant can visually perceive her or his own posture based on the schematic human body diagram 42 and the posture specifying display 43, and can effectively improve the seating posture based on the posture improvement assistance information 45.

The time-series graph 47 showing a change over time in the lumbar curve value is displayed in the graph display region 46. The time-series graph 47 is updated in real time by the output control unit 36 of the control unit 30. The seated occupant can recognize the change over time in the lumbar curve value by checking the time-series graph 47, and when the lumbar curve value varies greatly, the seated occupant can recognize that the state of the lower back is unstable and the seated occupant is highly likely to suffer from lower back pain.

Daily history information 49 indicating the history of lumbar curve values calculated in the past is displayed in the history display region 48. The seated occupant can recognize changes in his or her own posture and the tendency thereof based on the daily history information 49. Accordingly, when the lumbar curve value tends to change in a direction in which the lumbar curve value deviates from a desired numerical value, the seated occupant can be encouraged to improve the posture.

Returning to FIG. 8, when it is not determined that the lumbar curve value is smaller than the threshold value TH1 (step S30: No), the control unit 30 determines whether the seated occupant is in a slightly hunched posture by comparing the lumbar curve value with a threshold value TH2 (step S32). When it is determined that the lumbar curve value is smaller than the threshold value TH2 (step S32: Yes), the control unit 30 determines that the seated occupant is in a slightly hunched posture. Then, the control unit 30 causes the schematic human body diagram 42 and the posture specifying display 43 corresponding to the slightly hunched posture to be displayed on the posture display screen 40 of the mobile terminal MT (step S33), and ends the posture display process.

In addition, when it is not determined that the lumbar curve value is smaller than the threshold value TH2 (step S32: No), the control unit 30 determines whether the seated occupant is in a standard posture by comparing the lumbar curve value with a threshold value TH3 (step S34). When it is determined that the lumbar curve value is smaller than the threshold value TH3 (step S34: Yes), the control unit 30 determines that the seated occupant is in a standard posture. Then, the control unit 30 causes the schematic human body diagram 42 and the posture specifying display 43 corresponding to the standard posture to be displayed on the posture display screen 40 of the mobile terminal MT (step S35), and ends the posture display process.

In addition, when it is not determined that the lumbar curve value is smaller than the threshold value TH3 (step S34: No), the control unit 30 determines whether the seated occupant is in a slightly arched posture by comparing the lumbar curve value with a threshold value TH4 (step S36). When it is determined that the lumbar curve value is smaller than the threshold value TH4 (step S36: Yes), the control unit 30 determines that the seated occupant is in a slightly arched posture. Then, the control unit 30 causes the schematic human body diagram 42 and the posture specifying display 43 corresponding to the slightly arched posture to be displayed on the posture display screen 40 of the mobile terminal MT (step S37), and ends the posture display process.

In addition, when it is not determined that the lumbar curve value is smaller than the threshold value TH4 (step S36: No), the control unit 30 determines that the seated occupant is in an arched posture. Then, the control unit 30 causes the schematic human body diagram 42 and the posture specifying display 43 corresponding to the arched posture to be displayed on the posture display screen 40 of the mobile terminal MT (step S38), and ends the posture display process.

Returning to FIG. 7, subsequently to the posture display process, the control unit 30 determines whether the predetermined measurement time has elapsed (step S23). The measurement time is, for example, five minutes. When it is determined that the measurement time has not elapsed (step S23: No), the control unit 30 returns to step S20, and repeats step S20 to step 23. On the other hand, when it is determined that the measurement time has elapsed (step S23: Yes), the control unit 30 ends the posture determination process.

Returning to FIG. 6, the control unit 30 executes the kneading process (step S13). FIG. 10 shows the flow of the kneading process executed by the control unit 30. As shown in FIG. 10, first, the control unit 30 calculates an LC variation amount (step S40). Specifically, the control unit 30 calculates a cumulative squared deviation between the lumbar curve value and an average value.

Next, the control unit 30 compares the LC variation amount with a variation amount threshold value (step S41). Specifically, the control unit 30 determines whether the seated occupant suffers from lower back pain by comparing the LC variation amount with the variation amount threshold value determined in advance.

When it is determined that the LC variation amount is larger than the variation amount threshold value, the control unit 30 controls the air cells 11 to inflate and contract (step S42). Specifically, the control unit 30 acquires an inflation and contraction pattern for inflating and contracting the air cells 11, and controls the air supply pump 20 and the valve unit 21 based on the inflation and contraction pattern. Accordingly, the air cells 11 inflate and contract.

In addition, the control unit 30 causes the air cells 11 to inflate and contract, and outputs the kneading execution screen 50 on the display unit of the mobile terminal MT.

FIG. 11 shows the kneading execution screen 50 that is output on the display unit of the mobile terminal MT. As shown in FIG. 11, in the kneading execution screen 50, two display regions, namely, a kneading position display region 51 and a kneading state display region 54 are formed, and a mode selection button 56 and a display switching button 57 are provided.

A schematic kneading diagram 52 showing kneading positions by the air cells 11 and kneading auxiliary information 53 for auxiliary description of kneading contents are output to the kneading position display region 51. FIG. 11 shows a state where the lumbar of the seated occupant is kneaded. Since the schematic kneading diagram 52 and the kneading auxiliary information 53 are output to the kneading execution screen 50, the seated occupant can perceive what type of kneading is performed.

The remaining time of the kneading process is displayed in the kneading state display region 54. However, the kneading state display region 54 is not limited to displaying the remaining time of the kneading process, and may further display the elapsed time after the start of the kneading process.

The mode selection button 56 can be operated by the seated occupant to change the inflation and contraction pattern of the air cells 11. Accordingly, the seated occupant can cause the kneading process to be executed according to the inflation and contraction pattern of her or his own preference.

The display switching button 57 can be operated by the seated occupant to switch the output of the display unit of the mobile terminal MT to the posture display screen 40. Accordingly, the seated occupant can cause the kneading process to be executed while checking her or his own posture and the lumbar curve value on the posture display screen 40.

The posture improvement assistance device 10 configured as described above can notify the seated occupant of the posture of the seated occupant. Therefore, the seated occupant can perceive her or his own posture, and then accurately improve the posture.

### <Second example>

The configuration of the posture improvement assistance device 10 of the first example according to the first embodiment of the present invention has been described above; however, the above-described embodiment is merely one example for easy understanding of the present invention, and does not limit the present invention. However, the invention is defined by the appended claims.

In the above-described embodiment, the control unit 30 that takes charge of controlling the vehicle seat S has been described as being built into the seat cushion 1; however, the present invention is not limited thereto. The control unit 30 may be built into the seat back 2.

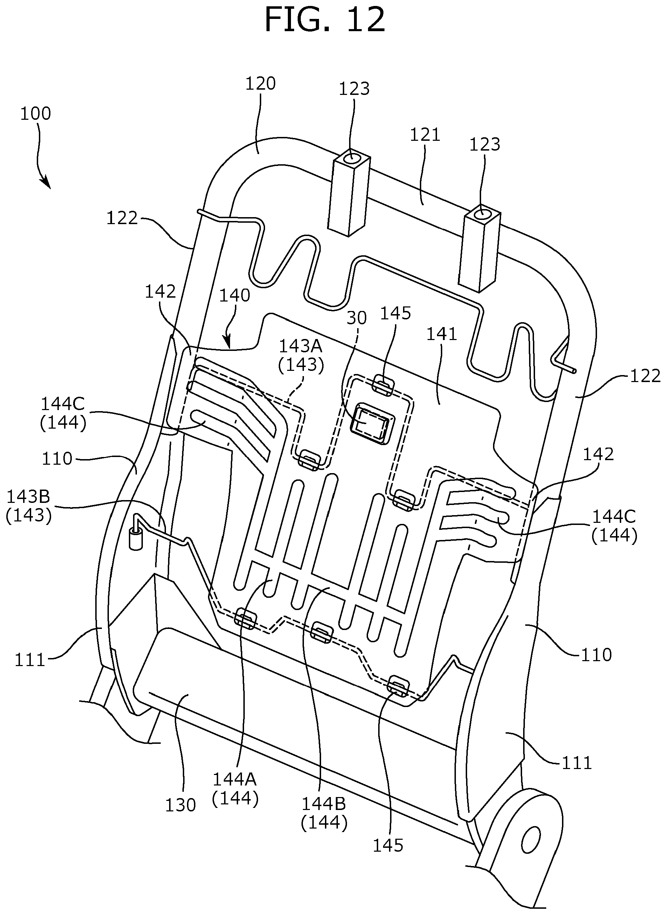

FIG. 12 is a perspective view of a back frame 100 of the vehicle seat S according to a second example when viewed from the front. As shown in FIG. 12, the back frame 100 is configured in a frame shape by right and left side frames 110 extending in the up to down direction and an upper frame 120 and a lower frame 130 that couple upper end portions and lower end portions of the right and left side frames 110, respectively. Furthermore, a pressure-receiving member 140 is disposed inside the back frame 100 having a frame shape.

The right and left side frames 110 include side frame plates 111 between the upper end portions coupled to the upper frame 120 and the lower end portions coupled to the lower frame 130.

The upper frame 120 is made of a pipe material bent in a substantially U-shape. The upper frame 120 includes a horizontal portion 121 extending horizontally at an upper end portion of the upper frame 120, and vertical portions 122 extending vertically downward from both ends of the horizontal portion 121. Pillar support portions 123 for supporting headrest pillars (not illustrated) of the headrest 3 are attached to the horizontal portion 121.

The pressure-receiving member 140 is a plate-shaped member made of resin, and is interposed between the right and left side frames 110. The pressure-receiving member 140 includes a pressure-receiving portion 141 that is located at the center in the seat width direction and that supports the back of the seated occupant, and support portions 142 located on the right and left of the pressure-receiving portion 141. The pressure-receiving member 140 is coupled to and supported by the right and left side frames 110 via coupling wires 143. In more detail, the pressure-receiving member 140 is configured to engage with an upper coupling wire 143A and a lower coupling wire 143B via engaging pawls 145, and to be able to receive a load from the back of the seated occupant while deforming rearward.

Bead portions 144 protruding rearward are formed in the pressure-receiving member 140. In more detail, first bead portions 144A extending in the up to down direction, a second bead portion 144B coupling the first bead portions 144A and extending in a horizontal direction, and third bead portions 144C inclined in the right to left direction are formed in the pressure-receiving member 140. The rigidity of the pressure-receiving member 140 in directions in which the beads extend can be increased by the first bead portions 144A, the second bead portion 144B, and the third bead portions 144C.

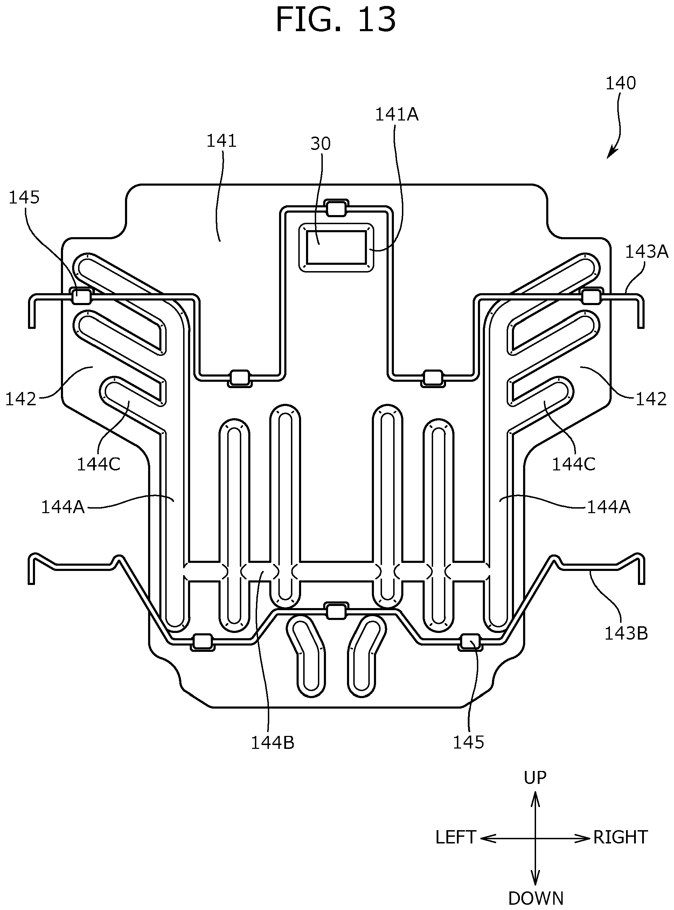

FIG. 13 is a rear view of the pressure-receiving member 140. A center recess 141A that curves from the rear toward the front is formed at an upper portion of the pressure-receiving member 140 at the center in the right to left direction. The control unit 30 is disposed on the back side of the pressure-receiving member 140 to be accommodated in the center recess 141A. By disposing the control unit 30 in the center recess 141A, the seated occupant can be seated in the vehicle seat S without feeling discomfort in the back. Incidentally, the valve unit 21 may be accommodated in the center recess 141A. By disposing the valve unit 21 in the center recess 141A, the seated occupant can be seated in the vehicle seat S without feeling discomfort in the back. In addition, compressed air can be efficiently delivered to the shoulder air cells 12, the lumbar air cell 13, and the pelvis air cell 14 built into the seat back 2.

### <Modification example>

In the above-described example, the control unit 30 has been described as being disposed to be accommodated in the center recess 141A located at the center of the pressure-receiving member 140 in the seat width direction; however, the present invention is not limited thereto.

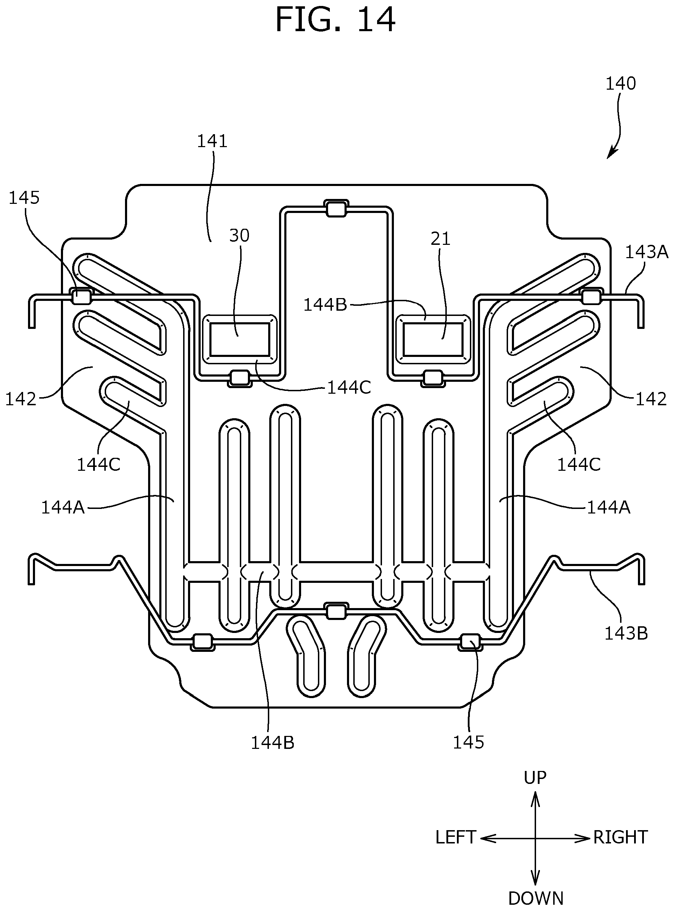

FIG. 14 is a rear view of the pressure-receiving member 140 according to a modification example. As shown in FIG. 14, a right recess 141B and a left recess 141C are formed on the right and left in the seat width direction. Therefore, the control unit 30 can be disposed in one of the right recess 141B and the left recess 141C, and the valve unit 21 can be disposed in the other. Accordingly, similarly to the above-described embodiment, the seated occupant can be seated in the vehicle seat S without feeling discomfort in the back. In addition, compressed air can be efficiently delivered to the shoulder air cells 12, the lumbar air cell 13, and the pelvis air cell 14 built into the seat back 2. Incidentally, electrical components other than the control unit 30 and the valve unit 21 may be disposed in the center recess 141A, the right recess 141B, and the left recess 141C.

### <Third example>

In the above-described example, the control unit 30 and the valve unit 21 have been described as being disposed to be accommodated in the recesses formed in the pressure-receiving member 140; however, the present invention is not limited thereto. It is preferable that the control unit 30 and the valve unit 21 are disposed at positions not overlapping the air cells 11 in the front to rear direction.

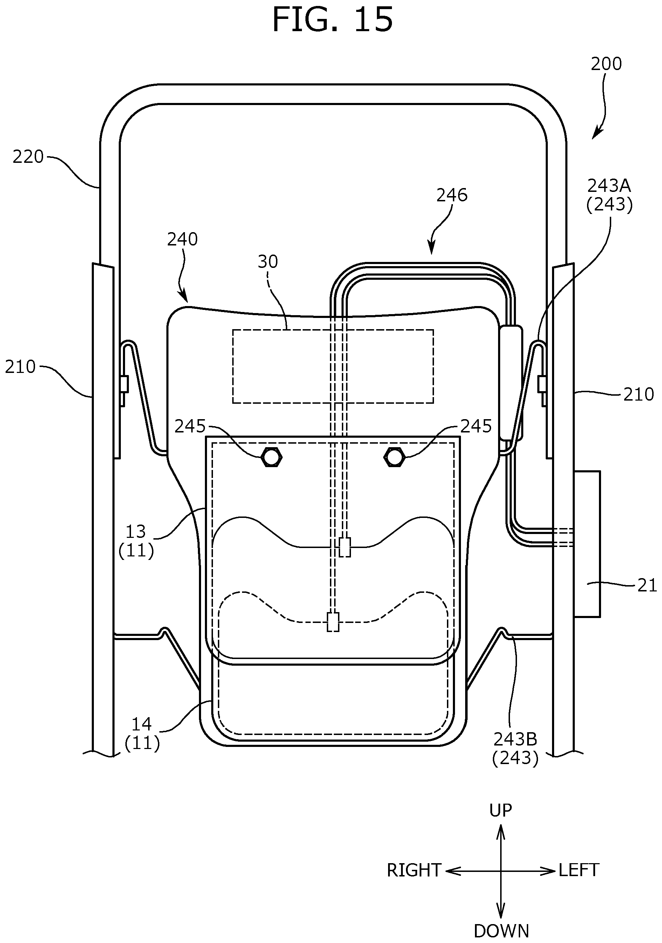

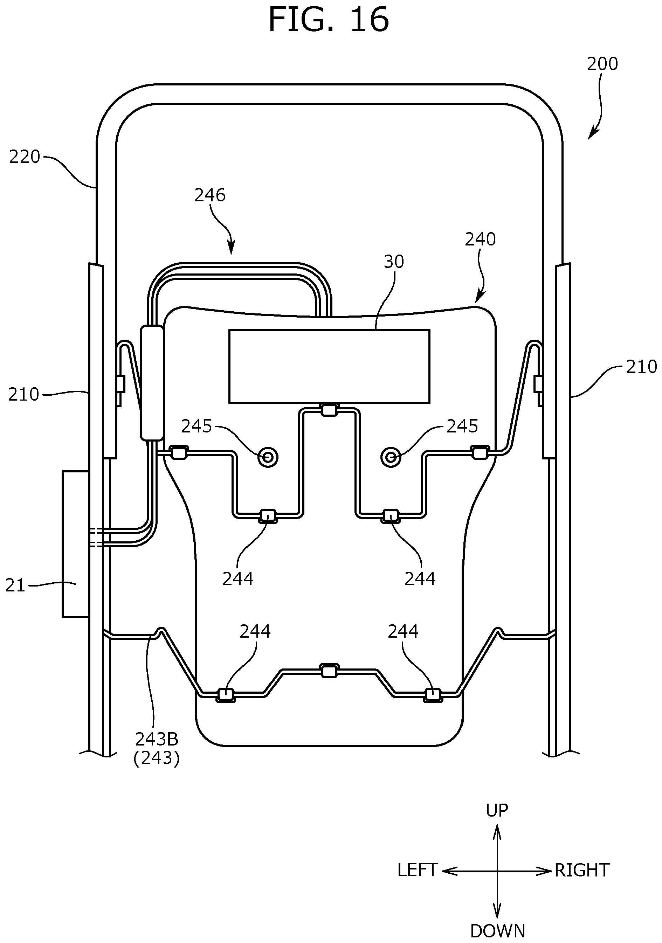

FIG. 15 is a front view of a back frame 200 of the vehicle seat S according to a third example of the first embodiment. In addition, FIG. 16 is a rear view of the back frame 200. As shown in FIGS. 15 and 16, the back frame 200 is configured in a frame shape by right and left side frames 210 extending in the up to down direction and an upper frame 220 and a lower frame (not illustrated) that couple upper end portions and lower end portions of the right and left side frames 210, respectively. Furthermore, a pressure-receiving member 240 to which the air cells 11 are attached is disposed inside the back frame 200 having a frame shape.

The pressure-receiving member 240 is a plate-shaped member made of resin, and is interposed between the right and left side frames 110. The pressure-receiving member 240 is coupled to and supported by the right and left side frames 210 via coupling wires 243. In more detail, the pressure-receiving member 240 is configured to engage with an upper coupling wire 243A and a lower coupling wire 243B via engaging pawls 244 (refer to FIG. 16), and to be able to receive a load from the back of the seated occupant while deforming rearward.

The lumbar air cell 13 and the pelvis air cell 14 are suspended and fixed to the pressure-receiving member 240 by air cell fasteners 245. In more detail, the lumbar air cell 13 and the pelvis air cell 14 are fixed to the pressure-receiving member 240 such that a lower portion of the lumbar air cell 13 and the pelvis air cell 14 overlap each other in the front to rear direction. Each of the air cell fasteners 245 is composed of a screw hole formed in the pressure-receiving member 240, and a screw inserted into the screw hole.

Tubes 246 for supplying compressed air from the valve unit 21 are connected to the lumbar air cell 13 and the pelvis air cell 14.

As shown in FIG. 16, the control unit 30 is disposed above the lumbar air cell 13. In other words, the control unit 30 is disposed on the pressure-receiving member 240 at a position not overlapping the air cells 11 in the front to rear direction. Accordingly, an increase in the size of a component in the front to rear direction, the component existing behind the back of the seated occupant, can be suppressed. In addition, even when the air cells 11 are inflated, the seated occupant can be seated in the vehicle seat S without feeling discomfort in the back.

### <Fourth example>

In the above-described example, the control unit 30 and the valve unit 21 have been described as being attached to the pressure-receiving member 140; however, the present invention is not limited thereto. The control unit 30 and the valve unit 21 may be attached to a back frame 300.

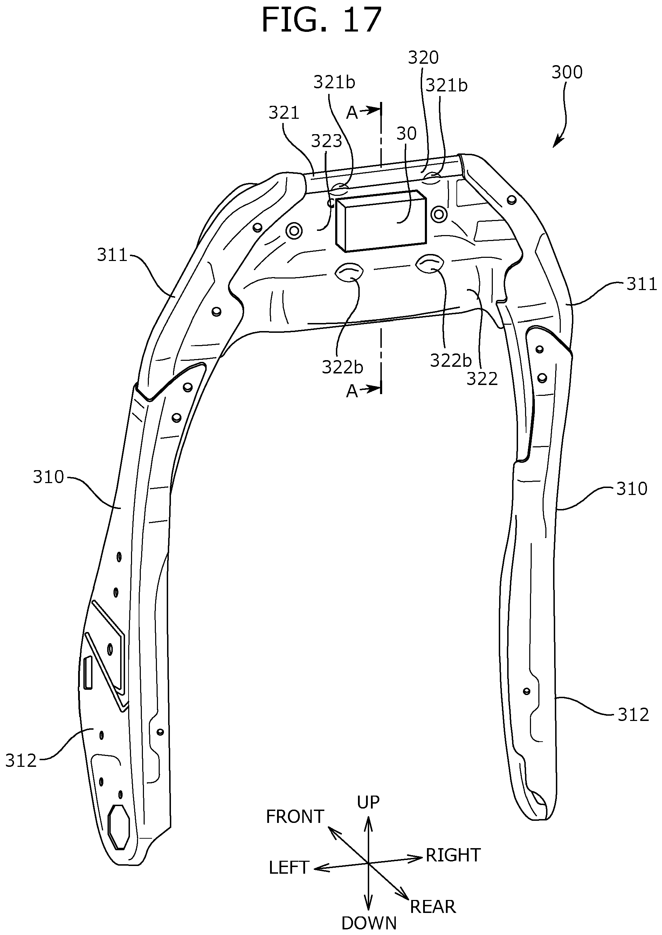

FIG. 17 is a perspective view of the back frame 300 of the vehicle seat S according to a fourth example of the first embodiment when viewed from the rear. As shown in FIG. 17, the back frame 300 is configured in a frame shape by right and left side frames 310 extending in the up to down direction and an upper frame 320 and a lower frame (not illustrated) that couple upper end portions and lower end portions of the right and left side frames 310, respectively.

Each of the side frames 310 is made of a metal plate, and is divided into two portions in the up to down direction. Specifically, each of the side frames 310 is configured by partially overlapping a first side frame piece 311 located above and a second side frame piece 312 located below.

The upper frame 320 is made of a metal plate, and both end portions of the upper frame 320 in the seat width direction are welded to respective upper end portions of the first side frame pieces 311.

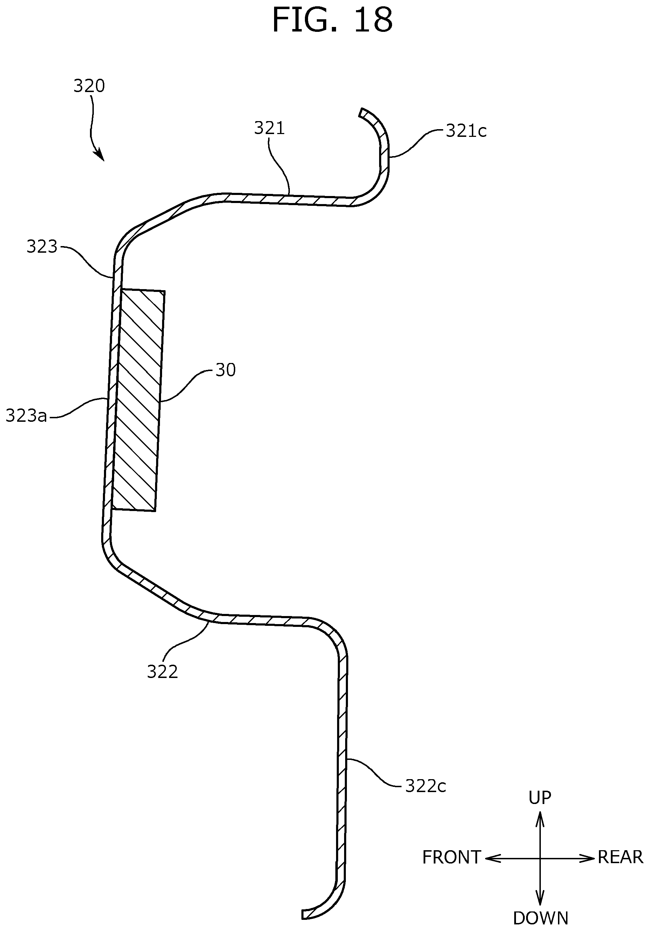

The upper frame 320 is bent into a substantially C-shape when viewed from the side. In detail, as shown in FIG. 18 that is a cross-sectional view taken along line A-A of FIG. 17, the upper frame 320 includes an upper wall portion 321; a lower wall portion 322 facing the upper wall portion 321; and a front wall portion 323 coupling the upper wall portion 321 and the lower wall portion 322.

An upper reinforcing portion 321b and a lower reinforcing portion 322b are formed in the upper wall portion 321 and the lower wall portion 322, respectively. The upper reinforcing portion 321b is formed by partially recessing the upper wall portion 321. Similarly, the lower reinforcing portion 322b is formed by partially recessing the lower wall portion 322. The number and disposition of the upper reinforcing portions 321b and the lower reinforcing portions 322b are not limited to the number and disposition shown in FIG. 17. The upper reinforcing portion 321b may be formed at a front end portion of the upper wall portion 321 at a boundary portion between the upper wall portion 321 and the front wall portion 323. Similarly, the lower reinforcing portion 322b may be formed at a front end portion of the lower wall portion 322 at a boundary portion between the lower wall portion 322 and the front wall portion 323.

As shown in FIG. 18, an upper bent portion 321c bent upward is formed at a rear end of the upper wall portion 321. In addition, a lower bent portion 322c bent downward is formed at a rear end of the lower wall portion 322.

The front wall portion 323 includes a center portion 323a consisting of a flat surface. The center portion 323a, the upper wall portion 321, and the lower wall portion 322 form an accommodation region that accommodates the control unit 30. In other words, the control unit 30 is attached to the upper frame 320 at the center in the seat width direction in a state where the control unit 30 is surrounded on three sides by the upper wall portion 321, the lower wall portion 322, and the front wall portion 323. Accordingly, interference of the control unit 30 with other members can be suppressed, and an increase in the size of the back frame 300 can be suppressed by attaching the control unit 30 to the upper frame 320. Incidentally, an electrical component other than the control unit 30, for example, the valve unit 21 may be disposed in the above-described accommodation region.

### <Modification example>

In the above-described fourth example, the control unit 30 has been described as being attached to the back side of the front wall portion 323; however, the present invention is not limited thereto. The control unit 30 may be attached to the front side of the lower wall portion 322.

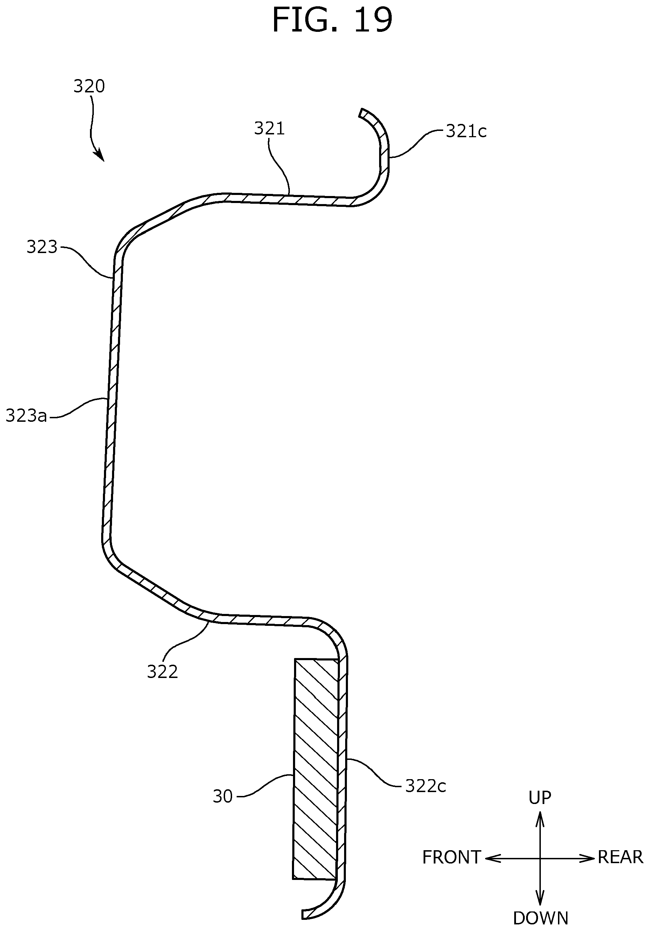

FIG. 19 is a cross-sectional view taken along line A-A of FIG. 17, and shows a state where the control unit 30 is attached to the lower bent portion 322c of the lower wall portion 322. In other words, the control unit 30 is attached to the upper frame 320 in a state where the control unit 30 is surrounded on three sides by the lower bent portion 322c of the lower wall portion 322. Accordingly, similarly to the above-described fourth example, interference of the control unit 30 with other components can be suppressed, and an increase in the size of the back frame 300 can be suppressed by attaching the control unit 30 to the upper frame 320.

### <Second modification example>

In addition, in the above-described fourth example, the control unit 30 has been described as being attached to the upper frame 320; however, the present invention is not limited thereto. The control unit 30 may be attached to the side frame 310.

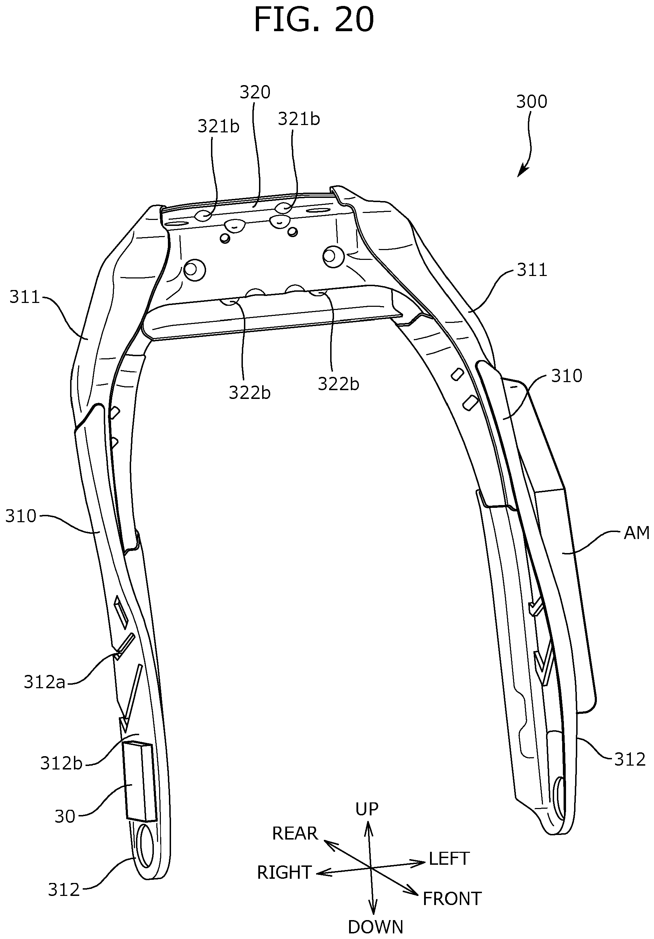

FIG. 20 shows a state where the control unit 30 is attached to the second side frame piece 312. As shown in FIG. 20, of the right and left side frames 310, the control unit 30 is attached to the second side frame piece 312 of the right side frame 310. Meanwhile, an airbag module AM is attached to the second side frame piece 312 of the left side frame 310. In other words, the control unit 30 is attached to the side frame 310 that is one of the right and left side frames 310 to which the airbag module AM is not attached. Accordingly, the control unit 30 can be attached to the side frame 310 without interference of the control unit 30 with the airbag module AM. In addition, when the airbag module AM operates, the influence of the control unit 30 on the operation of the airbag module AM can be suppressed.

Here, the attachment position of the control unit 30 will be described in more detail. In the second side frame piece 312, an uneven portion 312a for improving the rigidity of the second side frame piece 312 is formed at a substantially center position in the up to down direction, and a flat portion 312b coupled to the reclining device is formed below the uneven portion 312a. The control unit 30 is attached to the flat portion 312b. The attachment strength of the control unit 30 can be improved by attaching the control unit 30 to the flat portion 312b.

### <Third modification example>

As a third modification example, the control unit 30 may be attached to be accommodated in a recess of the uneven portion 312a formed in the second side frame piece 312.

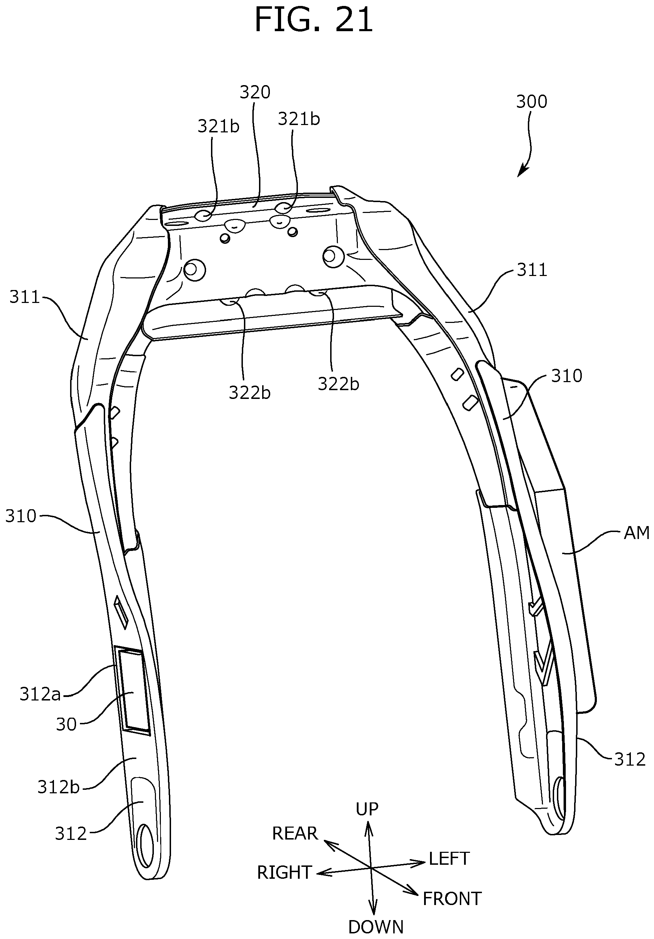

FIG. 21 shows a state where the control unit 30 is attached to the uneven portion 312a of the second side frame piece 312. By attaching the control unit 30 to the side frame 310, interference of the control unit 30 with other members can be suppressed. In addition, since the uneven portion 312a has improved rigidity compared to the flat portion 312b, vibration resistance and impact resistance of the control unit 30 can be improved.

In the above-described embodiment, Calculation Equation (1) and Calculation Equation (2) have been described as linear first-order polynomials; however, the present invention is not limited thereto. The lumbar curve value may be calculated using a calculation equation consisting of a quadratic polynomial or a cubic polynomial.

In addition, in the above-described embodiment, the air cells 11 have been described as being built into the vehicle seat S; however, the present invention is not limited thereto. Any movable body may be used as long as the movable body can be built into the vehicle seat S and can be displaced to apply stimuli to the muscles of the seated occupant, and an elastic body including a drive member consisting of an actuator or the like may be built in.

In addition, in the above-described embodiment, the case where the posture improvement assistance device 10 is mounted in a vehicle has been provided as one example, and the configuration example thereof has been described; however, the present invention is not limited thereto. The posture improvement assistance device 10 can also be mounted in, for example, seats installed in other ground-traveling conveyances including wheels such as trains, or aircrafts, ships, or the like that move in environments other than the ground. In addition, the posture improvement assistance device 10 may be mounted in chairs used in offices or general households.

### <<Second embodiment>>

Next, a second embodiment of the present invention will be described.

### <<Technical field>>

A second embodiment of the present invention relates to a vehicle, particularly to a vehicle including an opening/closing body capable of sliding movement.

### <<Background art>>

Conventionally, a vehicle in which the disposition of vehicle seats installed in the vehicle can be arranged in an interior space of a vehicle body by configuring the vehicle seats to be movable has been known.

For example, in a vehicle disclosed in JP 2021-142771 A, each of a plurality of vehicle seats that are installed includes a rotation mechanism, a reclining mechanism, and a slide mechanism, and by moving the plurality of vehicle seats, for example, a bed surface on which an occupant can lie down can be formed even in a narrow space.

### <<Summary of invention>>

### <<Technical problem>>

In the vehicle in which the seats can be arranged, which is disclosed in JP 2021-142771 A, since the seats are configured to be movable in a narrow space, there is a possibility that it is difficult to get in and out depending on the positions of the vehicle seats. In addition, in order to make the vehicle seats movable in a passenger compartment, in-vehicle equipment that can accommodate various arrangements is required.

The second embodiment of the present invention has been made in view of the above-described problems, and an object of the second embodiment is to provide a vehicle with improved ease of getting in and out for an occupant.

In addition, another object is to provide a vehicle including in-vehicle equipment that can accommodate various arrangements.

### <<Solution to problem>>

The above-described problems are solved by a vehicle of the present invention, the vehicle including: an opening/closing body capable of sliding movement on a side of the vehicle; and at least one seat including a headrest inside the vehicle. At least a part of an upper portion of the opening/closing body is located further toward an inside of the vehicle than an inner end portion of the headrest directly above the headrest.

By locating a part of the upper portion of the opening/closing body further toward the inside than the inner end portion of the headrest of the seat, when the opening/closing body is opened, a region above the headrest opens up, so that even when an occupant stands up during getting in and out, the hitting of the head of the occupant against a roof is suppressed and getting in and out is facilitated. In addition, a sense of openness when the opening/closing body is moved open is improved. Therefore, it is possible to provide the vehicle with improved ease of getting in and out for the occupant.

In addition, in the above-described vehicle, it is preferable that the opening/closing body is composed of a body portion located at a side portion of the vehicle and an upper portion constituting the upper portion of the opening/closing body, and it is preferable that the body portion and the upper portion are integrally formed.

By integrally forming the body portion and the upper portion of the opening/closing body, a sense of unity in the appearance can be achieved.

In addition, in the above-described vehicle, it is preferable that the opening/closing body is composed of a body portion located at a side portion of the vehicle and an upper portion constituting the upper portion of the opening/closing body, and it is preferable that the body portion and the upper portion are formed as separate bodies.

By forming the body portion and the upper portion of the opening/closing body as separate bodies, the manufacture of the opening/closing body can be facilitated.

In addition, in the above-described vehicle, it is preferable that the seat includes a height adjustment device that adjusts a height of the seat from a floor surface of the vehicle, and by the height adjustment device, it is preferable that the seat is configured such that at least an upper end of the headrest is movable above a lower end of the upper portion of the opening/closing body.

By configuring the upper end of the headrest of the seat to be movable above the lower end of the upper portion of the opening/closing body, a sense of openness when the opening/closing body is moved open is further improved.

In addition, in the above-described vehicle, it is preferable that the opening/closing bodies are disposed at right and left side portions of the vehicle, and it is preferable that an occupant protection device is provided at a central portion of a roof of the vehicle, the central portion being sandwiched between the upper portions of the right and left opening/closing bodies.

By providing the occupant protection device (for example, an airbag) at the central portion of the roof, a seated occupant can be protected from a central portion of the vehicle.

In addition, in the above-described vehicle, it is preferable that an instrument panel is provided at an interior front of the vehicle, and it is preferable that the instrument panel is configured as an extendable and retractable storage box.

By configuring the instrument panel as an extendable and retractable storage box, the interior space of the vehicle can be expanded, and it is possible to provide the vehicle including in-vehicle equipment that can accommodate various arrangements.

In addition, in the above-described vehicle, it is preferable that the instrument panel is extendable and retractable in a right to left direction of the vehicle.

By configuring the instrument panel to be extendable and retractable in the right to left direction, the interior space of the vehicle can be easily expanded.

In addition, in the above-described vehicle, it is preferable that the instrument panel is movable in an up to down direction of the vehicle.

By configuring the instrument panel to be movable in the up to down direction, the space below the instrument panel can be used, and the interior space of the vehicle can be further utilized. In addition, for example, when the vehicle is stopped, visibility from the outside can be shielded by the instrument panel that is moved upward.

In addition, it is preferable that the vehicle further includes a seat rail which is disposed on a floor surface of the vehicle and along which the seat slides, and it is preferable that the seat rail is disposed below the instrument panel.

By disposing the seat rail below the instrument panel, the seat can be moved to below the instrument panel, and a wider range of arrangements can be achieved.

In addition, in the above-described vehicle, it is preferable that an odor sensor that measures odors inside the vehicle is provided in the instrument panel.

By providing the odor sensor, odors inside the vehicle can be measured, and the environment inside the vehicle can be improved based on the measurement result.

In addition, in the above-described vehicle, it is preferable that the seat includes a seat rotating device that rotates the seat around an axis along an up to down direction of the vehicle, and inflation members at right and left side portions of the seat, and it is preferable that when the seat rotates around the axis along the up to down direction of the vehicle, the inflation member located on a side opposite to a rotation direction inflates.

When the seat rotates, since the inflation member located on the side opposite to the rotation direction inflates, the tilting of the body of the occupant to the side opposite to the rotation direction is suppressed, the occupant posture holding force is improved, and discomfort to the occupant is suppressed.

### <<Advantageous effects of invention>>

According to the vehicle of the present invention, it is possible to provide the vehicle with improved ease of getting in and out for the occupant.

In addition, a sense of unity in the appearance can be achieved by integrally forming the body portion and the upper portion of the opening/closing body.

In addition, the manufacture of the opening/closing body can be facilitated by forming the body portion and the upper portion of the opening/closing body as separate bodies.

In addition, a sense of openness when the opening/closing body is moved open is further improved by configuring the upper end of the headrest of the seat to be movable above the lower end of the upper portion of the opening/closing body.

In addition, the seated occupant can be protected from the central portion of the vehicle by providing the occupant protection device (for example, an airbag) at the central portion of the roof.

In addition, by configuring the instrument panel as an extendable and retractable storage box, the interior space of the vehicle can be expanded, and it is possible to provide the vehicle including in-vehicle equipment that can accommodate various arrangements.

In addition, the interior space of the vehicle can be easily expanded by configuring the instrument panel to be extendable and retractable in the right to left direction.

In addition, by configuring the instrument panel to be movable in the up to down direction, the space below the instrument panel can be used, and the interior space of the vehicle can be further utilized. In addition, for example, when the vehicle is stopped, visibility from the outside can be shielded by the instrument panel that is moved upward.

In addition, by disposing the rail below the instrument panel, the seat can be moved to below the instrument panel, and a wider range of arrangements can be achieved.

In addition, by providing the odor sensor, odors inside the vehicle can be measured, and the environment inside the vehicle can be improved based on the measurement result.

In addition, when the seat rotates, since the inflation member located on the side opposite to the rotation direction inflates, the tilting of the body of the occupant to the side opposite to the rotation direction is suppressed, the occupant posture holding force is improved, and discomfort to the occupant is suppressed.

### <<Description of embodiments>>

Hereinafter, a configuration of a vehicle 401 including an opening/closing body capable of sliding movement according to a first example of the second embodiment will be described with reference to the drawings. However, the embodiment to be described below is provided for easy understanding of the present invention, and does not limit the present invention. The invention is defined by the appended claims.

In addition, in the following description, the contents regarding the materials, shapes, and sizes of components such as a seat (vehicle seat 410), an instrument panel 420, and an airbag device 423 installed in a vehicle 401 are merely one specific example, and do not limit the present invention.

Incidentally, hereinafter, an electric vehicle capable of autonomous driving will be provided as an example of the vehicle 401 including an opening/closing body capable of sliding movement, and a configuration example thereof will be described. However, the present invention may also be applied to manually driven engine vehicles. In addition, the present invention is not limited to ground-traveling conveyances including wheels such as automobiles and railroad vehicles, and can also be applied to, for example, aircrafts, ships, or the like that move in environments other than the ground.

In addition, in the following description, the "front to rear direction" is a front to rear direction of the vehicle 401, and is a direction that coincides with a traveling direction of the vehicle when the vehicle travels. In addition, the "width direction" and the "right to left direction" are a lateral width direction and a right to left direction of the vehicle 401, and the "up to down direction" is an up to down direction of the vehicle 401, and is a direction that coincides with a vertical direction when the vehicle 401 travels on a horizontal surface.

In addition, in the following description, when various directions are described with "seat" added, such as a "seat width direction" and a "seat height direction", the various directions indicate directions with respect to the vehicle seat 410 installed in the vehicle 401, and when directions are described with "vehicle" added, such as "vehicle inside" and "vehicle outside", the directions indicate directions with respect to the vehicle 401.

### <Vehicle 401>

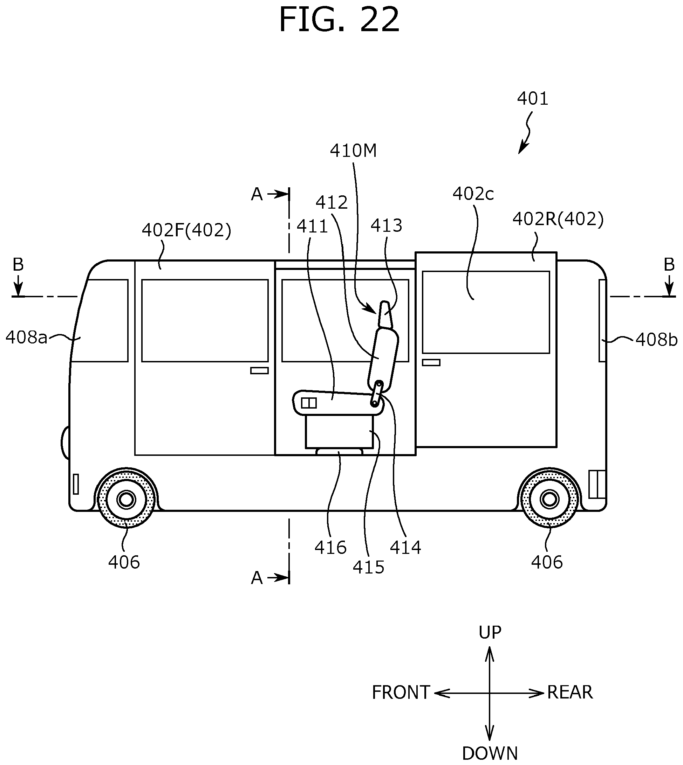

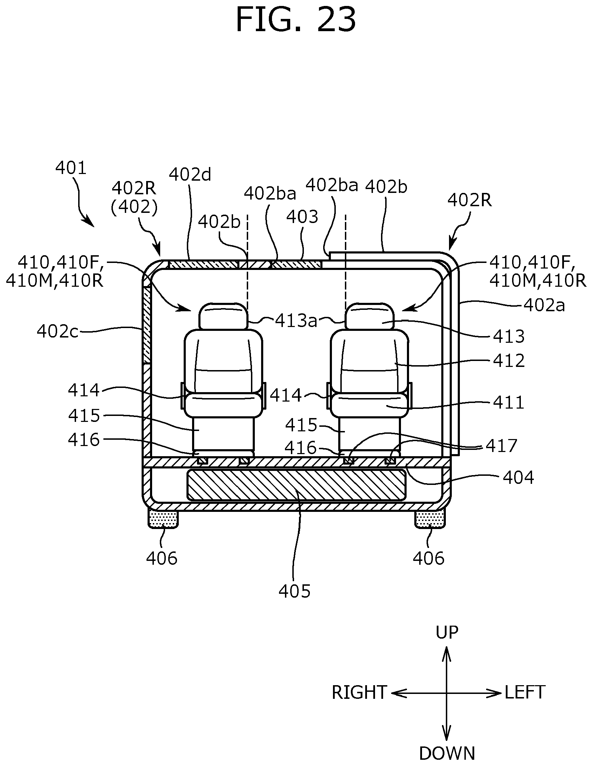

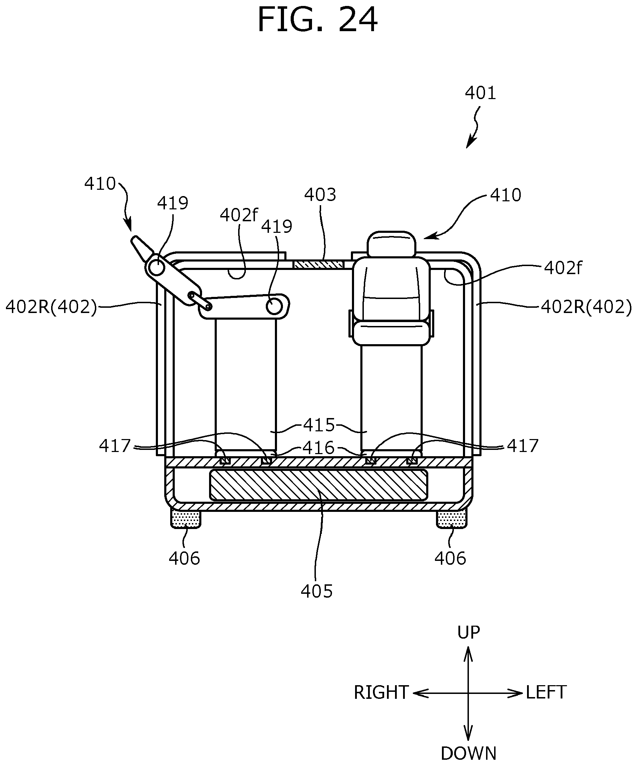

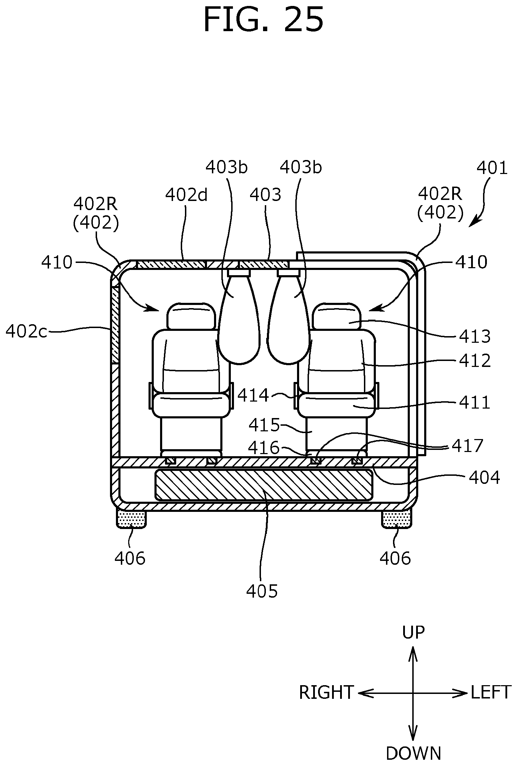

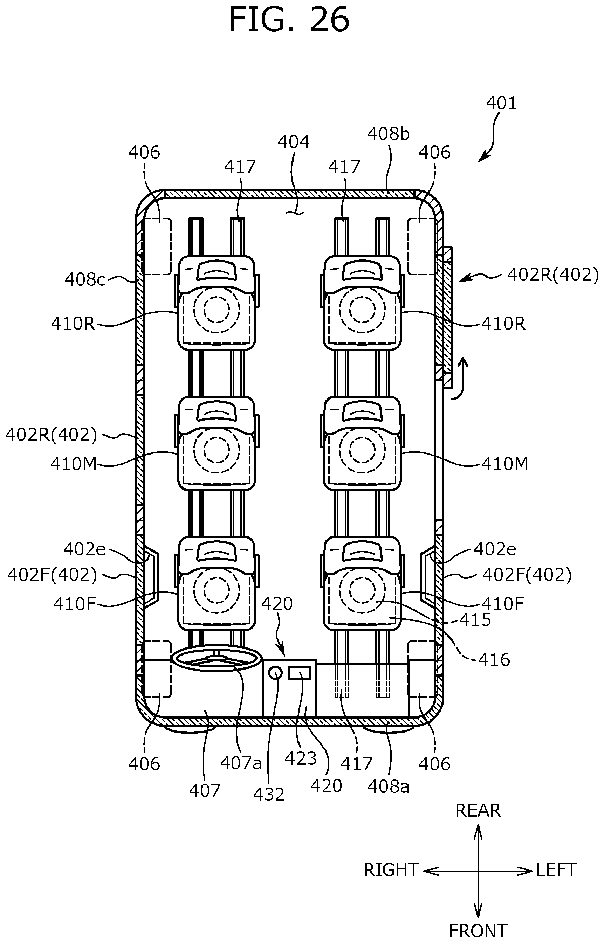

A configuration of the vehicle 401 will be described with reference to FIGS. 22 to 26. FIG. 22 is a side view of the vehicle 401, and shows a state where a rear door 402R that is a sliding door is opened, and FIG. 23 is a cross-sectional view taken along line A-A of FIG. 22, and shows an interior configuration of the vehicle. FIG. 24 is a view showing a state where the vehicle seat is moved upward, and FIG. 25 is a view showing a state where airbags provided at a center portion of a roof 403 are deployed. FIG. 26 is a cross-sectional view taken along line B-B of FIG. 22.

The vehicle 401 is a four-wheeled electric vehicle, and as shown in FIGS. 22 to 26, includes tires 406 at four corners of a floor 404, and a battery 405, which supplies electric power to a motor and the like, under the floor 404.

The vehicle 401 includes a front window 408a at the front and a rear window 408b at the rear as windows 408. The vehicle 401 includes side windows 408c on the sides.

In addition, the vehicle seats 410 arranged in three rows as shown in FIGS. 23 and 26 are disposed on the floor 404 of the vehicle 401. The vehicle 401 includes, on the floor 404, front seats 410F disposed on the right and left of a first row, middle seats 410M disposed on the right and left of a second row, and rear seats 410R disposed on the right and left of a third row. The front seats 410F, the middle seats 410M, and the rear seats 410R have basically the same configuration, and in the following description, are referred to as the vehicle seats 410 unless there is a particular need to distinguish therebetween.

### <Vehicle seat 410>

Each of the vehicle seats 410 (seat) includes a seat cushion 411 that supports the thighs and buttocks of an occupant H; a seat back 412 that supports the back of the occupant H; and a headrest 413 that is provided at an upper end portion of the seat back 412 and that supports the head of the occupant H.

A rear end portion of the seat cushion 411 and a lower end portion of the seat back 412 are coupled to each other, and a reclining device 414 is provided at a coupling portion. The reclining device 414 can rotate the seat back 412 with respect to the seat cushion 411 such that the rearward tilt angle of the seat back 412 can be adjusted.

A height adjustment device 415 that adjusts the height of the vehicle seat 410 is provided below the seat cushion 411. A seat rotating device 416 that rotates the vehicle seat 410 about an axis along the up to down direction of the vehicle 401 (direction perpendicular to the floor 404) is provided below the height adjustment device 415. A slide device that slides the vehicle seat 410 in the front to rear direction on the floor 404 of the vehicle 401 is provided below the seat rotating device 416.

The slide device is composed of upper rails (not illustrated) provided at a lower portion of the vehicle seat 410, and seat rails 417 disposed on the floor surface of the vehicle 401, and by sliding the upper rails on the seat rails 417, the vehicle seat 410 is movable in the front to rear direction inside the vehicle in a sliding manner.

Incidentally, in the present embodiment, the seat rails 417 are disposed as long rails to be embedded in the floor 404. As shown in FIG. 26, the front seat 410F, the middle seat 410M, and the rear seat 410R disposed on the right side of the vehicle are disposed on the seat rails 417 on a driver's seat side so as to be movable in a sliding manner or rotationally movable. In addition, the front seat 410F, the middle seat 410M, and the rear seat 410R disposed on the left side of the vehicle are disposed on the seat rails 417 on a passenger seat side so as to be movable in a sliding manner or rotationally movable.

The vehicle seat 410 may be detachably attached to the seat rails 417. In the present embodiment, the seat rails 417 are shared by the front seat 410F to the rear seat 410R disposed in the front to rear direction; however, the seat rails 417 may not be shared. Namely, the front seat 410F may use a pair of independent rails, and the middle seat 410M and the rear seat 410R may be movably disposed on a pair of rails provided separately from the independent rails.

In addition, it is preferable that the seat rails 417 disposed on the driver's seat side and the seat rails 417 on the passenger seat side are disposed on a planar portion of the floor that covers an upper portion of the battery 405.

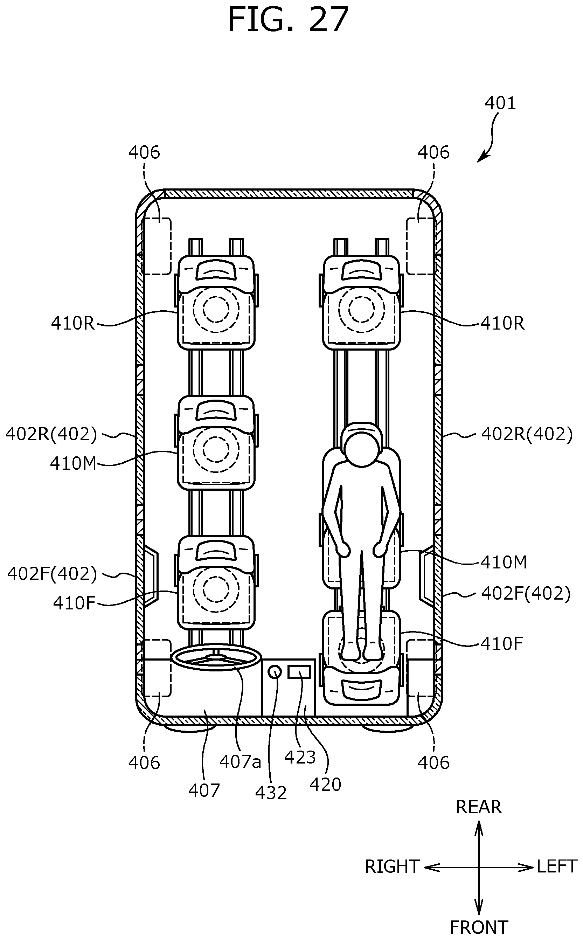

The seat rails 417 disposed on the passenger seat side extend to a front end of the vehicle 401 as shown in FIG. 26, and are disposed such that front portions of the seat rails 417 are located below the instrument panel 420. Therefore, when the lateral width of the instrument panel 420 is reduced, the front seat 410F on the passenger seat side is movable to a front end portion of the vehicle 401 (refer to FIG. 27).

Although only the movement direction of the vehicle seat 410 shown in FIG. 26 is the front to rear direction, a rail device that is movable in the right to left direction may be added to each of the vehicle seats 410 to be able to move each of the vehicle seats 410 in the right to left direction.

In addition, the sliding movement, rotational movement, and up and down movement (height adjustment) of the vehicle seat 410 may be manually performed by the occupant, or a configuration in which a drive device such as a motor is provided to allow each of the vehicle seats 410 to automatically perform sliding movement, rotational movement, and up and down movement may be implemented.

When the vehicle seat 410 can automatically perform sliding movement, rotational movement, and up and down movement, the vehicle seat 410 may be configured to automatically assume a registered seat arrangement, for example, upon operation of a transmitter held by the occupant. The transmitter operated by the occupant may be formed integrally with an operating switch for instructing locking or opening and closing of a door, or may be formed separately from the operating switch.

<Vehicle door 402>

Vehicle doors 402 (opening/closing bodies) that can be opened and closed with respect to a vehicle body are provided at right and left outer portions of the vehicle 401. The occupant H seated in the vehicle seat 410 can get in and out by moving the vehicle door 402 open. In the vehicle 401 of the present embodiment, front doors 402F are provided on the right and left of the front seats 410F of the first row, and the rear doors 402R are provided on the right and left of the middle seats 410M of the second row. A door pocket 402e that is removable is provided on the inner side of the front door 402F. The rear door 402R is provided with a window portion 402c.

As shown in FIG. 22, the rear door 402R is configured as a sliding door that is slidable rearward. In addition, as shown in FIG. 23, the rear door 402R includes not only a side portion of the vehicle 401, but also a part of the roof of the vehicle 401. Specifically, the rear door 402R is configured such that at least a part of an upper portion of the rear door 402R is disposed further toward the inside of the vehicle 401 than an inner end portion 413a of the headrest 413 directly above the headrest 413 of the middle seat 410M.

In more detail, the rear door 402R is composed of a door body portion 402a located at the side portion of the vehicle 401, and a door upper portion 402b constituting the upper portion of the rear door 402R, and the door upper portion 402b is formed to extend from an upper end portion of the door body portion 402a toward the inside of the vehicle 401. Furthermore, the rear door 402R is configured such that an inner end portion 402ba of the door upper portion 402b is located further toward the inside of the vehicle 401 than the inner end portion 413a of the headrest 413.

By configuring the upper portion of the rear door 402R, namely, the door upper portion 402b in such a manner, when the rear door 402R is opened, a region above the vehicle seat 410 opens up, so that when the occupant stands up, the hitting of the head of the occupant against the roof 403 is suppressed, and ease of getting in and out is improved. In addition, a sense of openness when the rear door 402R is opened is improved.

It is preferable that the door body portion 402a and the door upper portion 402b are integrally formed. Particularly, when an interior member including a skin of the door body portion 402a and an interior member including a skin of the door upper portion 402b are integrally formed, a sense of unity in the appearance of the door is achieved, which is preferable.

Incidentally, the door body portion 402a and the door upper portion 402b may be configured as separate bodies. By making the door body portion 402a and the door upper portion 402b as separate bodies, the manufacture of the rear door 402R can be facilitated.

In addition, as shown in FIG. 23, it is preferable that a sunroof 402d is provided in a part of the door upper portion 402b.

In addition, the roof 403 may also be provided with a sunroof 403a. By providing the sunroofs 402d and 403a, the occupant is allowed to feel a sense of openness even when the rear door 402R is in a closed state.

In the vehicle 401 of the present embodiment, when the rear door 402R is opened, a part of the roof is opened as shown in FIG. 24. Therefore, when the rear door 402R is in an open state, the vehicle seat 410 may be raised by the height adjustment device 415. In the present embodiment, the vehicle seat 410 is configured such that at least an upper end of the headrest 413 is movable above a lower end 402f of the upper portion of the rear door 402R in a closed state by the height adjustment device 415. In other words, the vehicle seat 410 is configured such that the headrest 413 can be raised above a lower end of the roof 403 by the height adjustment device 415. By allowing the upper end of the headrest 413 to move above the lower end 402f of the upper portion of the rear door 402R, the occupant H is allowed to feel a greater sense of openness.

In addition, when the headrest 413 of the vehicle seat 410 is raised above the lower end of the upper portion of the rear door 402R, namely, when the headrest 413 is raised above the roof 403, the headrest 413 becomes farther away from notification means, for example, a speaker, a lighting, or the like provided inside the vehicle. Therefore, it is preferable that the function of the notification means is more enhanced than normal, namely, when the vehicle seat 410 is disposed inside the vehicle.

For example, when the notification means is a speaker, the notification sound is made louder. When the notification means is lighting, the lighting is caused to emit more intense light. Consequently, even when the position of the vehicle seat 410 is raised and the head of the occupant is located outside the vehicle, a notification can be made.

In addition, as shown in FIG. 24, a speaker 419 may be disposed in the vehicle seat 410, as the notification means. Even in this case, there is a possibility of the notification sound radiating outside the vehicle, so that it is desirable that the sound is made louder than usual.

### <Roof airbag device 403b>

Roof airbag devices 403b that are occupant protection devices may be provided at a central portion of the roof 403 of the vehicle 401, the central portion being sandwiched between the upper portions (door upper portions 402b) of the right and left rear doors 402R. For example, in the event of a side impact, as shown in FIG. 25, bags of the roof airbag devices 403b can inflate downward to protect the occupant H.

### <Instrument panel 420>

Other equipment of the present embodiment will be described with reference to FIGS. 26 to 35. As shown in FIG. 26, an operating system box 407 and the instrument panel 420 are provided at the interior front of the vehicle 401. A steering wheel 407a (steering) or pedals (not illustrated) that are operated by a driver are attached to the operating system box 407. The instrument panel 420 is provided with an operating panel for operating devices inside the vehicle, and the occupant of the vehicle 401 can use the operating panel to input and change the disposition and the like of the vehicle seat 410.

In addition, the instrument panel 420 is provided with a function for storing items, and can be used as a storage box.

The steering wheel 407a of the vehicle 401 or the door pocket 402e provided on the front door 402F are configured to be detachably attached, and the steering wheel 407a or the door pocket 402e that is removed can be stored in the instrument panel 420 that is widened. By storing the steering wheel 407a and the door pocket 402e that are removed in the instrument panel 420, the degree of freedom of movement of the vehicle seat 410, for example, forward and rearward movement and rotational movement is expanded.

The instrument panel 420 is further configured to be extendable and retractable in the width direction of the vehicle 401. As shown in FIG. 27, it is preferable that the instrument panel 420 is configured to be extendable and retractable in the width direction (right to left direction) of the vehicle 401.

By reducing the size of the instrument panel 420, in other words, by downsizing the instrument panel 420, the vacant saved can be used as seat space. For example, as shown in FIG. 27, by rotating the front seat 410F and disposing the front seat 410F in the seat space, the seat space can be used as a footrest place for an occupant seated in the middle seat 410M. Further, when the seat cushion 411 or the seat back 412 of the front seat 410F is provided with a massage device or a movable device for exercising the feet of an occupant, the seat space can be utilized effectively.

Incidentally, the extension and retraction of the instrument panel 420 may be manually performed, or a motor or the like may be provided and the extension and retraction of the instrument panel 420 may be automatically performed. When the extension and retraction of the instrument panel 420 is automatically performed, a configuration in which the occupant H transmits an instruction signal using a transmitter capable of wireless communication and the instrument panel 420 extends or retracts in response to the signal may be implemented.

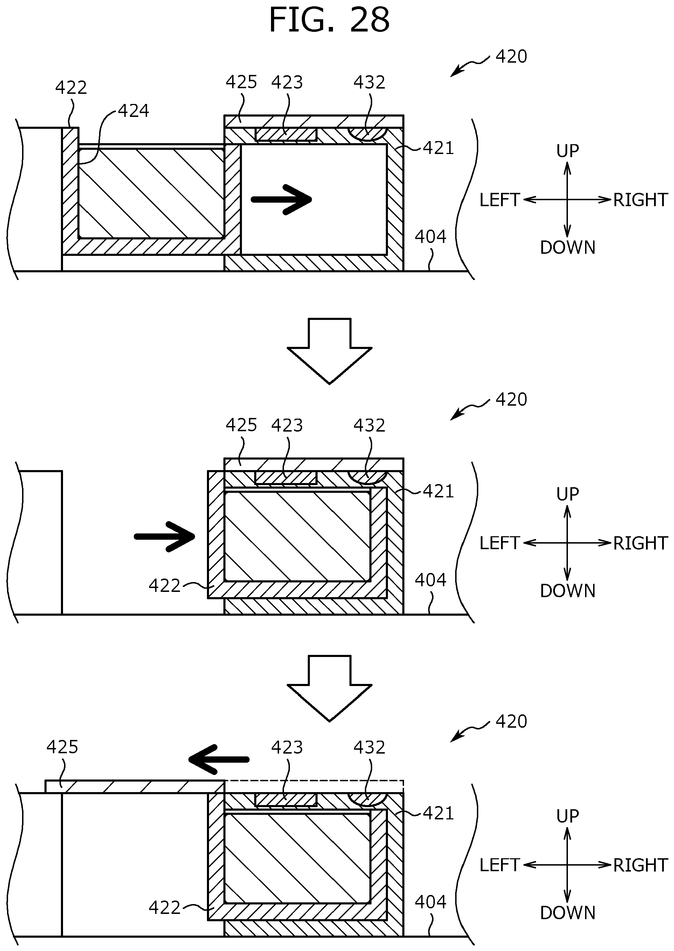

A structure of the instrument panel 420 that is extendable and retractable will be described with reference to FIG. 28. The instrument panel 420 is composed of a panel body 421 and a pull-out portion 422 stored in the panel body 421. When the instrument panel 420 is used as a storage box, as shown in the upper part of FIG. 28, the pull-out portion 422 is pulled out to the left side of the vehicle 401, and items, for example, the steering wheel 407a or the door pocket 402e can be stored in a storage part 424. A refrigeration device may be installed in the storage part 424, and the instrument panel 420 may be used as a refrigerator.

When the instrument panel 420 is reduced in size and the seat space is expanded, as shown in the middle part of FIG. 28, the pull-out portion 422 is moved in a rightward direction of the vehicle 401, and the pull-out portion 422 is stored in the panel body 421.

In addition, a storage type table 425 may be provided on an upper portion of the panel body 421 of the instrument panel 420, and when the pull-out portion 422 is stored in the panel body 421, as shown in the lower part of FIG. 28, the storage type table 425 may be pulled out in a leftward direction of the vehicle 401 for use. By moving the front seat 410F to the front of the vehicle 401, the seat arrangement can be set to a study mode in which the occupant faces the storage type table 425.

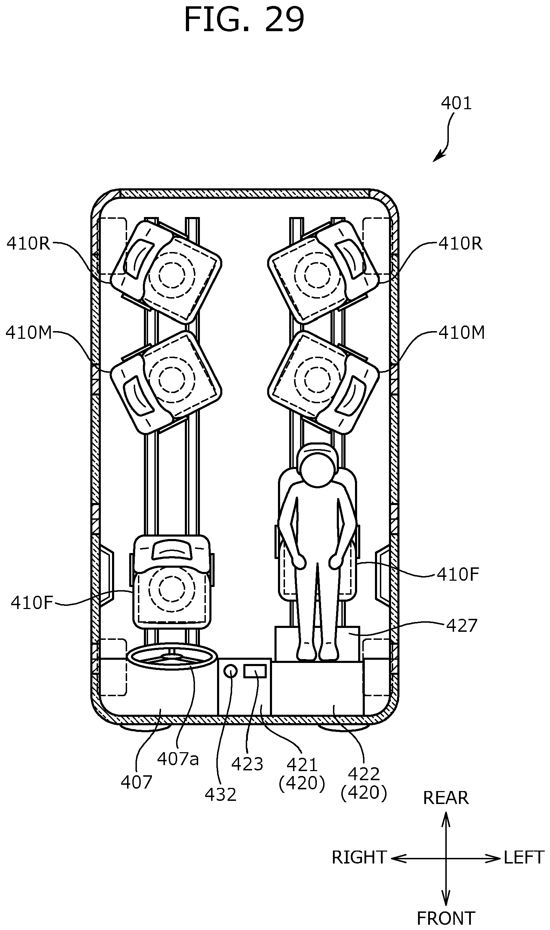

In addition, as shown in FIG. 29, a wall portion on a passenger compartment side of the instrument panel 420 may be used as a footrest portion 427 with the wall portion serving as a cushion by rotating the wall portion around an upper rotating shaft 427a, and fixing the wall portion at any position.

By providing the footrest portion 427 on a rear surface of the instrument panel 420, namely, a surface on the passenger compartment side, the occupant seated in the front seat 410F can place the feet and relax, so that the passenger compartment space can be utilized effectively.

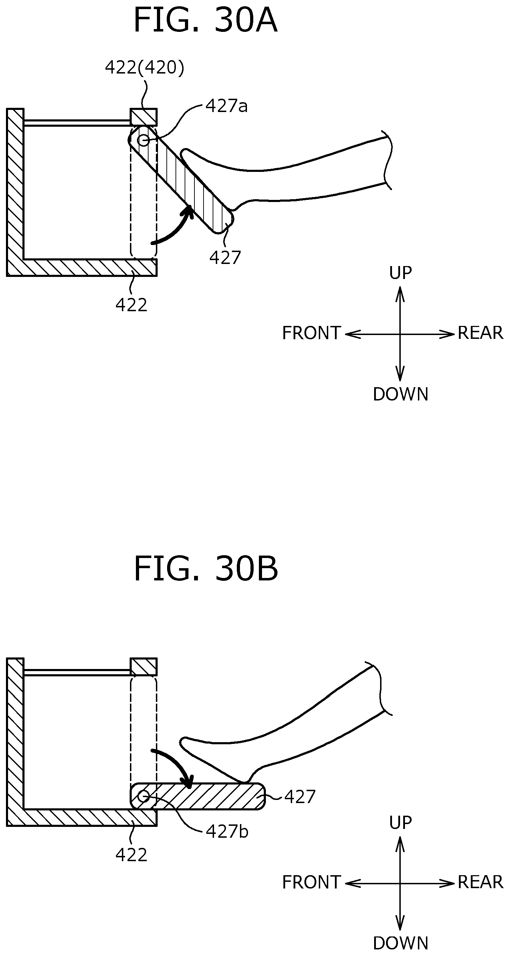

Regarding the drawing out of the footrest portion 427, for example, as shown in FIG. 30A, the upper rotating shaft 427a may be provided on an upper side of the pull-out portion 422, and the footrest portion 427 may be pulled out from below.

In addition, regarding the drawing out of the footrest portion 427, as shown in FIG. 30B, a lower rotating shaft 427b may be provided on a lower side of the pull-out portion 422, and the footrest portion 427 may be pulled out from the upper side.

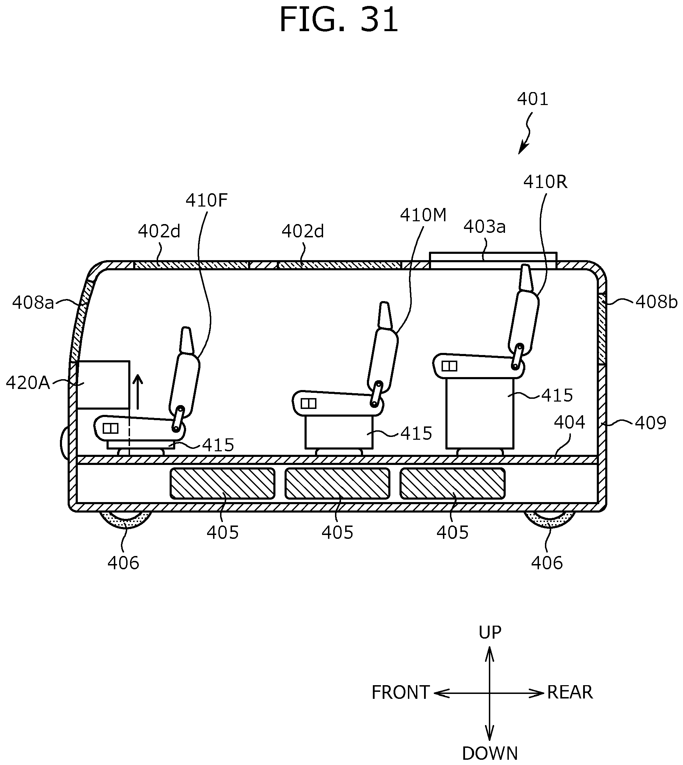

Another example of the instrument panel 420 is shown in FIG. 31. An instrument panel 420A is configured to be movable in the up to down direction. FIG. 31 shows a state where the instrument panel 420A is moved in an upward direction. For example, when the vehicle is stopped, visibility from the outside can be shielded by moving the instrument panel 420 upward. At this time, the front window 408a may be changed to a light-shielding state. In addition, the rear window 408b and the side windows 408c may be changed to a light-shielding state.

In addition, the position of the line of sight of the occupant H may be changeable by opening the sunroof 403a and raising the rear seat 410R using the height adjustment device 415.

In addition, since a space can be created below the instrument panel 420 by moving the instrument panel 420 upward, a space behind the front seat 410F can be utilized effectively by moving the front seat 410F forward in a state where the height of the front seat 410F is lowered, and storing a part of the seat cushion 411 below the instrument panel 420.

### <Center console 430>

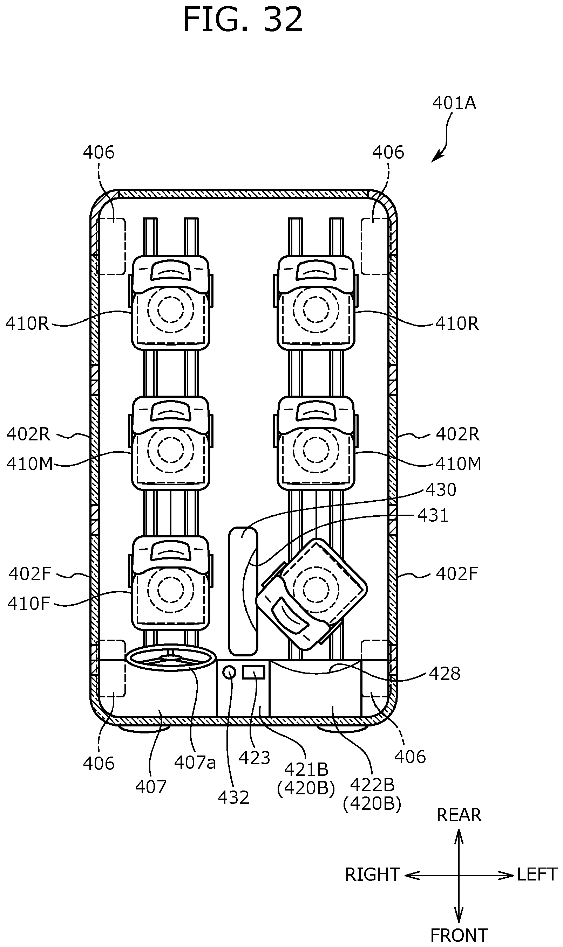

FIG. 32 shows a vehicle 401A that is another example. In the vehicle 401A, a center console 430 is disposed between the vehicle seats 410 disposed on the right and left. More specifically, the center console 430 is disposed between the front seat 410F on the driver side and the front seat 410F on the passenger seat side. The center console 430 is provided with, for example, operating portions such as a shift lever and a side brake lever, and a storage portion such as a cup holder.

When the center console 430 is disposed, there is a possibility that the rotational movement of the vehicle seat 410 is disturbed. Therefore, as shown in FIG. 32, a part of the center console 430 is configured to be deformable so as not to interfere with the vehicle seat 410 when the vehicle seat 410 rotationally moves. Specifically, a part of a side portion of the center console 430 is deformed to form a recess 431.

When the front seat 410F on the passenger seat side rotates, a left side portion of the center console 430 deforms. In addition, when the front seat 410F on the driver's seat side rotates, a right side portion of the center console 430 is configured to deform.

When the front seat 410F on the passenger seat side rotates, further, a pull-out portion 422B of an instrument panel 420B in front of the front seat 410F may be deformed to form a recess 428.

The instrument panel 420B is composed of a panel body 421B and the pull-out portion 422B that can be stored in the panel body 421B. When the front seat 410F on the passenger seat side rotates, the pull-out portion 422B may be stored in the panel body 421B.

In addition, an inner wall portion of the front door 402F or the rear door 402R or the door pocket 402e may be deformed to form a recess.

### <Odor sensor 432>

In order to quantitatively measure the environment inside the vehicle, particularly odors, an odor sensor 432 may be provided in the passenger compartment. In the present embodiment, the odor sensor 432 is provided in the instrument panel 420, as means for detecting an in-vehicle state.

Since the airbag device 423 as an occupant protection device is also attached to the instrument panel 420, the odor sensor 432 is attached at a position avoiding an attachment portion of the airbag device 423, for example, a deployment path in which the bag of the airbag device 423 is deployed or the like. By attaching the odor sensor 432 so as to avoid the attachment portion of the airbag device 423, the influence of the odor sensor 432 on the airbag device 423 can be suppressed.

The odor sensors 432 may be provided in interior members other than the instrument panel 420, for example, the roof 403, the vehicle seat 410, the vehicle door 402, and the like.

When the interior member is extendable and retractable as in the instrument panel 420 of the present embodiment, or includes a deformable portion such as the roof 403, the vehicle door 402, the center console 430, or a sun visor, and may extend or retract, it is preferable that the odor sensor 432 is provided at a position avoiding the extendable and retractable portion or the deformable portion. By disposing the odor sensor 432 so as to avoid the portions that deform, a decrease in the detection accuracy of the odor sensor 432 can be suppressed.

When the odor sensor 432 is provided in the interior member, it is preferable that the odor sensor 432 can be stored. For example, as a simple method, a configuration in which a cover that covers the odor sensor 432 is provided to be openable and closable and when the odor sensor 432 is not in use, the cover is opened and closed manually or automatically may be implemented.

When the odor sensor 432 is automatically stored, it is preferable that the odor sensor 432 stored in an interior member such as the instrument panel 420 is configured to be exposed to the interior of the vehicle when a sensor or the like confirms that the occupant H get in the vehicle. For example, the odor sensor 432 is configured to protrude toward the passenger compartment when it is detected that the occupant H gets in the vehicle. The detection capability is improved by exposing the odor sensor 432.

The odor sensor 432 may be configured to be stored in an interior member such as instrument panel 420 after the detection of odor ends, for example, after a predetermined time (five to ten seconds) has elapsed after getting in the vehicle is detected.

Incidentally, the detection of an occupant is performed, for example, by a seating sensor provided in the vehicle seat 410, a camera provided inside the vehicle, or the like.

When the odor sensor 432 is provided in an interior member such as the instrument panel 420, attachment workability can be greatly improved by integrating the odor sensor 432 and other sensors, for example, a temperature sensor, a humidity sensor, a sound sensor, and a light sensor into one unit.

Even when a plurality of sensors, namely, a first sensor and a second sensor (may have a detection target different from or the same as a detection target of the first sensor) are unitized and disposed in the instrument panel 420, it is preferable that the integrated unit is provided at a position avoiding an occupant protection device (airbag device or the like).

### <Occupant support member>

The vehicle seat 410 installed in the vehicle 401 of the present embodiment includes the seat rotating device 416 as described above, and can change the orientation around a rotation axis along the up to down direction of the vehicle 401. In the vehicle seat 410 including the seat rotating device 416, improving the occupant posture holding force and suppressing discomfort occurring during rotation of the seat have been desired.

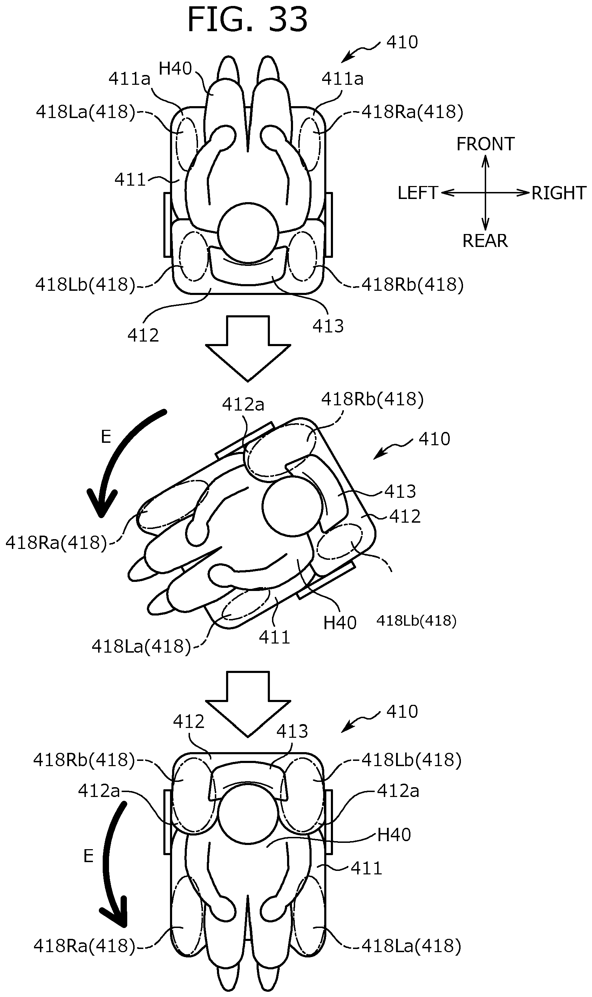

The vehicle seat 410 of the present embodiment is provided with side supports 418 (occupant support members) that support an occupant when the seat portion moves. The side supports 418 are disposed at right and left side portions of the vehicle seat 410, for example, as shown in the upper part of FIG. 33, and more specifically, are configured as air cells (inflation members) or the like provided inside bolster portions 411a of the seat cushion 411 and inside bolster portions 412a of the seat back 412.

The side supports 418 support the occupant H when the vehicle seat 410 moves (including forward and rearward movement and rotational movement). The side supports 418 operate when movement of the vehicle seat 410 is detected, for example, when a seat movement switch is operated by the occupant H.

It is preferable that the movement of the vehicle seat 410 is started after it is detected that the side supports 418 are in an operating state, preferably after the operation completion state of the side supports 418 is detected.

When the movement of the vehicle seat 410 and the operation of the side supports 418 are performed simultaneously, the movement speed of the vehicle seat 410 is set to be slower than usual in the initial stage of the movement of the vehicle seat 410. Consequently, discomfort can be suppressed.

When the movement of the vehicle seat 410 in the front to rear direction is detected, it is preferable that the right and left side supports 418 operate.

In addition, when the rotation of the vehicle seat 410 is detected, namely, when a seat rotation switch is operated, it is preferable that the side supports 418 disposed on the side opposite to a rotation direction operate.

Specifically, as shown in the middle part of FIG. 33, when the vehicle seat 410 rotates left (counterclockwise, in the direction of arrow E in FIG. 33), the side supports 418 disposed on the right side of the vehicle seat 410, in more detail, a cushion air cell 418Ra and a back air cell 418Rb operate and inflate to support the occupant H.

Consequently, the tilting of the body of the occupant to the side opposite to the movement direction (rotation direction) due to the movement (rotation) of the vehicle seat 410 can be suppressed. Accordingly, discomfort to the occupant can be suppressed, or the occupant posture holding force can be improved.

In addition, it is preferable that until the movement (rotation) of the vehicle seat 410 ends or when the movement ends, the side supports 418 disposed on the same side as the movement direction (rotation direction) of the vehicle seat 410 operate.

Specifically, as shown in the lower part of FIG. 33, in a case where the vehicle seat 410 rotates left, when the rotation ends, the side supports 418 disposed on the left side of the vehicle seat 410, in more detail, a cushion air cell 418La and a back air cell 418Lb further operate and inflate to support the occupant H.

By operating the side supports 418 in such a manner, the tilting of the body of the occupant to the same side as the movement direction (rotation direction) immediately after the movement (rotation) of the seat ends can be suppressed. Accordingly, discomfort to the occupant can be suppressed, or the occupant posture holding force can be improved.

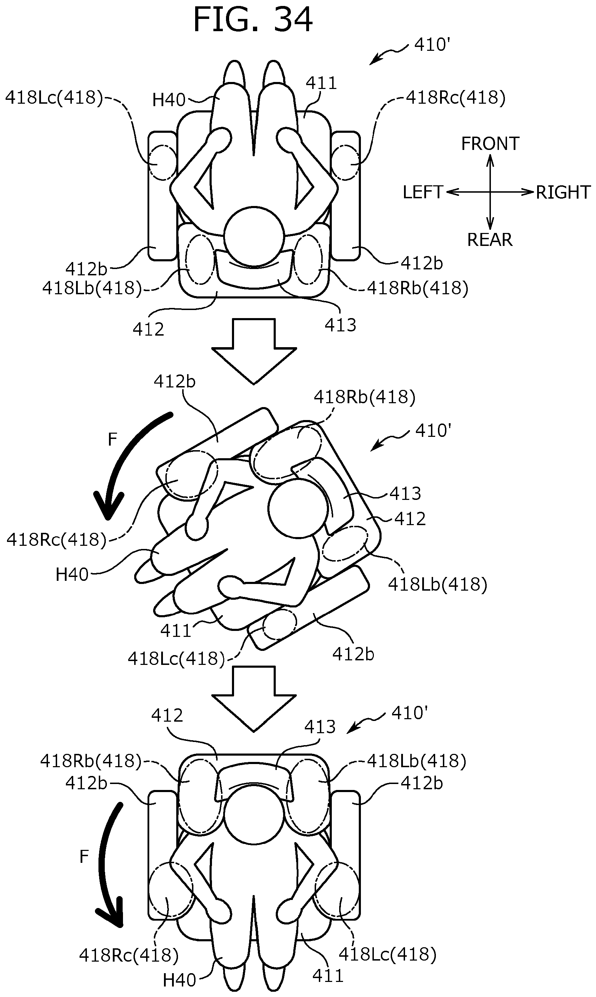

In addition, as in a vehicle seat 410' shown in the upper part of FIG. 34, the side supports 418 may be provided in armrests 412b provided at side portions of the seat back 412.

As shown in the middle part of FIG. 34, when the vehicle seat 410' rotates left (in the direction of arrow F), the side support 418 (armrest air cell 418Rc) provided in the right armrest 412b of the vehicle seat 410' inflates to support the occupant H. At this time, the cushion air cell 418La or the back air cell 418Lb on the same side as the armrest air cell 418Rc may inflate.

In addition, as shown in the lower part of FIG. 34, when the rotation ends, the side support 418 (armrest air cell 418Lc) disposed on the left side of the vehicle seat 410' inflates to support the occupant H. At this time, the cushion air cell 418La or the back air cell 418Lb on the same side as the armrest air cell 418Lc may operate.

The side supports 418 shown in FIGS. 33 and 34 are realized by air cells; however, the side supports 418 may be realized by a structure other than air cells as long as the structure can support the occupant.

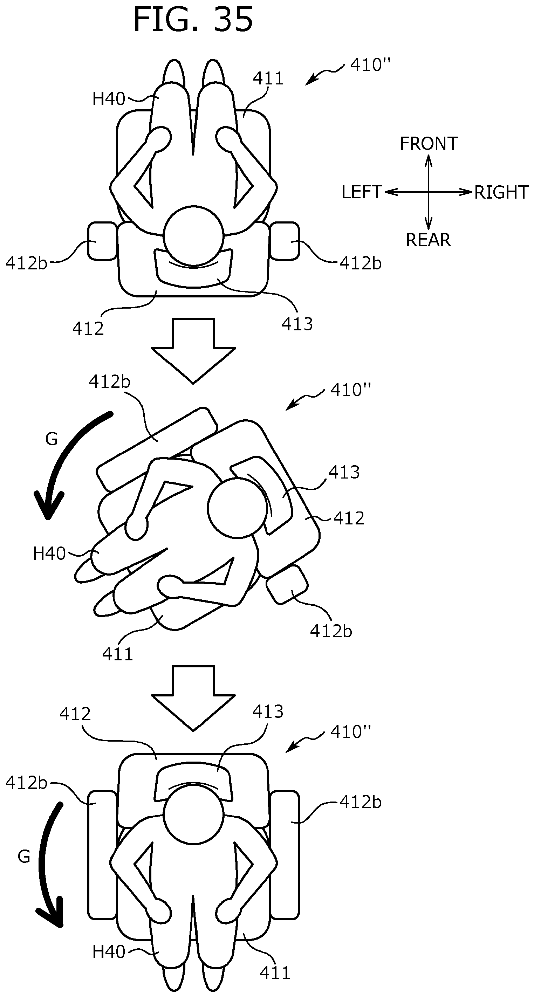

FIG. 35 shows side supports 418A that are another example of the side supports 418. The side supports 418A of a vehicle seat 410'' shown in the upper part of FIG. 35 are provided at the side portions of the seat back 412, and are realized by the armrests 412b that are automatically movable between a storage position and a use position. The armrest 412b in the storage position is configured to be moved to the use position during movement (rotation) of the seat to hold the occupant posture.

As shown in the middle part of FIG. 35, when the vehicle seat 410'' rotates left, only the right armrests 412b on the side opposite to the rotation direction move from the storage position to the use position.

Furthermore, as shown in the lower part of FIG. 35, until the rotation of the vehicle seat 410 ends or when the rotation ends, the tilting of the occupant (inclination in the rotation direction) after the rotation ends is suppressed by moving the left armrests 412b on the same side as the rotation direction to the use position.

The second embodiment of the present invention has been described above with reference to the drawings. In the present embodiment, a vehicle door that moves rearward in a sliding manner when opened has been provided as an example of the opening/closing body capable of sliding movement, and the vehicle 401 including the vehicle door has been described; however, the opening/closing body capable of sliding movement may be a front door that moves forward in a sliding manner when opened. In addition, the opening/closing body capable of sliding movement may be a window that is slidable in the front to rear direction or the width direction. By locating an upper portion of the window further toward the inside of the vehicle than a region directly above the headrest, a sense of openness felt by the occupant when the window is opened is improved.

In addition, the instrument panel 420 that is extendable and retractable or the center console 430 that is deformable, which are installed in the vehicle 401, can be applied not only to vehicles including doors capable of sliding movement, but also to vehicles including doors that open and close normally in the right to left direction.

### <<Third embodiment>>

Next, a third embodiment of the present invention will be described.

### <<Technical field>>

The third embodiment of the present invention relates to a conveyance, particularly to a conveyance including a conveyance seat.

### <<Background art>>

Conventionally, a vehicle in which the disposition of vehicle seats installed in the vehicle can be arranged in an interior space of a vehicle body by configuring the vehicle seats to be movable has been known.

For example, in a vehicle disclosed in JP 2021-142771 A, each of a plurality of vehicle seats that are installed includes a rotation mechanism, a reclining mechanism, and a slide mechanism, and by moving the plurality of vehicle seats, for example, a bed surface on which an occupant can lie down can be formed even in a narrow space.

### <<Summary of invention>>

### <<Technical problem>>

In a conventional vehicle, since a bed surface or the like is formed by deforming or rotating and moving seats, in order to use an interior space more spaciously, a complicated procedure is required. Therefore, using the interior space of the conveyance spaciously with a simpler configuration is required.

The third embodiment of the present invention has been made in view of the above-described problems, and an object of the third embodiment is to provide a conveyance vehicle that allows an interior space to be used easily and spaciously, and that has improved comfort or convenience.

In addition, another object is to provide a conveyance including equipment that can accommodate various arrangements and layouts.

### <<Solution to problem>>

The above-described problems are solved by a conveyance of the present invention, the conveyance including: an interior space that an occupant enters; a wall portion including a floor, an upper wall, and a side wall that surround the interior space; a mattress that is disposed on the floor and that supports at least a lower body of the occupant; and a seat back that supports an upper body of the occupant. The seat back is attached to be storable in a storage portion provided in any one of the mattress, the upper wall, and the side wall.

Since the seat back is attached to be storable in the storage portion provided in any one of the mattress, the upper wall, and the side wall, it is possible to provide the conveyance in which the seat back can be stored in the storage portion, in which the interior space can be used easily and spaciously, and which has improved comfort or convenience.

In addition, in the above-described conveyance, it is preferable that the seat back is coupled to the mattress and is stored in the storage portion provided in the mattress.

Since the mattress includes the storage portion that stores the coupled seat back, the seat back can be easily stored, and the interior space can be used spaciously.

In addition, in the above-described conveyance, it is preferable that the storage portion is provided behind a coupling portion where the seat back is coupled to the mattress.

By moving the seat back rearward, the seat back can be stored in the storage portion, and the interior space can be easily expanded.

In addition, in the above-described conveyance, when the seat back is stored in the storage portion of the mattress, it is preferable that a support surface of the seat back and a support surface of the mattress are flush with each other.

By setting the support surface of the seat back and the support surface of the mattress to be flush with each other, a bed surface can be easily created.

In addition, in the above-described conveyance, it is preferable that a plurality of the seat backs and the storage portions corresponding to the plurality of seat backs are provided in a single mattress.

By providing the plurality of seat backs and the storage portions corresponding to the seat backs in the single mattress, the man-hour required to assemble the mattress to the conveyance or the number of components required for a conveyance seat is reduced.

In addition, in the above-described conveyance, it is preferable that the seat back is independent of the mattress and is stored in the storage portion provided in the upper wall.

By storing the seat back in the storage portion provided in the upper wall, the seat back can be easily stored, and the interior space can be used spaciously.

In addition, in the above-described conveyance, it is preferable that a foot recess for inserting feet of the occupant is formed on a support surface of the mattress.

By forming the foot recess on the support surface of the mattress, the occupant can be seated in a normal seating posture by inserting and extending the feet in the foot recess.

In addition, it is preferable that the above-described conveyance further includes a filling member with which the foot recess can be filled.

By providing the filling member with which the foot recess can be filled, the mattress can be set to a bed state.

In addition, in the above-described conveyance, it is preferable that the seat back is independent of the mattress and is stored in the storage portion provided in the side wall.

By storing the seat back in the storage portion provided in the side wall, the seat back can be easily stored, and the interior space can be used spaciously.

In addition, it is preferable that the above-described conveyance further includes a battery storage portion that stores a battery, it is preferable that at least a part of the battery storage portion is disposed on the floor, and it is preferable that an upper surface of the battery storage portion disposed on the floor is located at a higher position than an upper surface of the mattress.

By disposing a part of the battery storage portion on the floor, and locating the upper surface of the battery storage portion at a higher position than the upper surface of the mattress serving as a seat cushion, the battery that is conventionally provided under the floor can be eliminated to lower the floor, and ease of getting in and out can be improved.

In addition, in the above-described conveyance, it is preferable that the battery storage portion includes a mounting portion on which an external device is mounted.

By mounting the external device on the battery storage portion, the interior space can be used effectively.

In addition, it is preferable that the above-described conveyance further includes an equipment storage portion that stores conveyance equipment, and a display device attached to the equipment storage portion via an arm portion that is extendable and retractable.

The display device can be moved closer to an operator by the arm portion, and the operator can view an image or the like on a screen at hand.

In addition, it is preferable that the above-described conveyance further includes a sensor unit that acquires biometric information of the occupant, and it is preferable that the display device is configured to be automatically movable based on the biometric information acquired by the sensor unit.

The display device can be disposed at an appropriate position using the biometric information, for example, information about the seat in which the occupant is seated or the physique of the occupant.

In addition, it is preferable that the above-described conveyance further incudes a rail provided on the floor, and a table attached to be slidable on the rail.

By providing the table that is slidable on the rail, for example, the need to provide the table on each seat (the mattress or the seat back) in which the occupant is seated may be eliminated, and the occupant who wishes to use the table is allowed to use the table by moving the table nearby. Unnecessary tables can be eliminated, and the weight of the conveyance can be reduced.

In addition, in the above-described conveyance, it is preferable that the table is detachably attached to the rail.

By detachably attaching the table to the rail, the table can be removed from the rail when the table is not in use, thereby being able to use the interior space spaciously.

In addition, in the above-described conveyance, it is preferable that an occupant protection device is provided on a leg portion of the table.

By providing the occupant protection device on the leg portion of the table, the occupants seated around the table can be protected.

In addition, it is preferable that the above-described conveyance includes a plurality of seats including the seat backs and the mattress and attached to be rotatable and movable in a sliding manner with respect to the floor; sensor units that acquire biometric information of the occupants seated in the plurality of seats; and a control unit that controls the rotation or the sliding movement of the plurality of seats. It is preferable that the control unit estimates physiques of the occupants seated in the plurality of seats, based on the biometric information acquired by the sensor units, and it is preferable that when the plurality of seats rotate, the control unit calculates amounts of the rotation and amounts of the sliding movement of the plurality of seats and an order of the movements of the plurality of seats based on the estimated physique information.

The seats can be moved at appropriate timings.

### <<Advantageous effects of invention>>

According to the present invention, since the seat back is attached to be storable in the storage portion provided in any one of the mattress, the upper wall, and the side wall, the interior space can be used spaciously by storing the seat back in the storage portion, so that it is possible to provide the conveyance with improved comfort or convenience.

In addition, since the mattress includes the storage portion that stores the coupled seat back, the seat back can be easily stored, and the interior space can be used spaciously.

In addition, by moving the seat back rearward, the seat back can be stored in the storage portion, and the interior space can be easily expanded.

In addition, by setting the support surface of the seat back and the support surface of the mattress to be flush with each other, a bed surface can be easily created.

In addition, by providing the plurality of seat backs and the storage portions corresponding to the seat backs in a single mattress, the man-hour required to assemble the mattress to the conveyance or the number of components required for a conveyance seat is reduced.

In addition, by storing the seat back in the storage portion provided in the upper wall, the seat back can be easily stored, and the interior space can be used spaciously.

In addition, by forming the foot recess on the support surface of the mattress, the occupant can be seated in a normal seating posture by inserting and extending the feet in the foot recess.

In addition, by providing the filling member with which the foot recess can be filled, the mattress can be set to a bed state.

In addition, by storing the seat back in the storage portion provided in the side wall, the seat back can be easily stored, and the interior space can be used spaciously.

In addition, by disposing a part of the battery storage portion on the floor, and locating the upper surface of the battery storage portion at a higher position than the upper surface of the mattress serving as a seat cushion, the battery that is conventionally provided under the floor can be eliminated to lower the floor, and ease of getting in and out can be improved.

In addition, by mounting the external device on the battery storage portion, the interior space can be used effectively.

In addition, the display device can be moved closer to an operator by the arm portion, and the operator can view an image or the like on the screen at hand.

In addition, the display device can be disposed at an appropriate position using the biometric information, for example, information about the seat in which the occupant is seated or the physique of the occupant.

In addition, by providing the table that is slidable on the rail, for example, the need to provide the table on each seat (the mattress or the seat back) in which the occupant is seated may be eliminated, and the occupant who wants to use the table is allowed to use the table by moving the table nearby. Unnecessary tables can be eliminated, and the weight of the conveyance can be reduced.

In addition, by detachably attaching the table to the rail, the table can be removed from the rail when the table is not in use, thereby being able to use the interior space spaciously.

In addition, by providing the occupant protection device on the leg portion of the table, the occupants seated around the table can be protected.

In addition, the control unit can calculate the amounts of the rotation and the amounts of the sliding movement of the seats and the order of the movements of the seats based on the physique information of the occupants, thereby causing the seats to move at appropriate timings.

### <<Description of embodiments>>

Hereinafter, a configuration of a conveyance according to an embodiment of the present invention will be described with reference to the drawings. However, the embodiment to be described below is provided for easy understanding of the present invention, and does not limit the present invention. Namely, the present invention isdefined by the appended claims.

In addition, in the following description, the contents regarding the materials, shapes and sizes of components such as a seat cushion and a seat back installed in the conveyance are merely one specific example, and do not limit the present invention.

Incidentally, hereinafter, a vehicle such as an electric vehicle capable of autonomous driving will be provided as one example of the conveyance, and a configuration thereof will be described. However, the present invention may also be applied to manually driven engine vehicles. In addition, the present invention is not limited to ground-traveling conveyances including wheels such as automobiles and railroad vehicles, and can also be applied to, for example, aircrafts, ships, or the like that move in environments other than the ground.

In addition, in the following description, the "front to rear direction" is a front to rear direction of the vehicle, and is a direction that coincides with a traveling direction of the vehicle when the vehicle travels. In addition, the "width direction" and the "right to left direction" are a lateral width direction and a right to left direction of the vehicle, and the "up to down direction" is an up to down direction of the vehicle, and is a direction that coincides with a vertical direction when the vehicle travels on a horizontal surface.

In addition, in the following description, when directions are described with "vehicle" added, such as "vehicle inside" and "vehicle outside", the directions indicate directions with respect to the vehicle, and when various directions are described with "seat" added, such as a "seat width direction" and a "seat height direction", the various directions indicate directions with respect to the vehicle seat installed in the vehicle.

### <<First example>>

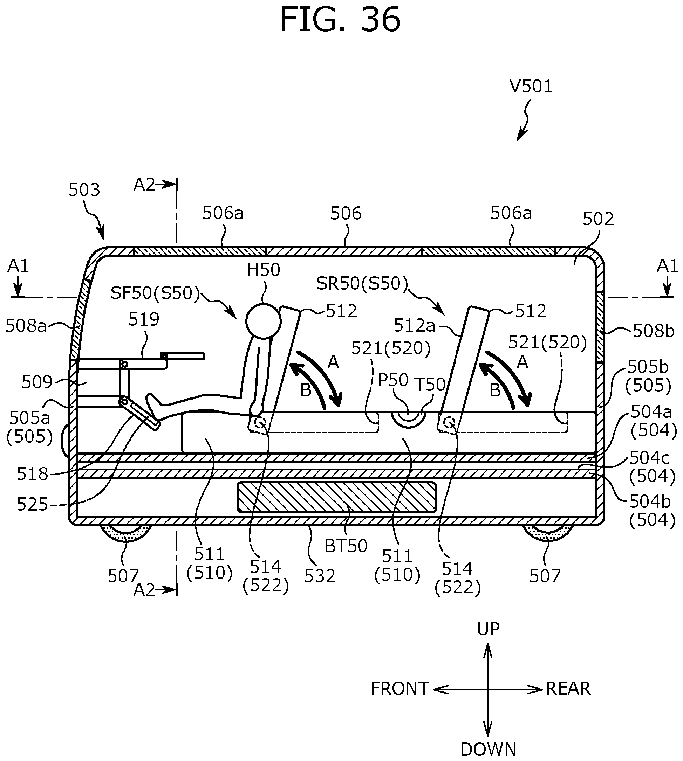

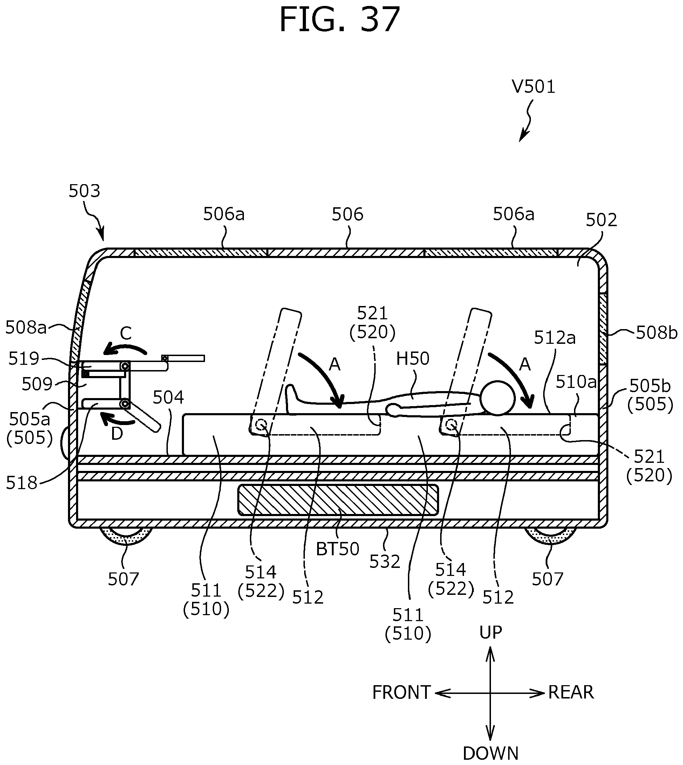

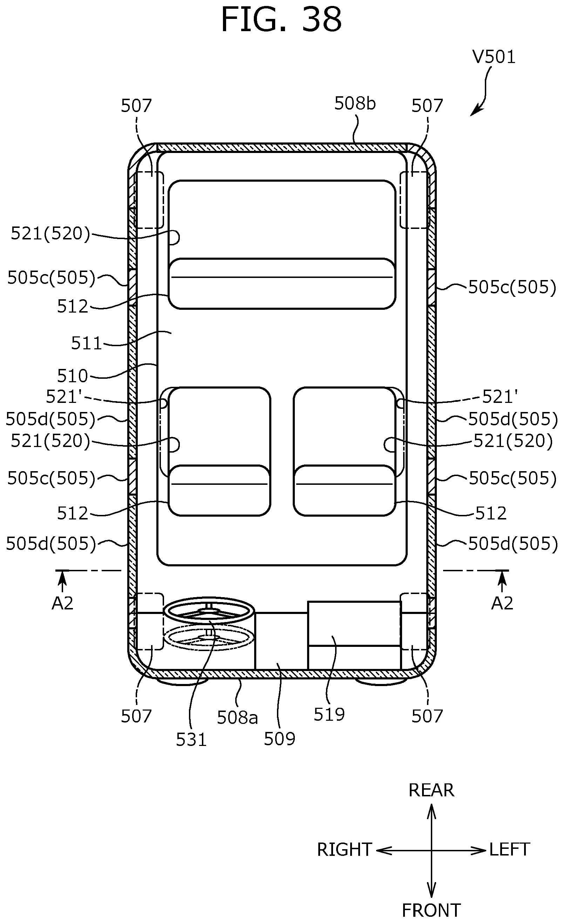

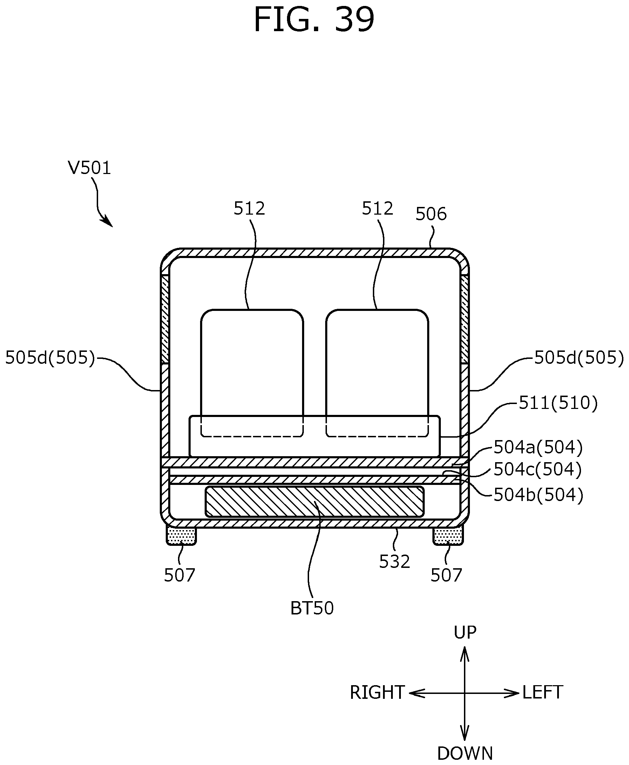

A configuration of a vehicle V501 according to a first example of the third embodiment will be described with reference to FIGS. 36 to 39. FIG. 36 is a view schematically showing the configuration of the vehicle V501, and is a cross-sectional view of the vehicle V501 when viewed from the side. FIG. 37 is a cross-sectional view showing a mat state created by storing seat backs 512. FIG. 38 is a view showing an interior configuration of the vehicle V501 when viewed from above, and FIG. 39 is a view showing an interior configuration of the vehicle V501 when viewed from the front.

As shown in FIGS. 36 to 39, the vehicle V501 includes a passenger compartment 502 that an occupant H50 enters. The passenger compartment 502 is also referred to as a cabin, and corresponds to an interior space of the present invention. The passenger compartment 502 is surrounded by a wall portion 503, and the wall portion 503 is composed of a floor 504, a side wall 505, and a roof 506 (upper wall).

The side wall 505 is specifically composed of a front portion 505a located at the front of the vehicle V501; a tailgate 505b located at the rear; and pillars 505c and doors 505d located on the sides. The door 505d is a sliding door, and can be opened and closed by sliding in the front to rear direction of the vehicle V501. The roof 506 constituting the wall portion 503 is provided with an opening/closing sunroof 506a. The tailgate 505b constituting the side wall 505 is also provided to be openable and closable.

In addition, a front window 508a is provided in the front portion 505a, as a window 508, and a rear window 508b is provided in the tailgate 505b, as the window 508.

The vehicle V501 is a four-wheeled electric vehicle, and as shown in FIG. 36, includes tires 507 at the four corners of the floor 504, and a battery BT50, which supplies electric power to a motor and the like, under the floor 504.

In more detail, the floor 504 is composed of an upper floor 504a and a lower floor 504b, and an air layer 504c is provided between the upper floor 504a and the lower floor 504b. A battery support wall 532 is provided below the lower floor 504b, and the battery BT50 is disposed between the lower floor 504b and the battery support wall 532. In addition, heat transfer from the battery BT50 is suppressed by providing the air layer 504c in the floor 504.

As shown in FIG. 36, a mattress 510 that supports at least the lower body of the occupant H50 and the seat back 512 that supports the upper body of the occupant H50 are provided on the floor 504 of the vehicle V501. Each of the mattress 510 and the seat back 512 is formed by a pad P50 and a skin T50 that covers the pad P50. Each of the seat back 512 and the mattress 510 may be provided with a frame serving as a skeleton.

A part of the mattress 510 can be used as a seat cushion 511 that supports the buttocks and the like of the occupant H50. The seat cushion 511 and the seat back 512 that is upright can be used as a vehicle seat S50 in which the occupant H50 can be seated.

The mattress 510 is provided with storage portions 520 in which the seat backs 512 are stored. Storage recesses 521 formed to substantially the same size as the seat backs 512 are formed in the storage portions 520, and the seat backs 512 can be set to a mat state by being stored in the storage recesses 521 (a bed state where the occupant H50 can lie down) .

Each of the seat backs 512 is coupled to the mattress 510 by a coupling portion 522, and can be stored in the storage recess 521. As described above, the storage recess 521 is formed as a recess having substantially the same shape as the outer shape of the seat back 512, and is configured such that the entirety of the seat back 512 fits into the storage recess 521.

More specifically, a lower end of the seat back 512 is rotatably coupled to the seat cushion 511 by the coupling portion 522. The storage recess 521 that stores the seat back 512 is provided behind the coupling portion 522, and the seat back 512 is stored in the storage recess 521 as shown in FIG. 37 by being rotated rearward (in the direction of arrow A in FIG. 36).

The seat backs 512 of the vehicle V501 are disposed in two rows in the front to rear direction, and the front seat back 512 and the seat cushion 511 located on the front side of the mattress 510 constitute a front seat SF50. In addition, the rear seat back 512 and the seat cushion 511 located in the vicinity of the center of the mattress 510 constitute a rear seat SR50. The seat backs 512 may be disposed in one row or in three or more rows.

As shown in FIG. 38, the front seat SF50 is formed to be divided into the seat back 512 on a driver's seat side and the seat back 512 on a passenger seat side, and each of the seat backs 512 is independently stored in the storage recess 521 (storage portion 520). The rear seat SR50 is configured as one seat back 512 that is long in the width direction, and is stored in the storage recess 521 provided at the rear.

When the seat backs 512 are stored in the storage recesses 521, a support surface 510a (upper surface) of the mattress 510 and support surfaces 512a (surfaces on an occupant side) of the seat backs 512 are configured to be flush with each other, namely, to be on the same horizontal plane.

By setting the support surface 510a of the mattress 510 (seat cushions 511) and the support surfaces 512a of the seat backs 512 to be flush with each other, namely, to an overall flat state, a bed surface is created, and the occupant H50 can use the mattress 510 (seat cushions 511) and the seat backs 512 as a bed mat. By simply reclining the seat backs 512 rearward and storing the seat backs 512, the passenger compartment 502 (interior space) can be easily used spaciously. In addition, since the surface area that can be used as a bed mat is expanded, discomfort felt by the occupant is suppressed, and the occupant H50 can easily take a rest or a nap.

In addition, a reclining device 514 is provided at the coupling portion 522 of each of the seat backs 512. The seat back 512 can be fixed by the reclining device 514 in a state where the seat back 512 is inclined at any angle with respect to the mattress 510. Therefore, the head of the lying occupant H50 can be raised. In addition, since the rotation angle of the seat back 512 can be adjusted by the reclining device 514, as shown in FIG. 36, the seat back 512 can be set to an upright state (use state) by being rotated around a rotation axis extending in the seat width direction.

Incidentally, in the use state, all the seat backs 512 may not be set upright. The vehicle V501 may be used with some of the seat backs 512, for example, the seat back 512 of the front seat SF50 on the driver side, upright.

When the seat back 512 is set upright, the storage recess 521 can be used as a space in which the feet of the occupant H50 seated in the rear seat SR50 are inserted (foot recess). In addition, the storage recess 521 behind the rear seat SR50 may be used as a space for accommodating luggage of the occupant H50 when the seat back 512 of the rear seat SR50 is set upright.

In addition, the storage recesses 521 shown in FIG. 38 are formed to a size that allows the seat backs 512 to be fitted thereinto; however, in consideration of an improvement in ease of getting in and out for the occupant to be seated in the rear seat, as in storage recesses 521' shown by one-dot chain lines in FIG. 38, outer side end portions of the storage recesses 521 may be extended to both right and left ends of the mattress 510. In addition, the mattress 510 may be divided into the front seat cushion 511 and the rear seat cushion 511 by extending the end portions of the storage recesses 521 to right and left end portions of the mattress 510.

The mattress 510 serving as a bed mat is disposed above the upper floor 504a, and as shown in FIGS. 36 to 38, is disposed from the front of the vehicle (a front end of the front seat SF50) to a rear end portion of the floor 504. Since the mattress 510 is disposed above the battery BT50 and substantially parallel to the battery BT50 on the upper floor 504a, the configuration makes it easy to ensure a space in the passenger compartment 502 where the occupant H50 spends her or his time.

In addition, as shown in FIG. 38, for a single mattress 510, three seat backs 512 and the storage recesses 521 corresponding to three seat backs 512 are provided. By attaching a plurality of the seat backs 512 to the single mattress 510, the man-hour required to assemble the single mattress 510 to the vehicle V501 can be reduced compared to when the mattress 510 is divided into a plurality of the seat cushions 511 and the seat backs 512 are provided for the respective seat cushions 511. In addition, the number of components is also reduced.

Incidentally, in the present example, the seat back 512 of the rear seat SR50 is formed as one member; however, similarly to the front seat SF50, the seat back may be divided into right and left portions and may be composed of two seat backs 512, or may be composed of three or more seat backs 512.

Incidentally, the mattress 510 may be formed by dividing the mattress 510 not only into front and rear portions, but also into right and left portions. The mattresses 510 may be usable by coupling the divided portions of the mattress 510 using coupling means such as linear fasteners or hook-and-loop fasteners. Dividing the mattress 510 makes it easier to insert the mattress 510 into the vehicle, and ease of assembly is improved.

### <Instrument panel 509>

As shown in FIGS. 36 to 38, an instrument panel 509 is provided at the front of the vehicle V501. A steering wheel 531 for operating the vehicle V501 is attached to the right side of the instrument panel 509. An occupant support member 518 that supports the feet of the occupant H50 is provided on the left side of the instrument panel 509.

As shown by dotted lines in FIG. 38, the steering wheel 531 is attached to be storable in the instrument panel 509.

In addition, as shown in FIG. 37, the occupant support member 518 is foldably attached to the instrument panel 509, and by deploying the occupant support member 518 toward a passenger compartment side, the occupant support member 518 can be used as an ottoman that supports the feet of the occupant H50 seated in the front seat SF50. In addition, when not necessary, the occupant support member 518 can be folded and stored in the instrument panel 509.

In addition, heating means, for example, a heater 525 may be provided on a surface of the occupant support member 518. The heater 525 can warm the feet of the occupant H50. When the occupant support member 518 is stored in the instrument panel 509, electric power consumption may be reduced by stopping the operation of the heater 525.

In addition, on the left side of the instrument panel 509, a table 519 that is foldable and storable is provided above the occupant support member 518. The occupant H50 can use the table 519 by deploying the table 519 as necessary.

Incidentally, the occupant support member 518 may be provided not only on the passenger seat side but also on the driver's seat side of the instrument panel 509. The occupant support member 518 may be provided to be storable, for example, below the steering wheel 531, and the occupant support member 518 may be able to be taken in and out as necessary. In addition, the heater 525 may also be provided on the surface of the occupant support member 518 on the driver side, as heating means.

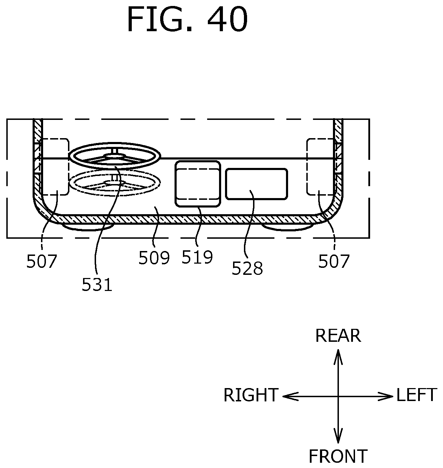

Another example of the instrument panel 509 is shown in FIG. 40. The folding type table 519 provided on the instrument panel 509 may be provided in the vicinity of the center of the instrument panel 509, and an occupant protection device 528, for example, a passenger seat airbag may be provided on the left side of the instrument panel 509.

### <Vehicle V501A>

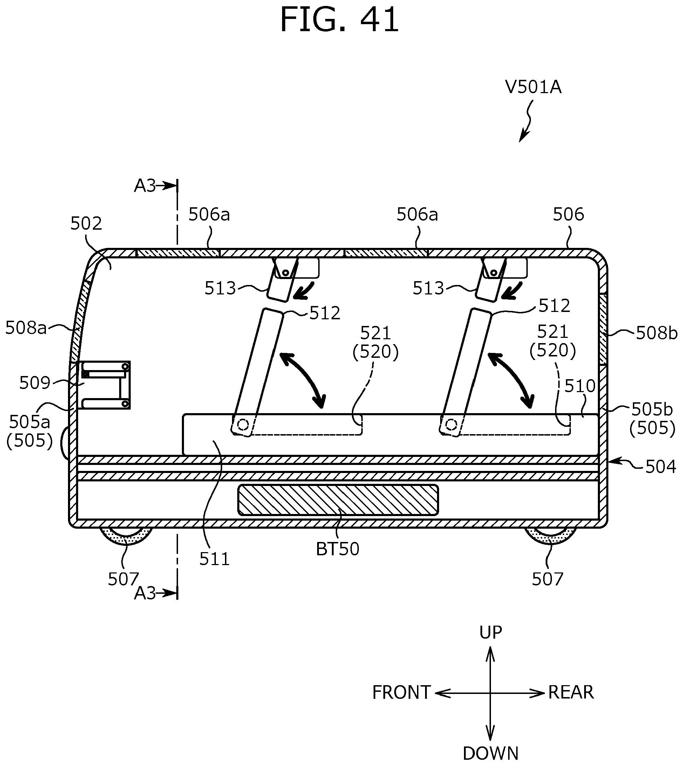

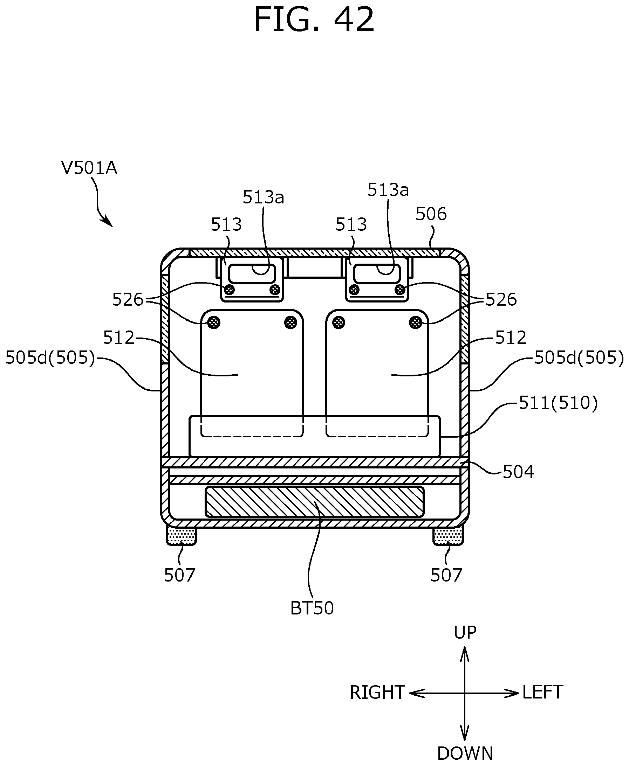

A vehicle V501A that is another example of the vehicle V501 of the first example will be described with reference to FIGS. 41 and 42. The vehicle V501A shown in FIG. 41 differs from the vehicle V501 shown in FIGS. 36 to 39 in that the vehicle V501A includes headrests 513 provided on the roof 506. The same numbers are used for configurations that are the same as or equivalent to those of the vehicle V501 shown in FIGS. 36 to 39, and descriptions thereof will be omitted.

The headrests 513 of the vehicle V501A are rotatably attached to a lower surface of the roof 506. When the seat back 512 is in an upright state, as shown by solid lines in FIG. 41, the headrest 513 can be rotated and fixed at a position where the headrest 513 hangs down, and can support the head of the occupant H50. By providing the headrest 513, the burden on the head can be reduced.

When the seat backs 512 are stored in the storage recesses 521 of the mattress 510 and are in a mat state, namely, when the seat backs 512 form a bed surface, as shown by one-dot chain lines in FIG. 41, the headrests 513 are rotated upward, and are stored in storage portions provided on the lower surface of the roof 506. Since the amount of protrusion of the headrests 513 is reduced, the passenger compartment 502 can be used spaciously.

In addition, as shown in FIG. 42, a through-hole 513a provided to penetrate through the headrest 513 in the front to rear direction when the headrest 513 is deployed downward may be provided in the headrest 513. By forming the through-hole 513a in the headrest 513, the rear of the vehicle V501A is made easily visible when a driver checks the rear. In addition, the headrest 513 can be made lighter by forming the through-hole 513a.

In addition, as shown in FIG. 42, notification devices 526 may be provided at shoulder portions of the seat back 512 or on the right and left of the headrest 513. The notification device 526 is configured by a speaker, a vibrator, a light-emitting device (display device), or a combination thereof. By providing the notification devices 526 at positions close to the head of the seated occupant, information can be reliably transmitted to the occupant.

### <Vehicle V501B>

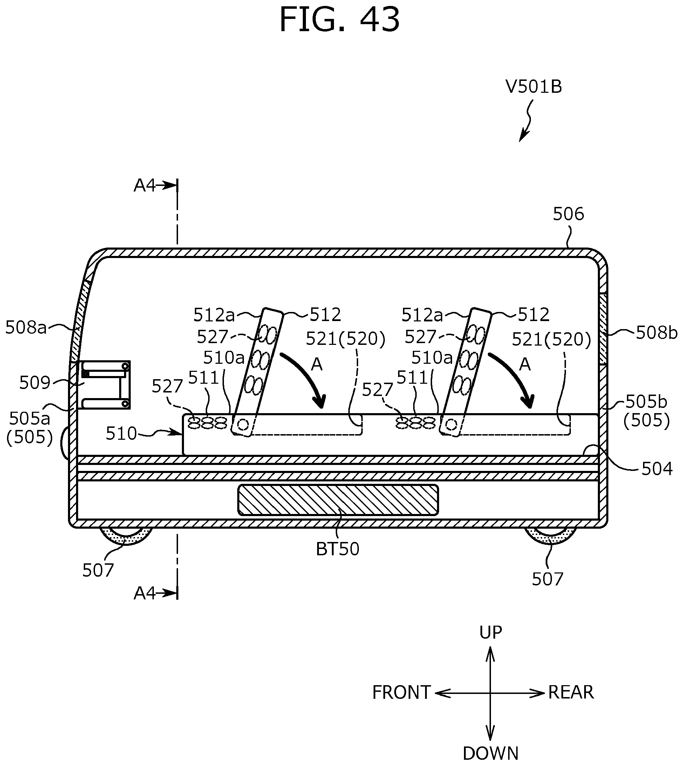

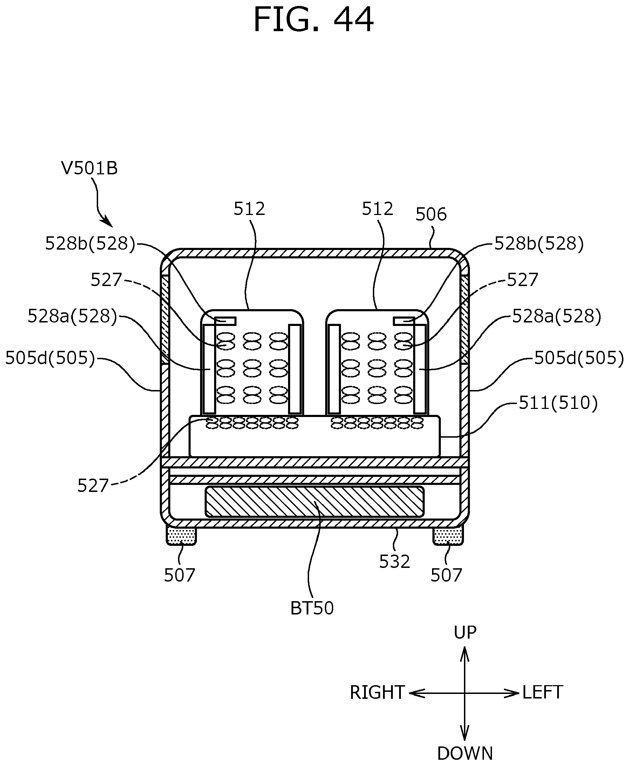

A vehicle V501B that is another example of the vehicle V501 of the first example will be described with reference to FIGS. 43 and 44. The vehicle V501B that is another example differs from the vehicle V501 in that support surface adjustment devices 527 are provided in the seat cushion 511 of the mattress 510 and the seat back 512. The same numbers are used for other components that are the same as or equivalent to the configurations of the vehicle V501 shown in FIGS. 36 to 39, and descriptions thereof will be omitted.

The support surface adjustment devices 527 are devices that adjust the shape of the support surfaces 510a and 512a that support the occupant H50, and in the present example, are composed of a plurality of air cells arranged vertically and horizontally. By changing the size of each air cell, the shape of the support surfaces 510a and 512a can be changed. When the occupant H50 is seated in the vehicle seat S50, or when the occupant H50 lies down on the vehicle seat S50 in a mat state, the support surface adjustment devices 527 deform the support surfaces 510a and 512a according to the posture of the occupant H50, so that the occupant H50 can be appropriately supported. In addition, the support surface adjustment devices 527 can also be used as massage devices that knead the body of the occupant H50.

The air cells may be disposed to overlap each other in a thickness direction of the seat cushion 511 and the seat back 512, and by disposing the air cells in two stages, the displacement in the thickness direction can be increased, and when the air cells knead the body of the occupant H50, the air cells can press the body more strongly.

Incidentally, in the present example, air cells are used as the support surface adjustment device 527, but are one example, and the support surface adjustment device 527 may be configured as a mechanical device such as a roller.

In addition, as shown in FIG. 43, side airbags 528a may be provided at both right and left side portion of the seat back 512, as the occupant protection devices 528. In addition, a belt extraction portion 528b (belt guide portion) for serving as the occupant protection device 528 and extracting a seat belt may be provided at an upper end of the seat back 512. When the seat back 512 is stored in the storage recess 521, the side airbags 528a and the belt extraction portion 528b are also stored in the storage recess 521.

### <Vehicle V501C>

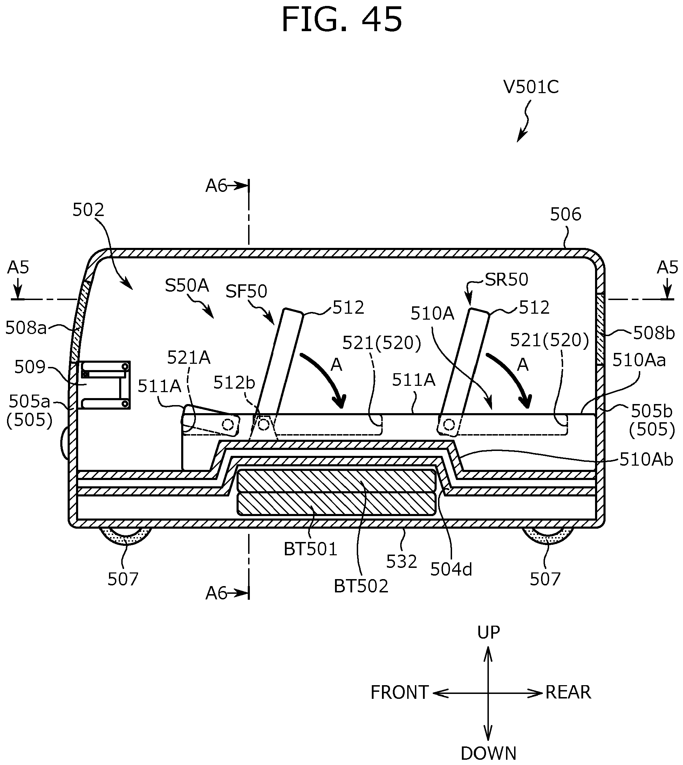

A vehicle V501C that is another example of the vehicle V501 of the first example will be described with reference to FIGS. 45 to 47. The vehicle V501C that is another example differs from the vehicle V501 in that the capacity of the battery BT50 installed under the floor is increased. The same numbers are used for other components that are the same as or equivalent to the configurations of the vehicle V501 shown in FIGS. 36 to 39, and descriptions thereof will be omitted.

As shown in FIG. 45, in the vehicle V501C, a second battery BT502 is further provided on the first battery BT501. Therefore, a battery recess 504d for storing the second battery BT502 is formed in an upper floor 504Aa and a lower floor 504Ab of a floor 504A. In other words, a protrusion protruding toward the passenger compartment side is formed in the upper floor 504Aa. In the present example, a floor clearance portion 510Ab formed in a recessed shape to store a protrusion of the upper floor 504Aa that protrudes toward the passenger compartment side is formed on a bottom surface of a mattress 510A, and consequently, an increase in the size of the mattress 510A in the height direction serving as the vehicle seat S50 is suppressed. In other words, an increase in the height of a support surface 510Aa of the mattress 510A (seat cushion 511A) that supports the lower body of the occupant H50 is suppressed. Accordingly, the storage space for the batteries BT501 and BT502 can be expanded.

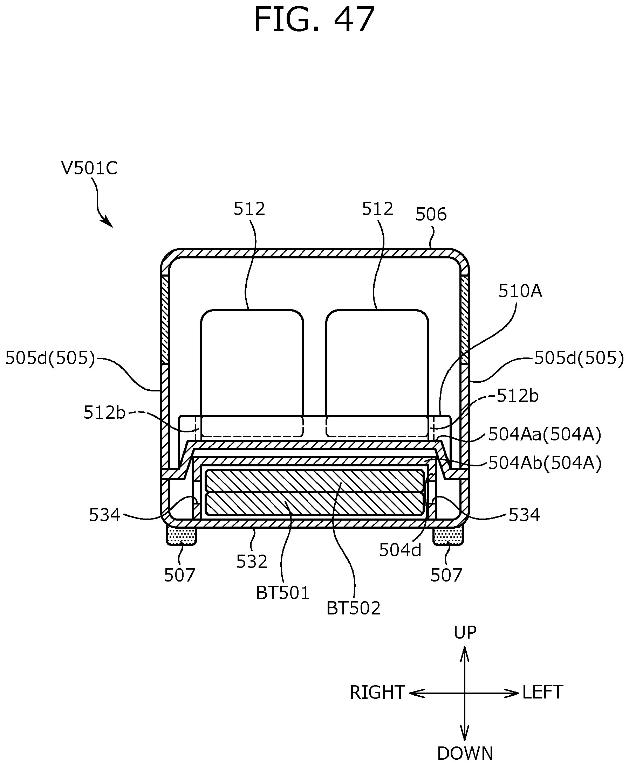

When the storage space for the batteries BT501 and BT502 is expanded, as shown in FIG. 47, ventilation holes 534 for ventilating the storage space may be formed on side surfaces of the lower floor 504Ab.

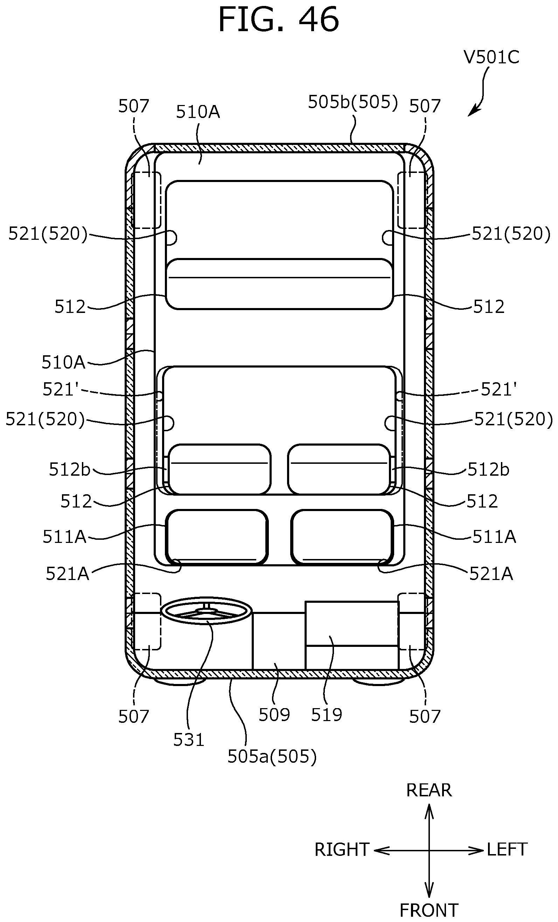

In addition, since an upper surface of the upper floor 504Aa becomes higher, as shown in FIG. 45, a lower end portion of the seat back 512 may be supported using a back support member 512b. The support rigidity of the seat back 512 is improved by using the back support member 512b. In this case, the back support members 512b may be disposed on both right and left sides of the seat back 512 to support the seat back 512. In addition, as shown in FIG. 46, only the outer side portion of the seat back 512 may be supported by the back support member 512b. In this case, only the end portions of the mattress 510A in the right to left direction are supported, and there is no high-hardness member disposed inside the mattress 510A, so that discomfort felt by the occupant H50 when lying down on the vehicle seat S50 in a mat state can be suppressed.

In addition, as shown in FIG. 45, a mechanism for lifting the seat cushion 511A that supports the lower body (mainly the thighs) of the occupant H50 may be provided in front of a vehicle seat S50A. In the present example, similarly to the seat back 512, a coupling portion that couples a rear end portion of the seat cushion 511A and the mattress 510A is provided, and the seat cushion 511A is configured to rotate around a rotation axis of the coupling portion in an upward direction. A storage recess 521A that accommodates the seat cushion 511A is formed in the mattress 510A.

By lifting the seat cushion 511A from the mattress 510A, the occupant H50 can be seated in a more natural posture.

In the vehicle V501C shown in FIG. 45, the seat cushion 511A that rises upward from the mattress 510A is provided on a front seat SF50 side; however, the seat cushion 511A that rises upward from the mattress 510A may be provided on a rear seat SR50 side.

In addition, not only the seat cushion 511A that supports the lower body of the occupant H50, but also armrests (not illustrated) capable of supporting the arms of the occupant H50 are provided to be storable in the mattress 510A or the seat back 512, and may be configured to move upward as necessary. By enabling switching between a storage state and a use state, the convenience of the vehicle seat is improved.

It is preferable that the seat cushion 511A and the armrests are formed such that, when the seat cushion 511A or the armrests are stored in the storage recess 521, similarly to the seat back 512, an upper surface of the mattress 510A is flush with an upper surface (support surface) of the seat cushion 511A and upper surfaces (support surfaces) of the armrests.

In addition, as in the storage recess 521' shown by a one-dot chain line in FIG. 46, the storage recess 521 that stores the seat backs 512 may be formed to extend to right and left side portions of the mattress 510A. The feet of the occupant H50 are easily inserted into the storage recesses 521' due to openings formed at the side portions of the mattress 510A.

### <<Second example>>

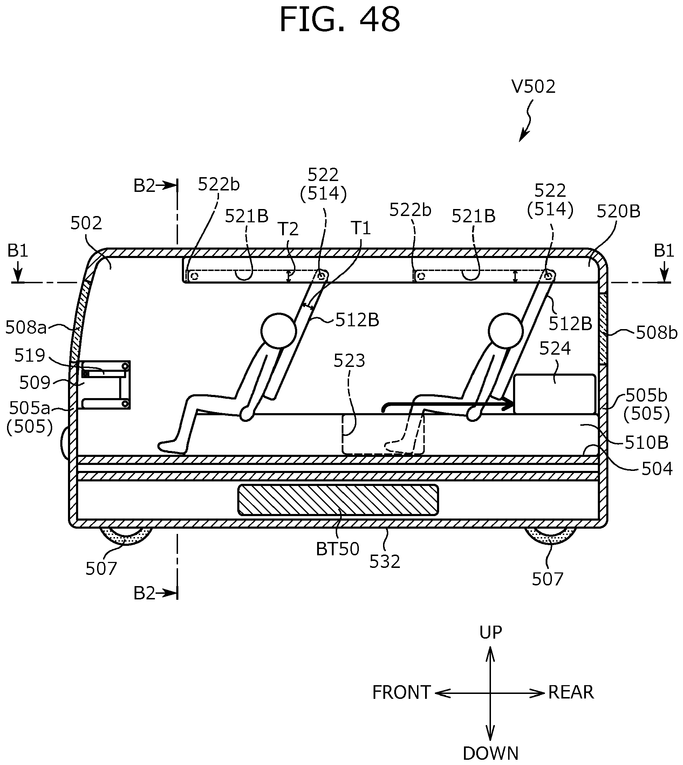

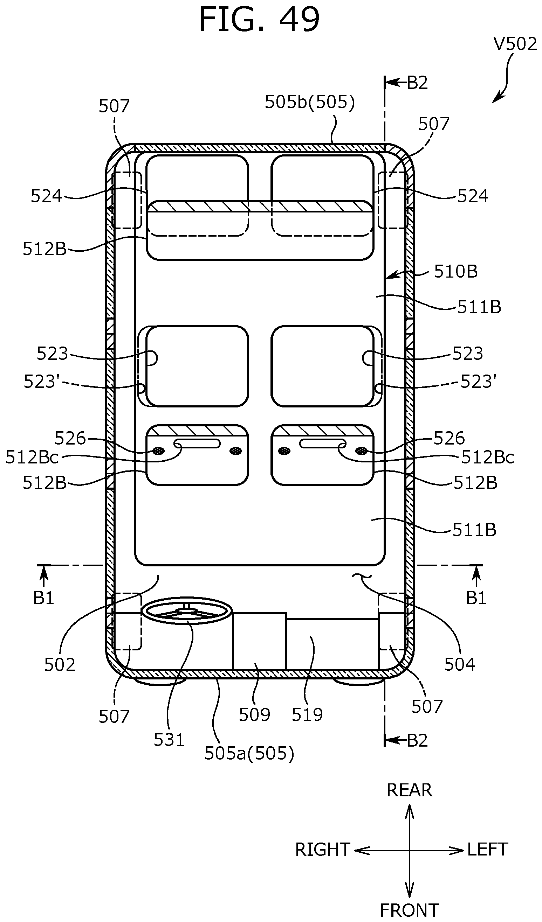

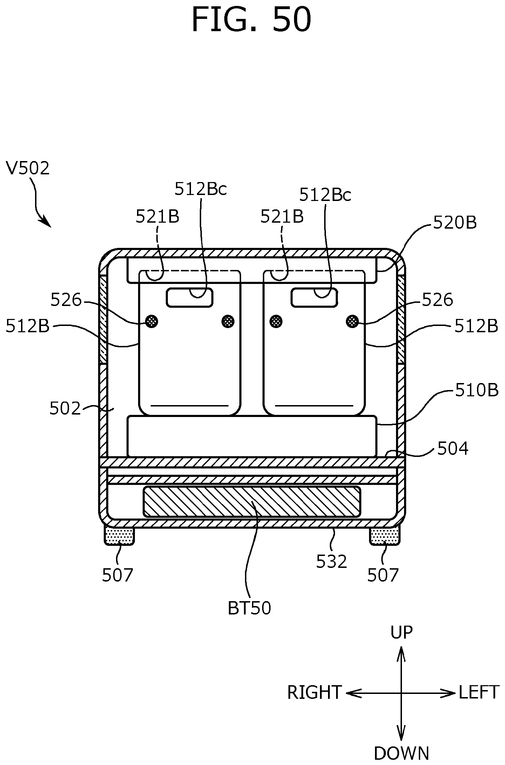

A vehicle V502 that is a second example of the present embodiment will be described with reference to FIGS. 48 to 50. FIG. 48 is a view schematically showing a configuration of the vehicle V502 of the second example, and is a cross-sectional view when viewed from the side. In addition, FIG. 49 is a cross-sectional view taken along line B1-B1 of FIG. 48, and FIG. 50 is a cross-sectional view taken along line B2-B2 of FIG. 48.

The vehicle V502 of the second example differs from the vehicle V501 of the first example in that a seat back 512B is provided to be storable in a storage portion 520B provided in the roof 506. The same numbers are used for components that are provided in the vehicle V502 of the second example and that are the same as or equivalent to the configurations of the vehicle V501 of the first example, and descriptions thereof will be omitted.

The vehicle V502 is a four-wheeled electric vehicle, and as shown in FIGS. 48 to 50, includes the passenger compartment 502 (interior space) that the occupant H50 enters. The passenger compartment 502 is surrounded by the wall portion 503, and the wall portion 503 is composed of the floor 504, the side wall 505, and the roof 506. The vehicle V502 includes the battery BT50, which supplies electric power to a motor and the like, under the floor 504.

As shown in FIG. 48, a mattress 510B that supports at least the lower body of the occupant H50 is disposed on the floor 504 of the vehicle V502. In addition, the seat back 512B that supports the upper body of the occupant H50 is attached to be storable in the storage portion 520B of the roof 506.

In more detail, a storage recess 521B having substantially the same size as the seat back 512B is formed in the storage portion 520B, and the seat back 512B is attached to be storable in the storage recess 521B.

The seat back 512B is provided to be rotatable with respect to the storage portion 520B, and is set to a usable state when the seat back 512B hangs down from the roof 506 as shown by a solid line in FIG. 48 and a lower end of the seat back 512B abuts against an upper surface (support surface 510Ba) of the mattress 510B.

The seat back 512B is provided with the reclining device 514 that adjusts the rotation angle of the seat back 512B with respect to the roof 506, and is fixed in a state where the seat back 512B is inclined at any rotation angle.

By moving the lower end of the seat back 512B in a usable state to a position where the lower end of the seat back 512B comes into contact with the upper surface (support surface 510Ba) of the mattress 510B, continuity between the seat back 512B and a seat cushion 511B of the mattress 510B is ensured, and consequently, seating comfort is improved.

When the vehicle V502 is stopped or when the vehicle V502 is in an autonomous driving mode, the mattress 510B can be set to a bed state by lifting the seat backs 512B to the roof 506 and storing the seat backs 512B in the storage portion 520B. Namely, by storing the seat backs 512B in the storage portion 520B, portions of the front seat SF50 and the rear seat SR50 that are used as seat cushions can be used as a bed mat. Since the overall flat area is expanded, it becomes easier to take a rest or a nap. In addition, since the seat backs 512B are provided on the roof 506, no storage recess may be provided in the mattress 510B on the floor 504. Therefore, the number of gaps and steps in the mattress 510B is reduced, so that discomfort felt by the occupant H50 when lying down on the mattress 510B can be suppressed.

In addition, it is preferable that a thickness T1 of the seat back 512B is equal to or smaller than a depth T2 of the storage recess 521B. When the seat back 512B is stored in the storage recess 521B of the roof 506, the seat back 512B does not protrude from the storage recess 521B, so that the appearance can be improved.

In addition, a tip portion of the seat back 512B lifted toward the roof 506 may be fixed by a lock device 522b provided in the storage portion 520B.

In addition, as shown in FIG. 50, a through-hole 512Bc penetrating through the seat back 512B in the front to rear direction is formed in the seat back 512B at a place where the head of the occupant H50 is supported. By forming the through-hole 512Bc penetrating therethrough in the front to rear direction, the driver is allowed to check the rear of the vehicle V502 through the through-hole 512Bc when seeing the rear. In addition, the weight of the seat back 512B can be reduced lighter by forming the through-hole 512Bc.

The position where the through-hole 512Bc is formed is not limited thereto, and the through-hole 512Bc may be formed at a position where the back or abdomen of the occupant H50 can be supported. Namely, the through-hole 512Bc may be formed at a central portion or a lower portion of the seat back 512B.

When the seat back 512B is in a use state, the notification devices 526 may be provided on the right and left of a place where the head of the occupant H50 is supported. The notification device 526 is a device configured by a speaker, a vibrator, a light-emitting device (display device), or a combination thereof. Since the notification devices 526 are disposed in the vicinity of the place where the head of the seated occupant H50 is located, information can be more reliably transmitted.

Incidentally, in order to improve ease of getting in and out for the occupant H50 to be seated in the rear seat SR50, a foot recess 523 into which the feet of the occupant H50 is inserted may be formed.

When the foot recess 523 is formed, a filling member 524 for filling the foot recess 523 may be provided since a step is created by the recess when the seat back 512B is lifted and stored in the storage portion 520B. The filling member 524 is, for example, a cushion member having the same size as the foot recess 523, and the mattress 510B can be set to a mat state, namely, an overall flat state by fitting the filling member 524 into the foot recess 523. When the seat back 512B is lowered from the roof 506 to be set to a use state, the filling member 524 may be extracted from the foot recess 523, and as shown in FIG. 48, the filling member 524 may be disposed behind the rear seat SR50.
as in foot recesses 523' shown by dotted lines in FIG. 49, in order to improve ease of getting in and out for the occupant to be seated in the rear seat SR50, the foot recesses 523 may extend to right and left side portions of the mattress 510B. Since openings for inserting the feet when getting in and out are formed in the right and left side portions of the mattress 510B, ease of getting in and out is improved.

Incidentally, two foot recesses 523 are formed in FIG. 49; however, one foot recess 523 created by making the two foot recesses 523 continuous with each other may be formed. In this case, the mattress 510B is divided into a front seat cushion and a rear seat cushion. In addition, in this case, the foot recess 523 may be filled with one filling member 524, or may be filled with a plurality of the divided filling members 524.

A hardness of the filling member 524 may be the same as a hardness of the mattress 510B. By setting the same hardness, it is possible to provide a bed surface that causes less discomfort to the occupant H50 in a mat state. In addition, the hardness of the filling member 524 may be different from the hardness of the mattress 510B. For example, by setting the hardness of the filling member 524 to be lower than the hardness of the mattress 510B, when the occupant H50 lies down on the mattress 510B, portions against which the lumbar, buttocks, or the like of the occupant H50 abut can be made softer than other portions, and contact feeling can be improved. In addition, the lumbar, buttocks, or the like of the occupant H50 may be able to be stably supported, conversely, by setting the hardness of the filling member 524 to be higher than the hardness of the mattress 510B.

The foot recess 523 for inserting the feet is formed in the mattress 510B disposed on the floor 504; however, with the emphasis on using the mattress 510B as a bed, a plurality of recesses may be formed and accommodate the filling members 524 of different hardness.

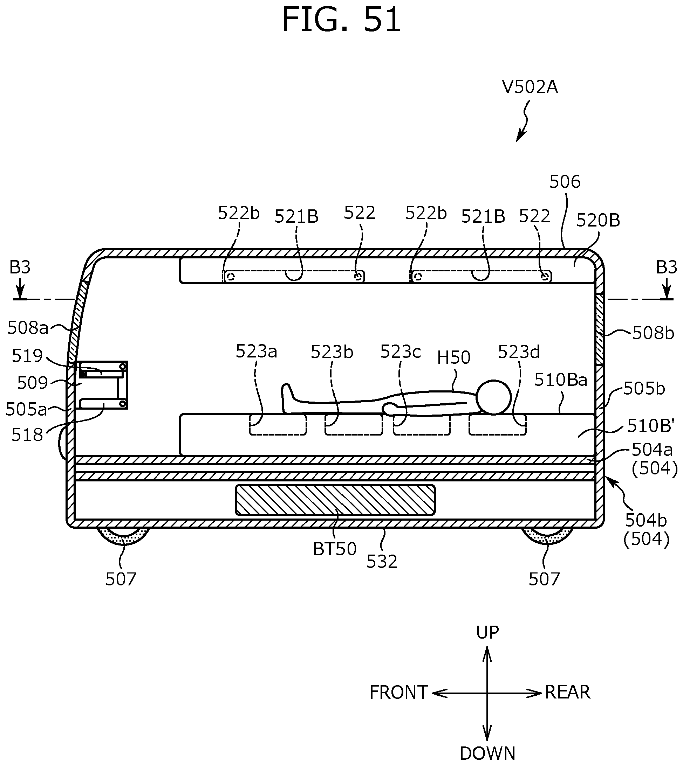

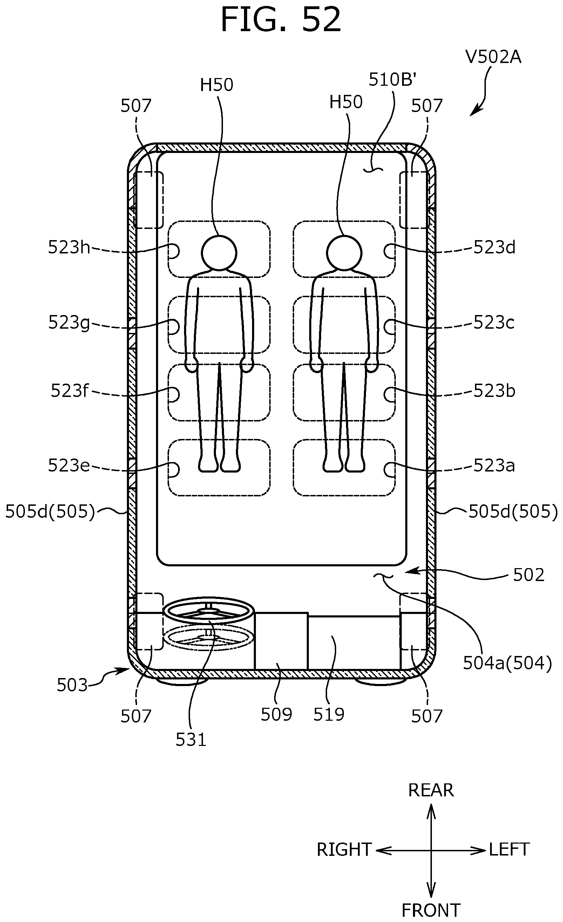

For example, as shown in FIGS. 51 and 52, a plurality of hardness adjustment recesses (a first recess 523a to an eighth recess 523h) arranged in parallel in the front to rear direction or the width direction may be formed. By storing the filling members 524 of different hardness, for example, high hardness, medium hardness, and low hardness, in the hardness adjustment recesses, a mattress 510B' having a hardness distribution according to the preference of the occupant H50 can be created.

For example, the filling member 524 with high hardness may be stored to prevent the posture from collapsing when seated. In this case, the hardness can be adjusted according to the preferences of the occupants seated in the driver's seat, the passenger seat, the rear seat, and the like. In addition, when the mattress 510B' is used as a bed, the support surface 510Ba of the mattress 510B' may be set to a hardness suitable for sleeping by storing the filling member 524 with low hardness.

### <Vehicle V502B>

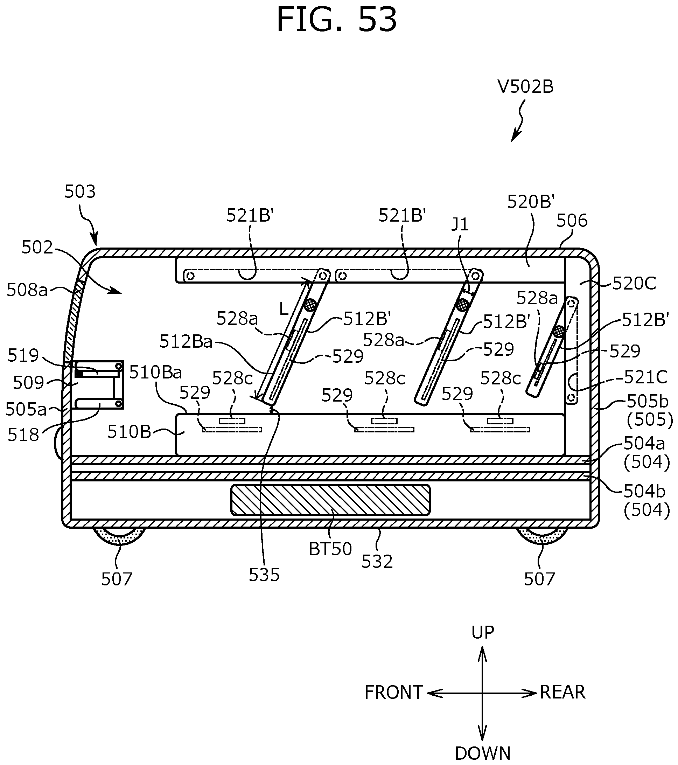

A vehicle V502B that is another example of the vehicle V502 of the second example will be described with reference to FIG. 53. The vehicle V502B that is another example differs from the vehicle V502 in that the length of the seat back 512B or equipment to be installed is different. Components that are the same as or equivalent to the components of the vehicle V501 of the first example or the vehicle V502 are denoted by the same numbers, and descriptions thereof will be omitted.

As shown in FIG. 53, the vehicle V502B includes the mattress 510B disposed on the floor 504, and seat backs 512B' attached to the roof 506. The seat backs 512B' are attached to be storable in storage recesses 521B' of a storage portion 520B' provided in the roof 506.

As shown in FIG. 53, each of the seat backs 512B' is configured such that a lower end thereof is located above the upper surface (support surface 510Ba) of the mattress 510B when in use. Therefore, a gap 535 is formed between the lower end of the seat back 512B' and the upper surface of the mattress 510B. It is preferable that the size of the gap 535 is larger than a thickness J1 of the seat back 512B'. The weight of the seat back 512B' can be reduced by shortening a length L of the seat back 512B'. Therefore, it becomes easier to raise and lower the seat back 512B'. In addition, since a support surface 512Ba that supports the lumbar or buttocks of the occupant H50 is reduced, discomfort to the occupant H50 can be suppressed.

In order to reduce the weight of the seat back 512B, a through-hole or a partially thinned recess may be formed in the seat back 512B. The through-hole or the thinned recess may be realized by removing the pad at a position that causes discomfort.

In addition, as shown in FIG. 53, the seat back 512B' may be provided not only on the roof 506, but also on the tailgate 505b. A storage portion 520C is provided in the tailgate 505b, and the seat back 512 is attached to be storable in a storage recess 521C formed in the storage portion 520C. A rotating shaft is provided at an upper end of the seat back 512B', and when used as the vehicle seat S50, the seat back 512B' is rotated toward the front of the vehicle, and is inclined to be able to support the upper body of the occupant.

As shown in FIG. 53, the side airbag 528a or the belt extraction portion 528b (seat belt) may be provided in the seat back 512B', as the occupant protection device 528. The mattress 510B may be provided with an airbag device 528c.

Frames 529 may be provided inside the mattress 510B and the seat back 512B'. In order to reduce weight, it is preferable that the frame 529 is made of resin. The support rigidity of the mattress 510B and the seat back 512B' for the occupant H50 is improved by providing the frames 529.

In addition, although not illustrated, the seat back 512B' and the mattress 510B may be provided with support surface adjustment devices that adjust the support surfaces 512Ba and 510Ba. The support surface adjustment device is composed of, for example, air cells. For example, the body of the occupant H50 can be kneaded by changing the support surfaces 512Ba and 510Ba.

In order to improve seating comfort of the occupants seated in a middle seat SM50 of a second row and the rear seat SR50 of a third row, foot recesses for occupants may be formed in the mattress 510B. To improve ease of getting in and out, it is preferable that the foot recesses may extend to the right and left side portions of the mattress 510B to form openings.

### <<Third example>>

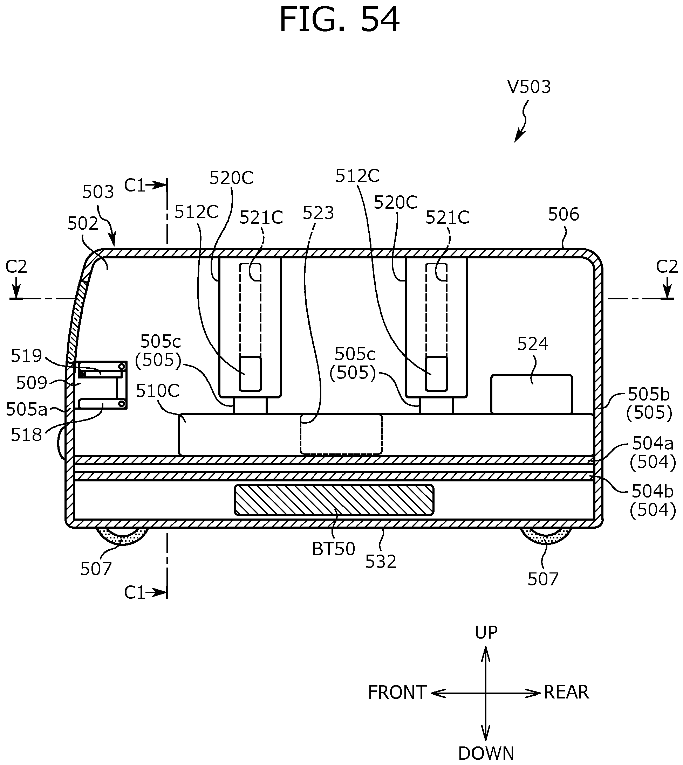

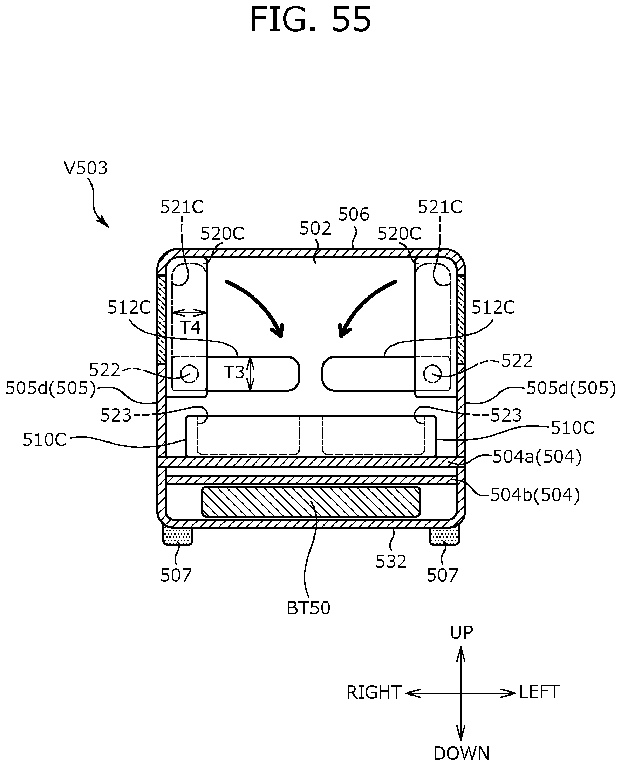

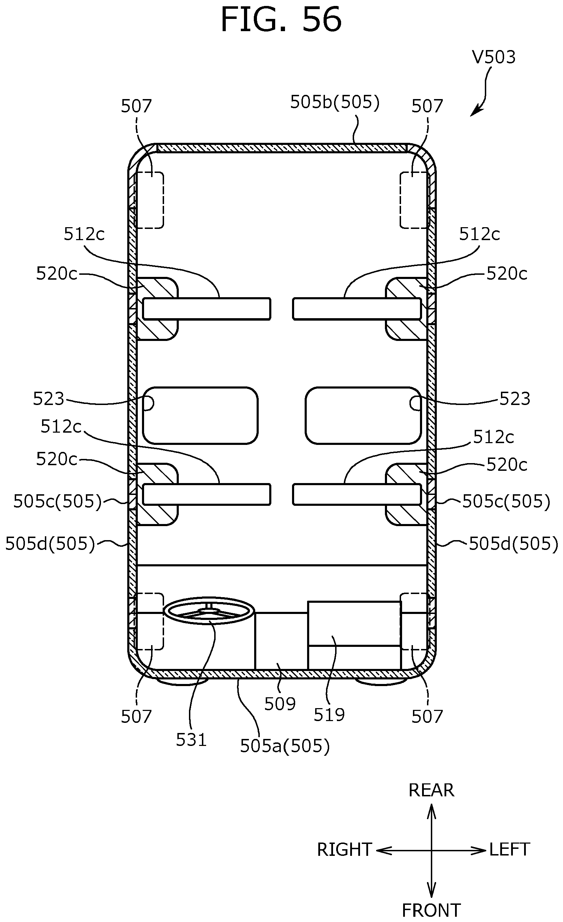

A vehicle V503 that is a third example of the present embodiment will be described with reference to FIGS. 54 to 56. FIG. 54 is a view schematically showing a configuration of the vehicle V503 of the third example, and is a cross-sectional view when viewed from the side. FIG. 55 is a cross-sectional view taken along line C1-C1 of FIG. 54, and is a view of the vehicle V503 when viewed from the front, and FIG. 56 is a cross-sectional view taken along line C2-C2 of FIG. 54, and is a view of the vehicle V503 when viewed from above.

The vehicle V503 of the third example differs from the vehicle V501 of the first example in that a seat back 512C is provided to be storable in the storage portion 520C provided in a pillar 505c of the vehicle V503. Components that are provided in the vehicle V503 of the third example and that are the same as or equivalent to the configurations of the vehicle V501 of the first example are denoted by the same numbers, and detailed descriptions thereof will be omitted.

Similarly to the vehicle V501, the vehicle V503 is a four-wheeled electric vehicle, and as shown in FIGS. 54 to 56, includes the passenger compartment 502 (interior space) that the occupant H50 enters. The passenger compartment 502 is surrounded by the wall portion 503, and the wall portion 503 is composed of the floor 504, the side wall 505, and the roof 506. The vehicle V503 includes the battery BT50, which supplies electric power to a motor and the like, under the floor 504.

As shown in FIG. 54, a mattress 510C that supports at least the lower body of the occupant H50 is provided on the floor 504 of the vehicle V503. In addition, the seat back 512C that supports the upper body of the occupant H50 is attached to be storable in the storage portion 520C provided in the pillar 505c that is a side wall. The seat back 512C is provided to be rotatable with respect to the storage portion 520C provided in the pillar 505c. When the seat back 512C is to be used, the seat back 512C is rotated and pulled out from the storage portion 520C to protrude toward the interior of the passenger compartment as shown by a solid line in FIG. 55.

The storage recess 521C that stores the seat back 512C is formed in the storage portion 520C provided in the pillar 505c. In a state where the seat back 512C is stored in the storage recess 521C, it is preferable that a thickness T3 of the seat back 512C is set to be equal to or smaller than a depth T4 of the storage recess 521C such that the seat back 512C does not protrude from the storage recess 521C.

In addition, in addition to the seat back 512C, a seat cushion, armrests, a headrest, an ottoman, and the like may be stored in the side wall, and pulled out to the passenger compartment side when in use.

By storing the seat back 512C in the storage portion 520C provided in the side wall 505 such as the pillar 515a, the interior space of the vehicle is expanded, so that the occupant H50 can use the mattress 510C disposed on the floor 504 as a bed. Since the flat portion is expanded, the occupant H50 can lie down and easily take a rest or a nap.

### <<Fourth example>>

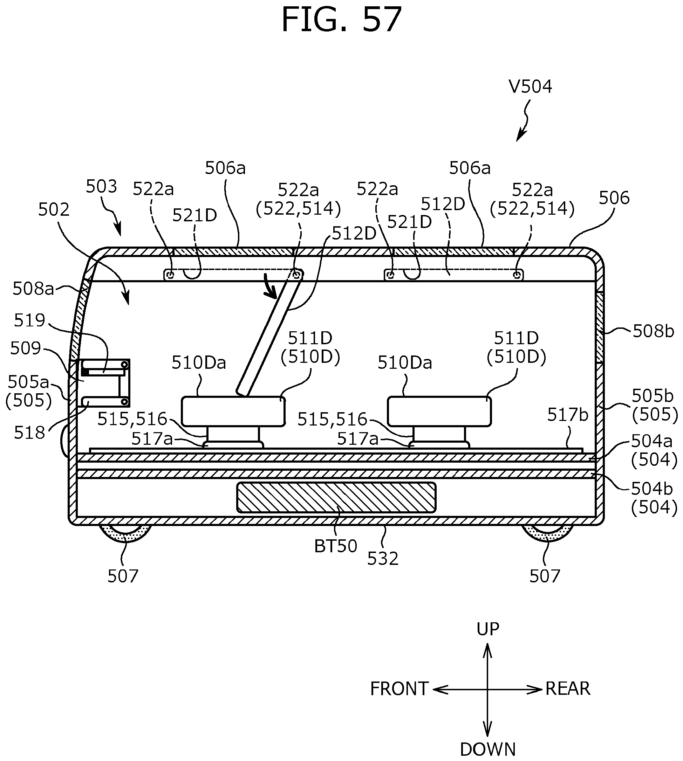

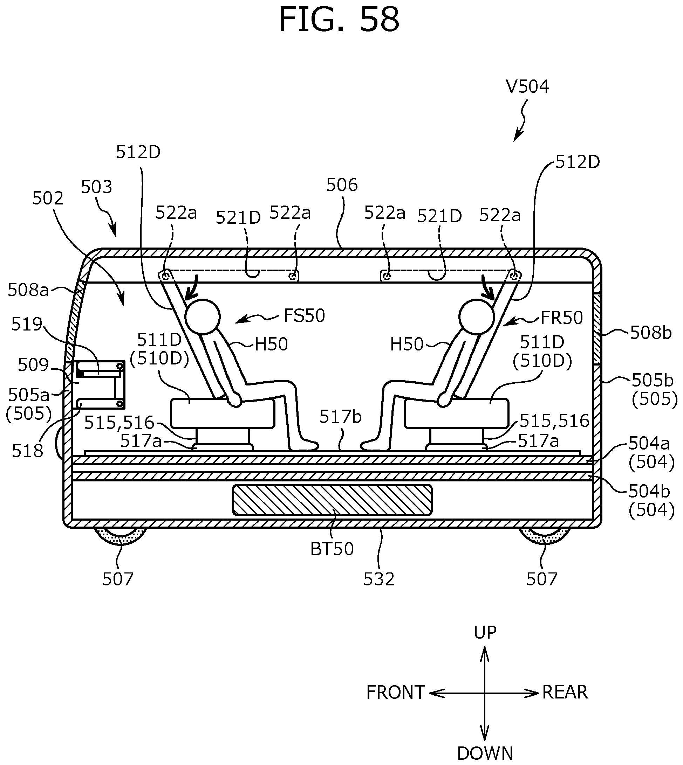

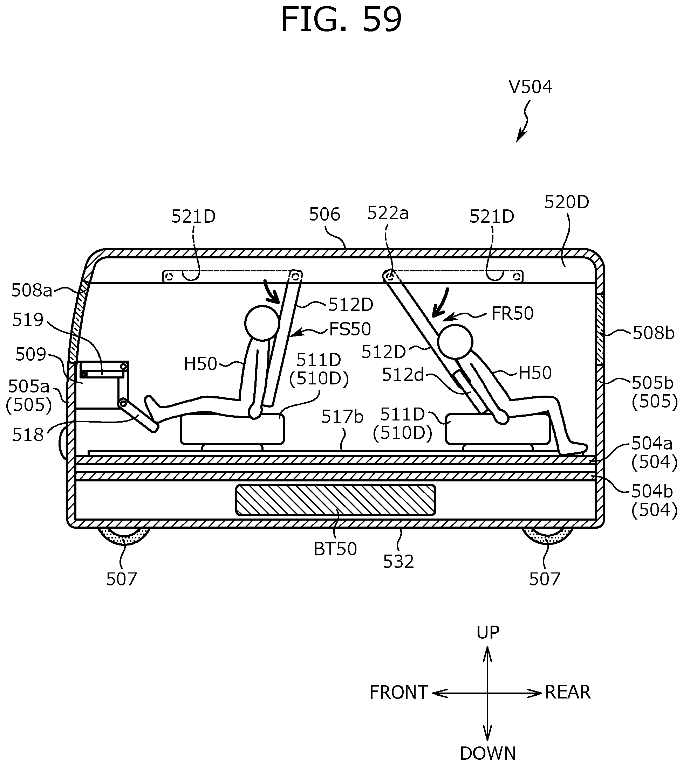

A vehicle V504 that is a fourth example of the present embodiment will be described with reference to FIGS. 57 to 60. FIG. 57 is a view schematically showing a configuration of the vehicle V504 of the fourth example, and is a cross-sectional view of the vehicle V504 when viewed from the side. FIG. 58 is a view showing a state where seats are arranged such that occupants can be seated facing each other, and FIGS. 58 and 59 are views showing other examples of the seat arrangement.

The vehicle V504 of the fourth example differs from the vehicle V502 of the second example in that a mattress 510D disposed on the floor 504 is divided and configured to be rotatable and slidable. Incidentally, in the following description, components that are the same as or equivalent to the configurations of the vehicle V502 of the second example are denoted by the same numbers, and detailed description thereof will be omitted.

The vehicle V504 is a four-wheeled electric vehicle, and as shown in FIGS. 57 to 60, includes the passenger compartment 502 (interior space) that the occupant H50 enters. The passenger compartment 502 is surrounded by the wall portion 503, and the wall portion 503 is composed of the floor 504, the side wall 505, and the roof 506. The vehicle V504 includes the battery BT50, which supplies electric power to a motor and the like, under the floor 504.

The roof 506 is provided to be movable in the up to down direction, the right to left direction, and the front to rear direction. When the roof 506 moves, a storage portion 520D that stores a seat back 512D also moves accordingly. The position of the seat back 512D can be moved in the up to down direction, the right to left direction, and the front to rear direction by moving the storage portion 520D. In addition, the roof 506 may be provided with the opening/closing sunroof 506a or the like, and when the seat back 512D is lowered and set to a use state, the sky may be visible to the seated occupant H50.

The vehicle V504 includes a plurality of the seat backs 512D that support the upper bodies of the occupants H50. The seat backs 512D are attached to be storable in the storage portion 520D provided in the roof 506. A storage recess 521D formed to substantially the same size as the outer shape of the seat back 512D is formed in the storage portion 520D. In a stored state (storage state), the seat back 512D is fitted into the storage recess 521D. The seat back 512D is provided to be rotatable with respect to the storage portion 520D, and as shown by a solid line in FIG. 57, becomes usable by hanging down from the roof 506.

In the storage state, the seat back 512D is coupled to the storage portion 520D at a front end portion or a rear end portion by the coupling portion 522. A rotating shaft 522a extending in the seat width direction is provided at the coupling portion 522. In addition, the seat back 512D is provided with the reclining device 514 that adjusts the rotation angle of the seat back 512D with respect to the roof 506, and is fixed in a state where the seat back 512D is inclined at any rotation angle.

The mattress 510D capable of supporting at least the lower body of the occupant H50 is disposed on the floor 504. A plurality of the mattresses 510D are provided with two mattresses 510D disposed on the front side and two mattresses 510D disposed on the rear side in the passenger compartment 502. The mattress 510D functions as a seat cushion 511D that supports the buttocks of the occupant H50.

Each of the mattresses 510D is provided with a seat rotating device 516 that rotates the mattress 510D around a rotation axis extending in the vertical direction, and a rail device 517 that moves the mattress 510D in the front to rear direction of the vehicle V504. The rail device 517 is composed of upper rails 517a provided on a mattress side and lower rails 517b disposed on the floor, and the mattress 510D is moved in the front to rear direction by sliding the upper rails 517a along the lower rails 517b.

The mattress 510D may further be provided with a height adjustment device 515 that changes the height of a support surface 510Da.

When the occupant H50 uses the mattress 510D for seating, the occupant H50 pulls out the seat back 512D in a storage state from the roof 506, fixes the seat back 512D at a predetermined angle, and then moves the mattress 510D to an appropriate position. At this time, the rotation angle of the seat back 512D may be adjusted automatically or manually based on the position of the mattress 510D.

Meanwhile, when the occupant H50 takes a rest or a nap, the occupant H50 creates a bed surface by lifting the seat back 512D, storing the seat back 512D in the storage portion 520D of the roof 506, and disposing the plurality of mattresses 510D in close proximity to each other.

In addition, as shown in FIG. 58, the rotating shaft 522a of the seat back 512D of the front seat SF50 may be switched to the rotating shaft 522a at the front, so that the occupant H50 can be seated facing rearward.

In addition, the height of the mattress 510D located at the rear may be changed by the height adjustment device 515 according to the size of the seated occupant H50, so that a seating posture suitable for the occupant H50 is achieved.

In addition, as shown in FIG. 59, after the positions of the seat back 512D and the mattress 510D are adjusted, when necessary, the occupant support member 518 provided on the instrument panel 509 may project to support the feet of the occupant H50 seated in the front seat SF50.

In addition, as shown in FIG. 59, the rotating shaft 522a (coupling portion 522) of the seat back 512D of the rear seat SR50 may be switched to locate the mattress 510D at a rearmost end of the passenger compartment 502. In this case, the tailgate 505b may be in an open state.

An extension portion 512d capable of adjusting the length of the seat back 512D may be provided to support the upper body of the occupant H50 at a larger inclination angle.

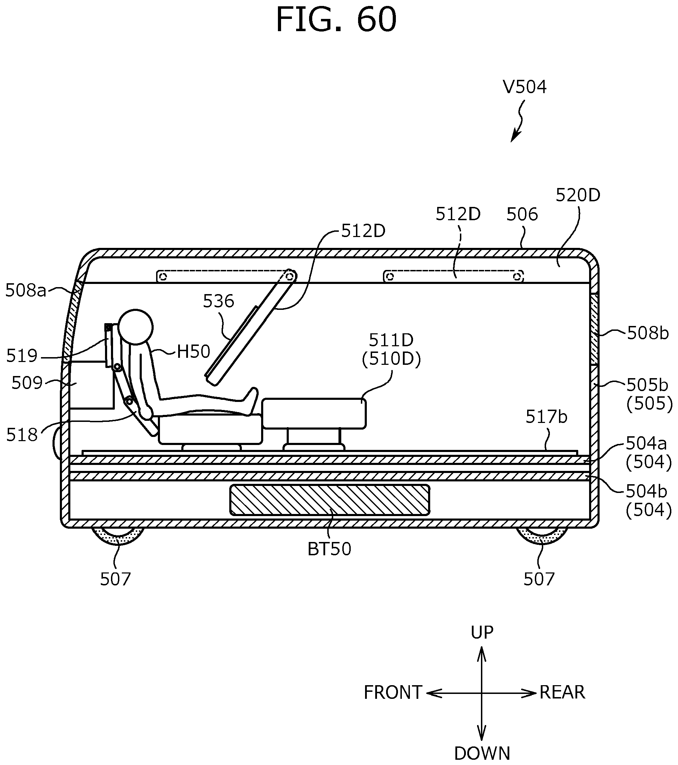

In addition, as shown in FIG. 60, a display device 536 may be detachably attached to a support surface 512Da of the seat back 512D. In order to make the attached display device 536 visible, the occupant support member 518 and the table 519 provided on the instrument panel 509 may be pulled out to support the upper body of the occupant H50. Further, by moving the mattress 510D of the rear seat SR50 forward to support the feet of the occupant H50, the occupant H50 may be allowed to appreciate an image on the display device 536 in a relaxed posture.

### <Vehicle V504A>

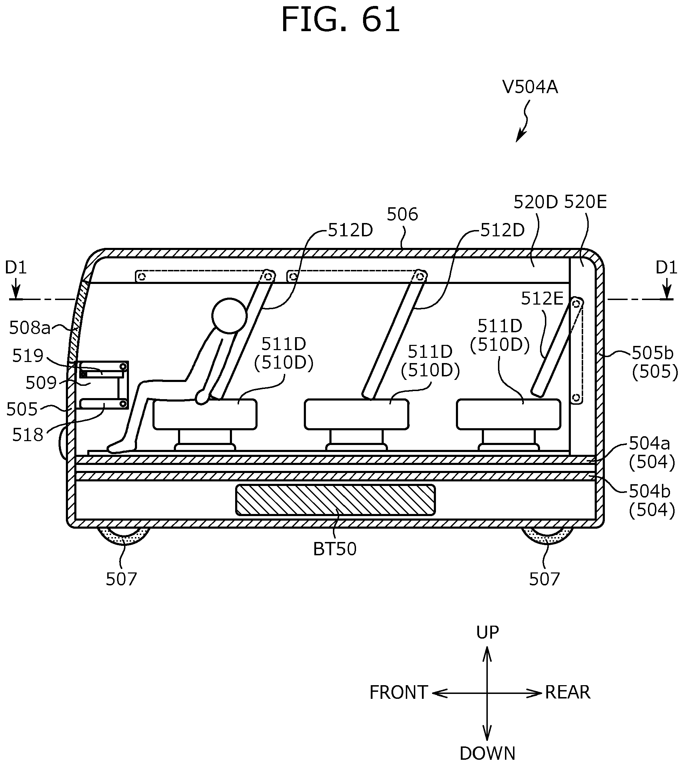

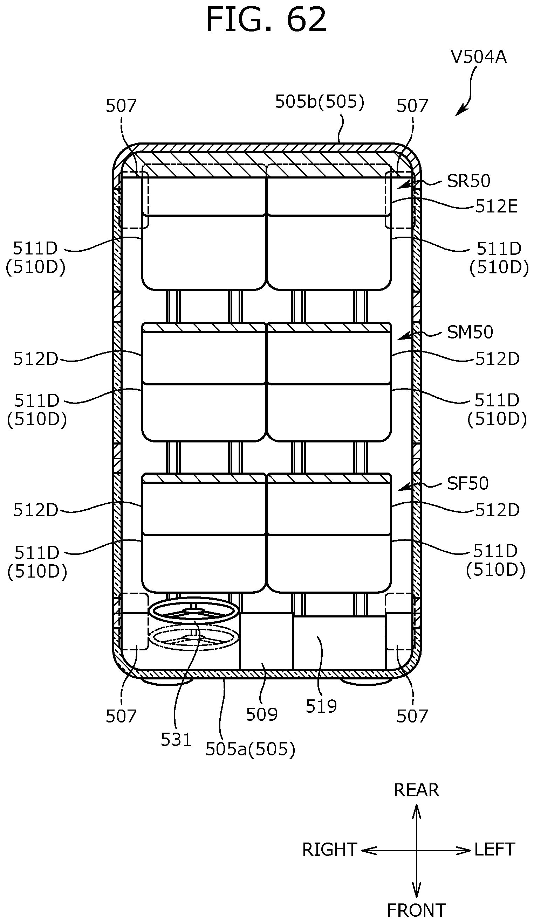

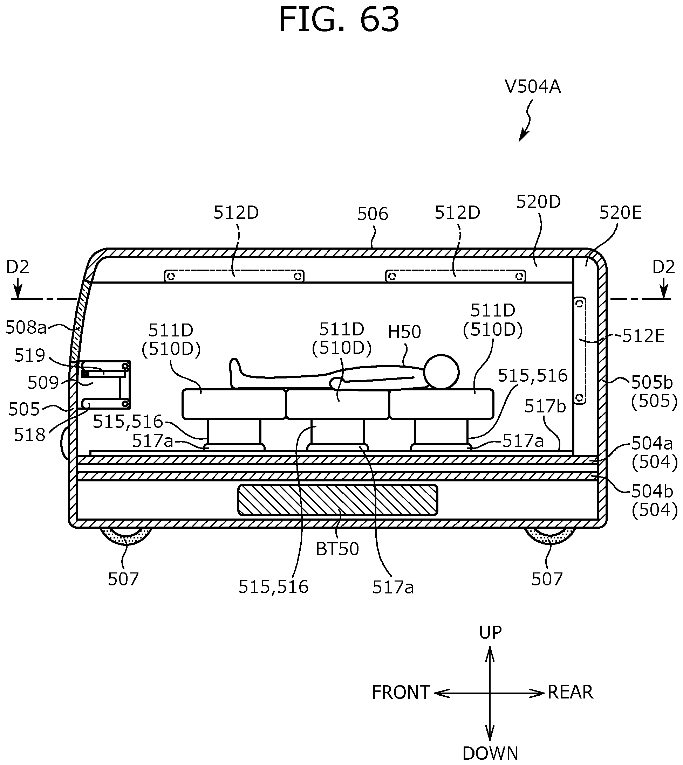

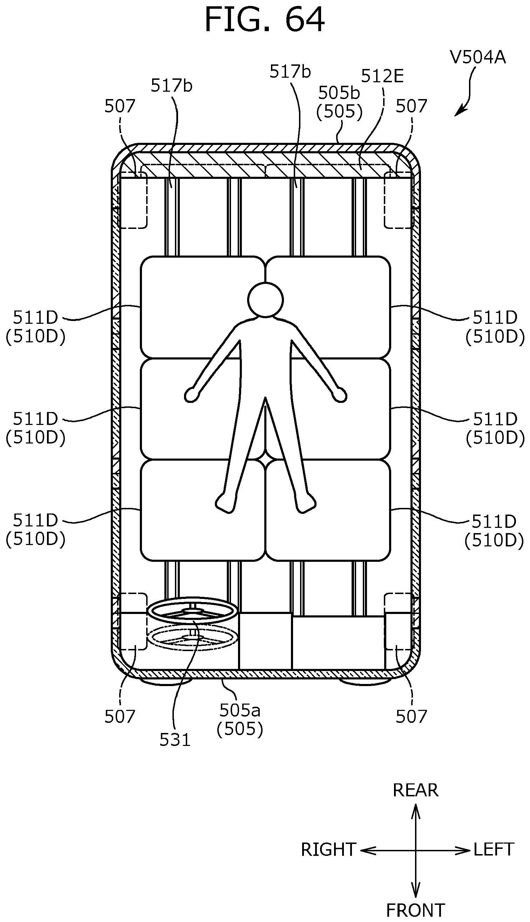

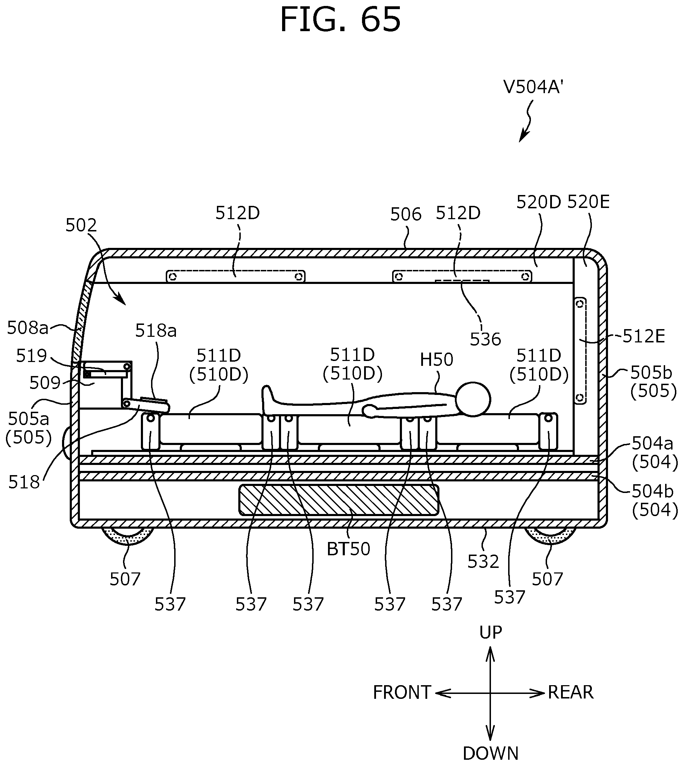

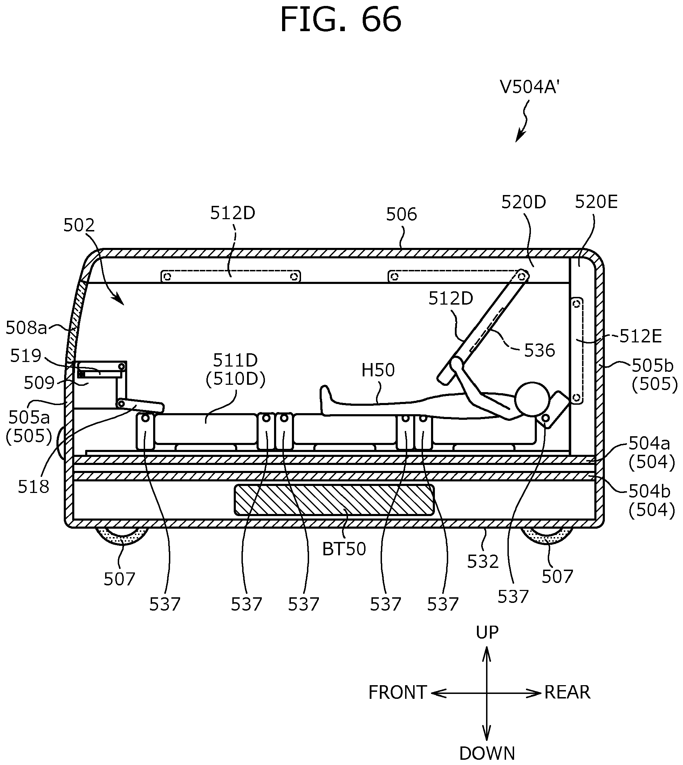

A vehicle V504A that is a modification example of the vehicle V504 of the fourth example will be described with reference to FIGS. 61 to 66. FIG. 61 is a view showing the vehicle V504A that is a modification example, and is a cross-sectional view showing a configuration of the vehicle V504A including three rows of seats, and FIG. 62 is a cross-sectional view taken along line D1-D1 of FIG. 61. FIG. 63 is a cross-sectional view showing a mat state where the seat backs 512D are stored and the mattresses 510D are brought close to each other. FIG. 64 is a cross-sectional view taken along line D2-D2 of FIG. 63. FIG. 64 is a view showing another example of the vehicle V504A, and is a cross-sectional view showing a configuration of a vehicle when viewed from the side, and FIGS. 65 and 66 are views showing other examples of seat arrangement.

The vehicle seat S50 provided in the vehicle V504 is composed of two rows of the mattresses 510D and the seat backs 512D; however, as in the vehicle V504A shown in FIGS. 61 to 64, the vehicle seat S50 may be composed of the mattresses 510D and the seat backs 512D arranged in three rows. In this case, a seat back 512E of a third row is attached to a storage portion 520E provided in the tailgate 505b. A lower end of the seat back 512E is configured to project forward from the tailgate 505b to support the upper body of the occupant H50. Other configurations that are the same as or equivalent to the configurations of the vehicle V504 shown in FIGS. 57 to 60 are denoted by the same numbers, and descriptions thereof will be omitted.

Similarly to the vehicle V504, each of the mattresses 510D is provided with the seat rotating device 516 and the rail device 517. In addition, the mattress 510D at the front serves as the seat cushion 511D, and the front seat back 512D provided in the storage portion 520D of the roof 506 is lowered, thereby forming the front seat SF50. In addition, the rear seat back 512D provided in the storage portion 520D of the roof 506 is lowered, and the mattress 510D of a second row serves as the seat cushion 511D, thereby forming the middle seat SM50. The seat back 512E of the tailgate 505b projects and the mattress 510D of the third row serves as the seat cushion 511D, thereby forming the rear seat SR50.

Similarly to the seat back 512D of the vehicle V504, each of the seat backs 512D may be provided with the extension portion 512d that extends the length of the seat back 512D. By changing the length of the seat back 512D according to the position of the mattress 510D serving as the seat cushion 511D, the upper body of the occupant H50 can be supported at an appropriate inclination angle.

As shown in FIGS. 63 and 64, a continuous bed surface can be formed by storing the seat backs 512D of the vehicle V504A in the storage portion 520D of the roof 506, adjusting the position of each of the mattresses 510D in the front to rear direction, the mattresses 510D being disposed on the floor 504, and disposing the mattresses 510D in contact with or in close proximity to each other. By providing three rows of seats, a bed surface having a wider area can be formed compared to a two-row seat arrangement. When a continuous bed surface is formed, the occupant support member 518 provided on the instrument panel 509 may be pulled out to support the feet of the occupant H50 who lies down on the bed surface.

A vehicle V504A' that is another example of the vehicle V504A will be described with reference to FIGS. 65 to 67.

As shown in FIG. 65, movable end portion support members 537 may be provided at right and left end portions of each of the mattresses 510D. When the mattress 510D is used as the vehicle seat S50 in which the occupant H50 is seated, by rotating the end portion support members 537 to be located upward, the end portion support members 537 can be used as side supports that support the lower body of the occupant H50 in the seat width direction.

Meanwhile, when the mattresses 510D are set to a mat state in order to use the passenger compartment 502 more spaciously, as shown in FIG. 65, the mattresses 510D are rotated 90 degrees such that the end portion support members 537 are located in the front to rear direction, and are further rotated such that end portions of the end portion support members 537 are located downward. In this state, a bed surface having a wider area can be formed by adjusting the positions of the mattresses 510D in the front to rear direction, and disposing the mattresses 510D in contact with or in close proximity to each other.

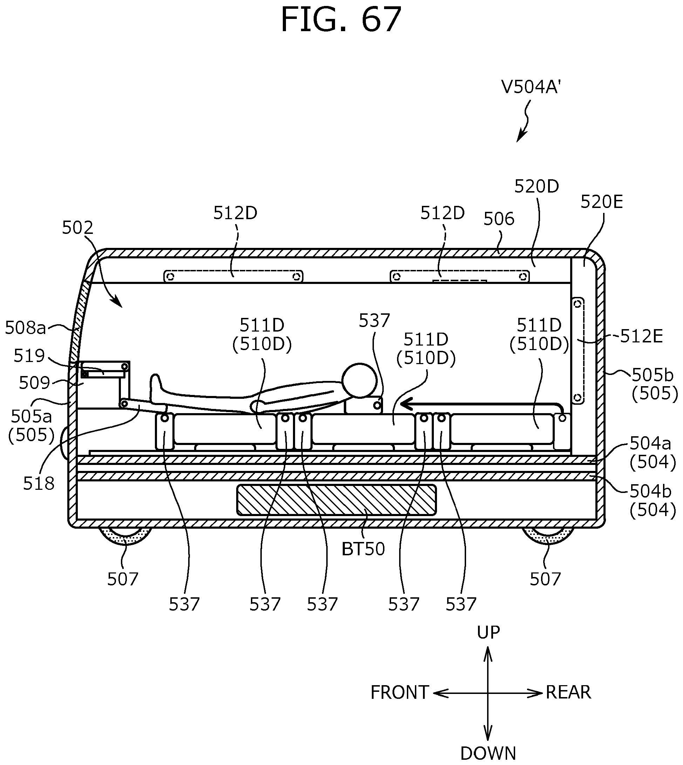

In this case, as shown in FIG. 66, the end portion support member 537 located at an end portion of the bed surface may be rotated and used as a pillow that supports the head of the lying occupant H50. In addition, as shown in FIG. 67, the end portion support member 537 may be removed from the mattress 510D and used as a pillow that supports the head of the occupant H50. In addition, the occupant support member 518 may be pulled out to support the feet. By supporting the head of the occupant H50 using the end portion support member 537, as shown in FIG. 66, the occupant H50 is allowed to view an image or the like, which is displayed on the display device 536 of the seat back 512D, at an appropriate angle.

### <Fifth example>

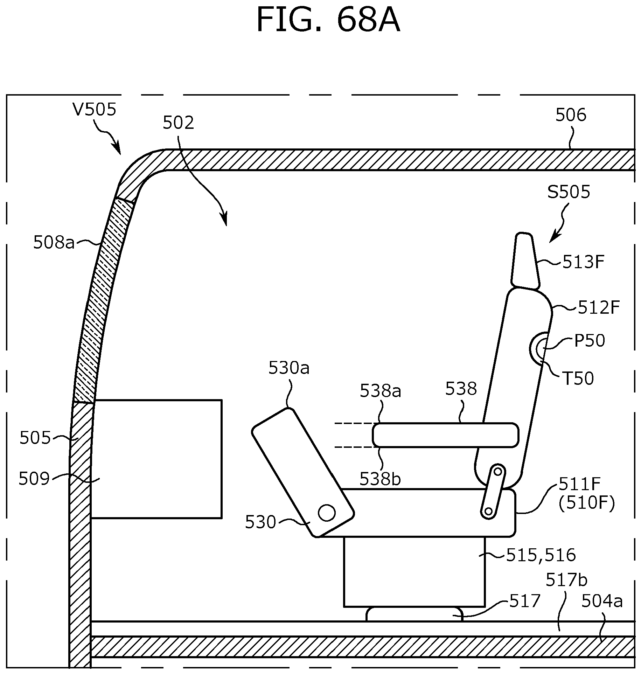

A vehicle seat S505 installed in a vehicle V505 that is a fifth example of the present embodiment will be described with reference to FIGS. 68A to 69.

Conventionally, a conveyance seat installed in a conveyance has been used not only as a seat in which the occupant H50 is seated, but also as a place where luggage of the occupant H50 is placed. However, since means for suppressing movement of the luggage is not provided at the front of the conveyance seat, the luggage may fall off the conveyance seat. Therefore, in a conveyance seat disclosed in International Publication No. 2022/092314, by rotating an ottoman upward from a horizontal position, and disposing the ottoman at an upper position, a luggage accommodation space is formed and the falling off of luggage is suppressed. However, sufficient holding force for luggage and the like may not be obtained simply moving the ottoman to the upper position above the horizontal position.

The vehicle seat S505 installed in the vehicle V505 of the fifth example of the present embodiment is composed of a seat cushion 511F that supports the lower body of the occupant H50, and a seat back 512F that supports the upper body. A lower end of the seat back 512F and a rear end of the seat cushion 511F are rotatably coupled to each other. In addition, armrests 538 that support the arms are provided at right and left side portions of the seat back 512F. An ottoman 530 that supports the feet of the occupant H50 is rotatably provided at a front end of the seat cushion 511F. Incidentally, the seat cushion 511F may be used as a mattress 510F. Each of the seat cushion 511F and the seat back 512F is composed of a pad P50 and a skin T50 that covers the pad P50. A headrest 513F is provided at an upper end of the seat back 512F. The height adjustment device 515, the seat rotating device 516, and the rail device 517 are provided below the seat cushion 511F, and can change the orientation of the vehicle seat S505 or the position and height thereof in the passenger compartment.

In the vehicle seat S505, when luggage is placed, the ottoman 530 is moved above an upper surface of the seat cushion 511F, and further, an upper end 530a of the ottoman 530 is located above lower ends 538b of the armrests 538 in a use state. Holding force for luggage and the like is improved by locating the ottoman 530 in such a manner. In addition, the vehicle seat S505 may be configured such that the position of the upper end 530a of the ottoman 530 is located above the upper ends 538a of the armrests 538.

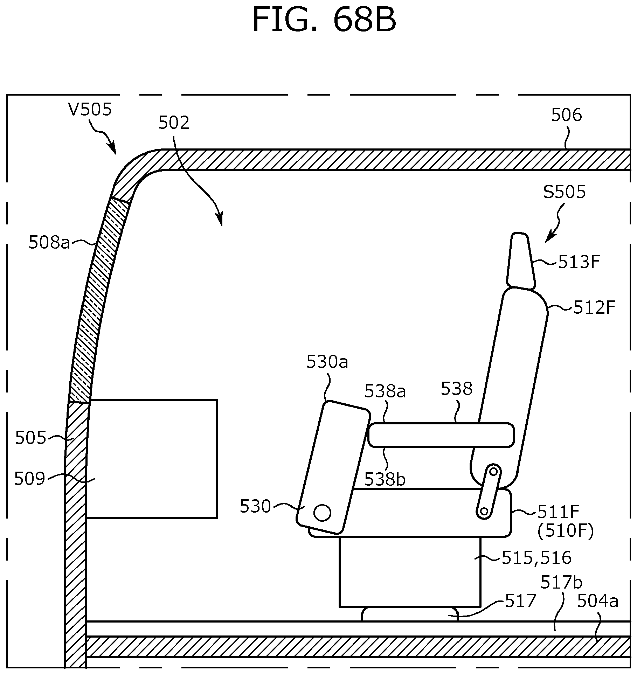

In addition, as in the vehicle seat S505 shown in FIG. 68B, in a state where the ottoman 530 is moved above the upper surface of the seat cushion 511F, the ottoman 530 may be in direct contact with and connected to the armrests 538 in a use state.

Incidentally, the ottoman 530 may be in contact with and connected to one of the right and left armrests 538. In addition, the ottoman 530 may be in contact with and connected to both the right and left armrests 538.

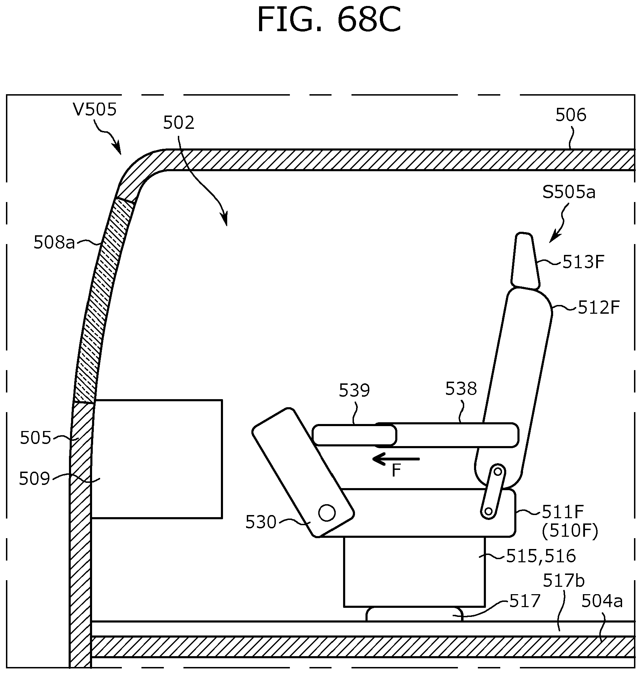

As in a vehicle seat S505a shown in FIG. 68C, the ottoman 530 that is moved upward and the armrest 538 may be configured to be connected or come into contact with each other via a movable member 539.

It is preferable that the movable member 539 is a support member or an auxiliary armrest provided at the tip of the armrest 538. In the example shown in FIG. 68C, the support member or the auxiliary armrest is attached to be movable in a sliding manner in the direction of arrow F shown in FIG. 68C along a direction in which the armrest 538 extends. The support member or the auxiliary armrest may be provided at the tip of the armrest 538 to be rotationally movable.

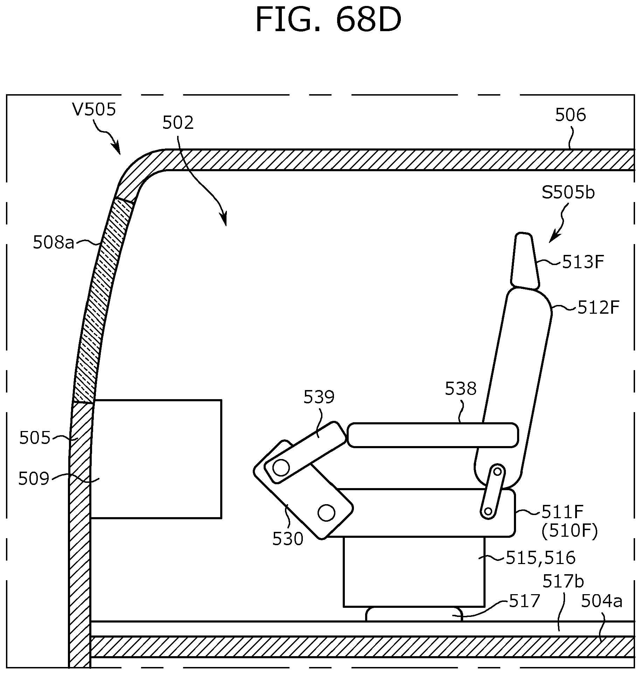

In addition, as in a vehicle seat S505b shown in FIG. 68D, the movable member 539 that connects the ottoman 530 and the armrest 538 may be a support member or an auxiliary ottoman provided in the ottoman 530.

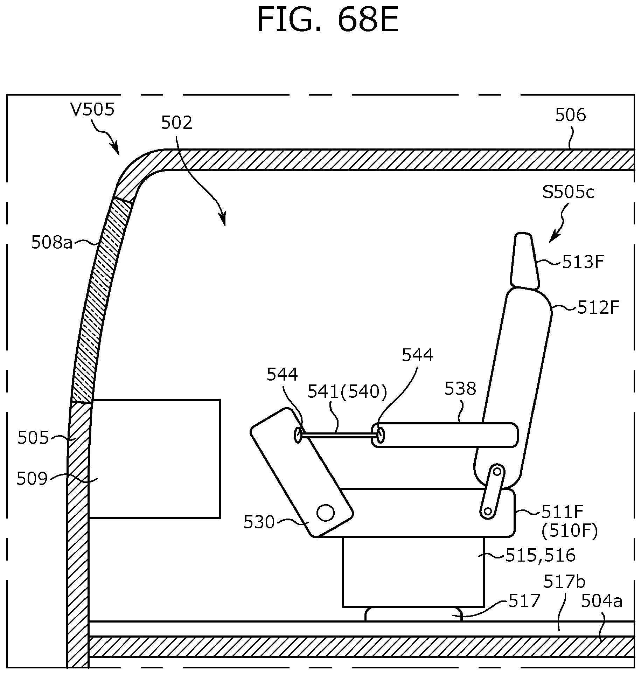

The support member or the auxiliary ottoman provided in the ottoman 530 may be provided at the tip of the ottoman 530 to be rotationally movable. In addition, the support member or the auxiliary ottoman may be provided in the ottoman 530 to be movable in a sliding manner.
as in a vehicle seat S505c shown in FIG. 68E, the armrest 538 in a use state and the ottoman 530 that is moved upward may be configured to be coupled or come into contact with each other via a coupling member 540 (connecting member) . The coupling member 540 may be, for example, a wire-shaped member 541 made of a rope, rubber, or the like.

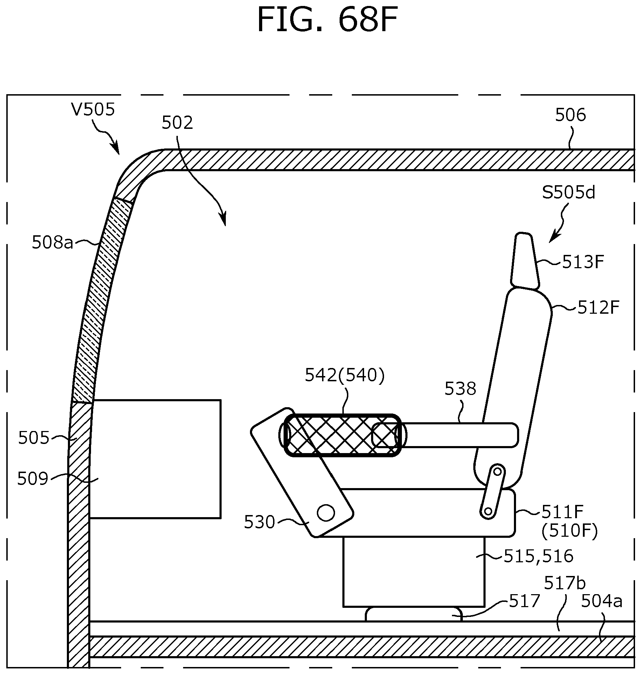

In addition, the coupling member 540 may be configured as a net-shaped member 542 as in a vehicle seat S505d shown in FIG. 68F.

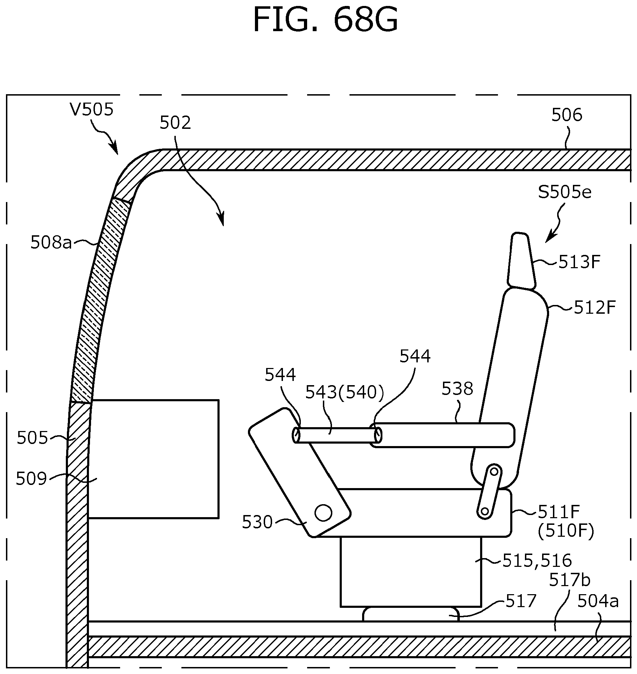

In addition, the coupling member 540 may be a planar member 543 made of fabric, leather, or the like as in a vehicle seat S505f shown in FIG. 68G. The coupling member 540 is fixed to the tip of the ottoman 530 and the tip of the armrest 538 by attachment members 544 such as clips or hook-and-loop fasteners. The ottoman 530 and the armrest 538 can be coupled to each other with a simple configuration.

The coupling member 540 may be provided not only to couple or connect the ottoman 530 and the armrest 538, but also to couple or connect the armrest 538 to the seat cushion 511F or the seat back 512F. In addition, the coupling member 540 may be provided to couple or connect the ottoman 530 to the seat cushion 511F or the seat back 512F.

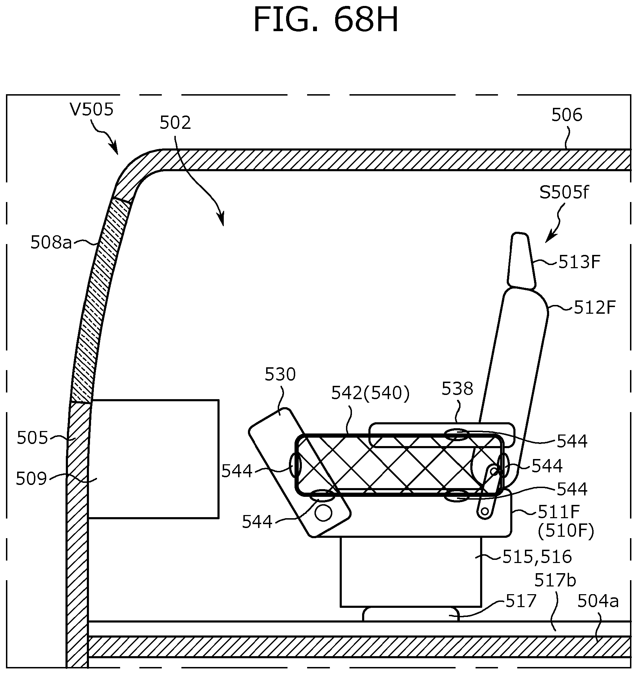

For example, as in a vehicle seat S505g shown in FIG. 68H, the coupling member 540 may couple or connect the ottoman 530, the armrest 538, the seat cushion 511F, and the seat back 512F. In this case, it is desirable that the coupling member 540 is configured by a net-shaped member 542 or a planar member 543 such as fabric. The coupling member 540 is fixed to a side portion of the seat cushion 511F and the seat back 512F by the attachment members 544 such as clips. The holding force for luggage and the like is further improved by providing the coupling member 540 in such a manner.

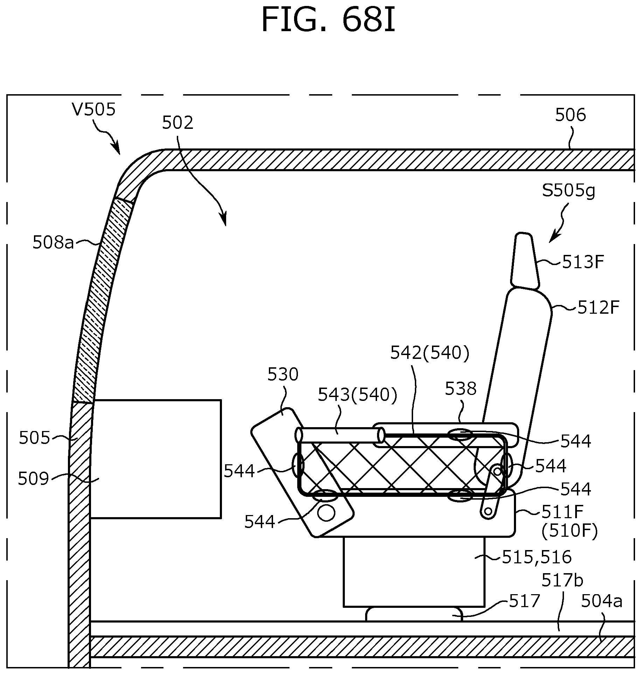

As in a vehicle seat S505g shown in FIG. 68I, a plurality of the coupling members 540 may cover a side portion of the vehicle seat S505g. The ottoman 530 and the tip of the armrest 538 are coupled to each other by the wire-shaped member 541 that is the coupling member 540. In addition, a side opening portion surrounded by the seat back 512F, the armrest 538, the seat cushion 511F, and the ottoman 530 are configured to be covered by the planar member 543 or the net-shaped member 542.

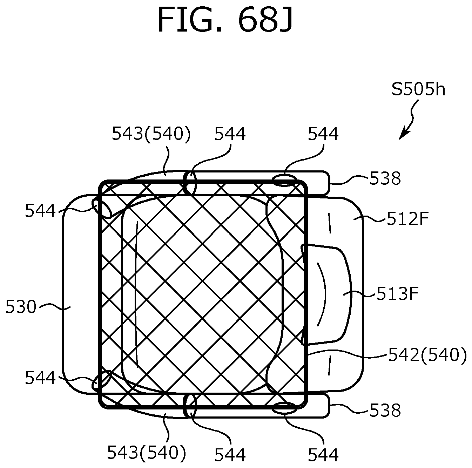

In addition, as in a vehicle seat S505h shown in FIG. 68J, the right and left armrests 538 may be coupled to each other using the net-shaped member 542 that is the coupling member 540. The coupling member 540 may be the planar member 543. In this case, the tips of the armrests 538 and the ottoman 530 may be coupled or connected to each other by the wire-shaped members 541. When the net-shaped member 542 or the planar member 543 is used as the coupling member 540, the holding force can be improved over a wider area. In addition, the wire-shaped members 541 and the planar member 543 may be integrally formed.

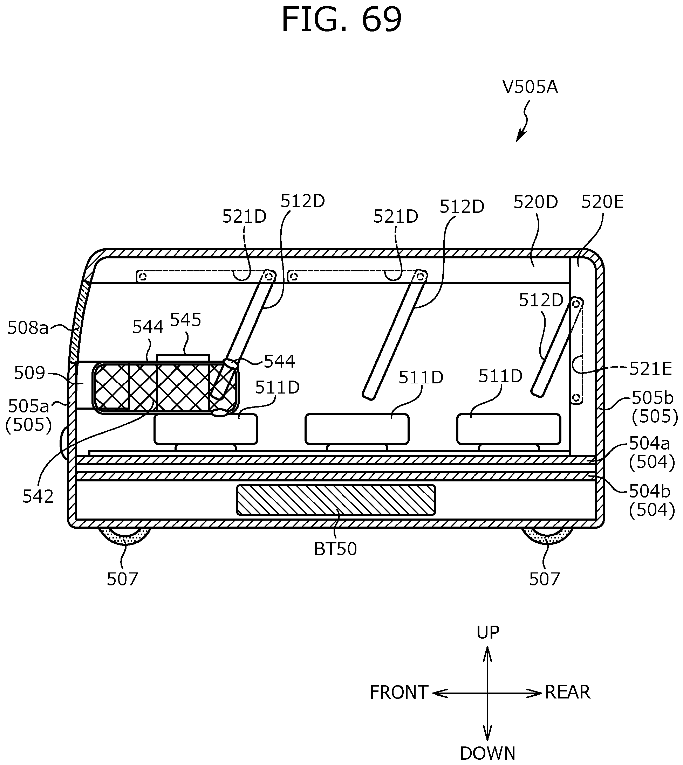

In addition, as in a vehicle V505A shown in FIG. 69, the instrument panel 509 and the seat back 512D and the seat cushion 511D of the front seat SF50 may be coupled or connected to each other by the net-shaped member 542. The planar member 543 may be used in place of the net-shaped member 542. Since the coupling member 540 functions as a luggage holding member 546 that holds luggage 545, the falling off of the luggage 545 from the side portion of the front seat SF50 is suppressed, and the holding force for the luggage 545 and the like is improved.

The coupling member 540 such as the net-shaped member 542 may be stored in the instrument panel 509 and may be pulled out from the instrument panel 509 when in use.

### <Sixth example>

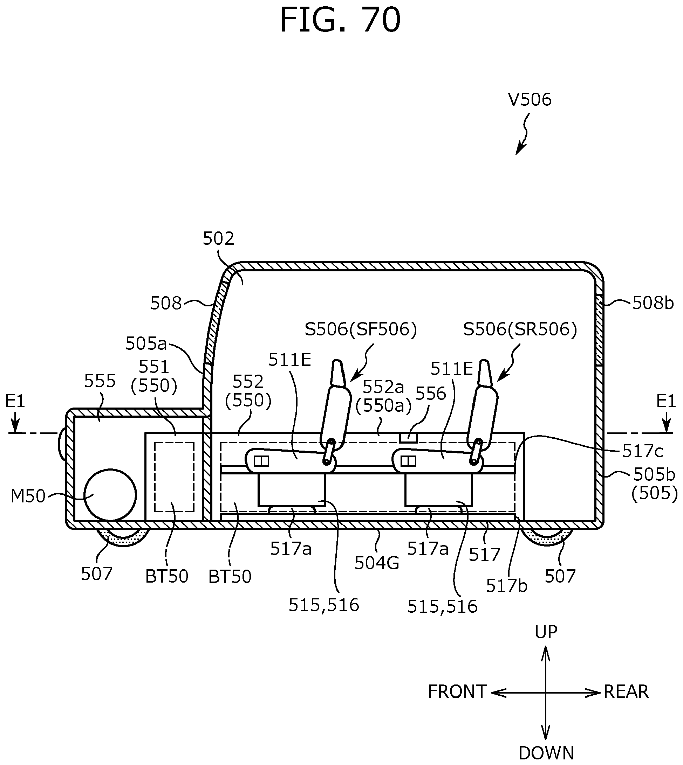

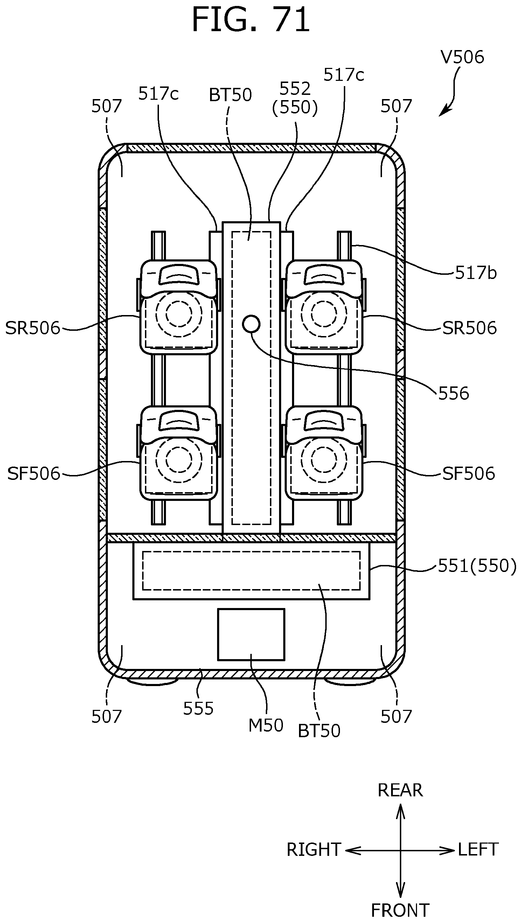

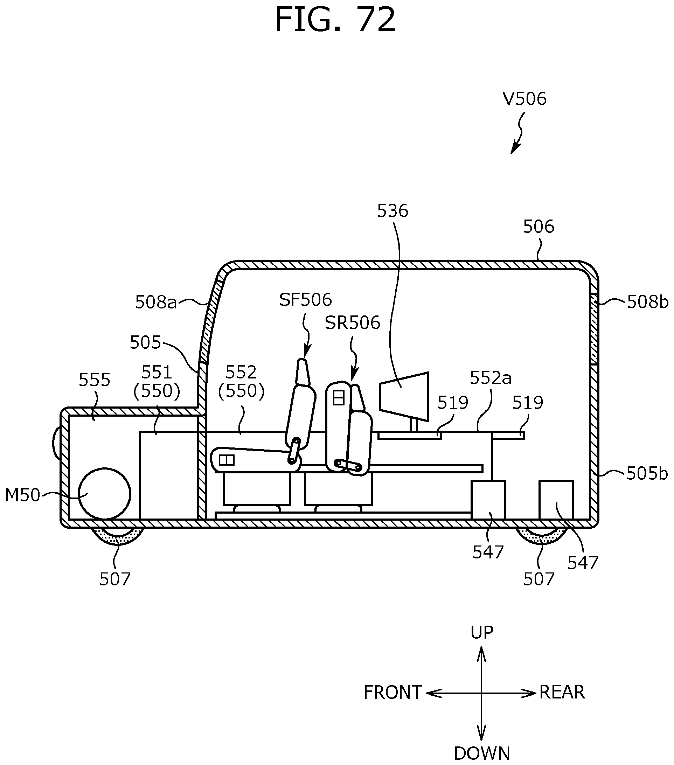

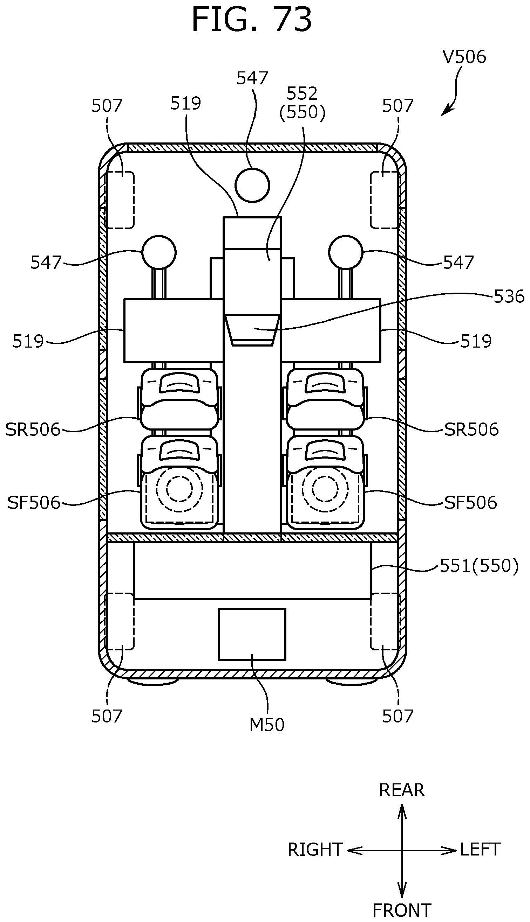

A vehicle V506 that is a sixth example of the present embodiment will be described with reference to FIGS. 70 to 73. FIG. 70 is a view schematically showing a configuration of the vehicle V506 of the sixth example, and is a view of the vehicle V506 when viewed from the side. FIG. 71 is a cross-sectional view taken along line E1-E1 of FIG. 70, and is a view of the vehicle V506 when viewed from above. FIG. 72 is a view showing another example of a seat arrangement, and is a view of the vehicle V506 when viewed from the side, and FIG. 73 is a view of the vehicle V506 when viewed from above.

In recent years, there has been a growing desire to more actively use a passenger compartment of an automobile as a room where an individual spends her or his time. In addition, since occupants enter and exit the passenger compartment frequently, ease of getting in and out is also required.

Meanwhile, in an electric vehicle, since there is also a desire to install more batteries, when the batteries are installed under a floor, the floor becomes higher and it becomes difficult to get in and out, which is a problem.

The vehicle V506 of the sixth example includes a battery storage portion 550 that stores the battery BT50, and at least a part of the battery storage portion 550 is disposed on the floor of the vehicle V506. In addition, an upper surface 552a of the battery storage portion 550 disposed on the floor is located higher than an upper surface of a seat cushion 511E of a vehicle seat S50 installed inside the vehicle.

Since the battery BT50 is not disposed under the floor, the height of the floor 504 can be lowered, and it becomes easier to get in and out.

The vehicle V506 is an electric vehicle, and as shown in FIG. 70, includes the passenger compartment 502 in which vehicle seats S506 are installed; a motor compartment 555 which is provided at the front of the passenger compartment 502 and in which a motor M50 and the like are installed; and the tires 507 disposed at four corners of the vehicle body. Conventionally, the battery that supplies electric power to the motor M50 is often disposed under the floor of the passenger compartment, and therefore, the floor is provided at a high position.

The vehicle V506 of the sixth example includes the battery storage portion 550 that stores the battery BT50. In addition, as shown in FIG. 70, the battery storage portion 550 is composed of a first battery storage portion 551 disposed in the motor compartment 555, and a second battery storage portion 552 disposed mainly in the passenger compartment 502. The first battery storage portion 551 is disposed such that a major axis direction of the first battery storage portion 551 is aligned with the right to left direction (width direction) of the vehicle V506. In addition, the second battery storage portion 552 is disposed such that a major axis direction of the second battery storage portion 552 is aligned with the front to rear direction of the vehicle V506. Therefore, the battery storage portion 550 is formed in a T-shape as a whole when viewed from above.

As shown in FIG. 70, the second battery storage portion 552 is disposed above the floor 504G in the passenger compartment. In addition, the upper surface 552a of the second battery storage portion 552 is formed to be located higher than the upper surface of the seat cushion 511E. In other words, a large center tunnel is provided at a center portion of the passenger compartment 502, and the battery BT50 is disposed inside the center tunnel.

Since the battery is not disposed under the floor, the floor can be made low. In addition, since the battery storage portion 550 has high rigidity, the rigidity of the vehicle body can be increased by disposing the battery storage portion 550 at the center of the passenger compartment.

A plurality of the vehicle seats S506 (front seats SF506 and rear seats SR506) are installed in the passenger compartment 502 of the vehicle V506. As shown in FIG. 70, the vehicle seats S506 are disposed on the right and left of the second battery storage portion 552, and are movable in the front to rear direction. The movement in the front to rear direction is realized by the rail device 517 provided in each of the vehicle seats S506. The rail device 517 is composed of upper rails 517a provided on the seat side, and a lower rail 517b and a side rail 517c along which the upper rails 517a slide. The lower rail 517b is an outer slide rail, and is disposed on the floor on the outer side of the vehicle. In addition, the side rail 517c is an inner slide rail, and is disposed on a side surface of the second battery storage portion 552. The seat rigidity of the vehicle seats S506 can be increased by disposing the side rail 517c on the side surface of the second battery storage portion 552.

The second battery storage portion 552 may be divided such that a space through which a person can pass is formed in the middle thereof. The occupant H50 can easily move to an opposite seat.

In addition, the battery BT50 disposed inside the second battery storage portion 552 may be allowed to slide rearward. The replacement of the battery BT50 is made easier by allowing the battery BT50 to slide.

In addition, the battery storage portion 550 of the present example is formed in a T-shape; however, a third battery storage portion extending in the width direction may be further provided in the passenger compartment, so that the battery storage portion 550 has an H-shape as a whole.

In addition, a cup holder, a storage box, an armrest, and the like may be provided on the upper surface 552a of the second battery storage portion 552. In addition, attachments 556 (mounting portions) for attaching various external devices such as a lighting device, a display device such as a display, and a table may be provided. In addition, an electric current may be allowed to flow through the external devices by connecting the external devices to the attachments 556. For example, even when a separate power supply is not connected to an electrical outlet, an electric current is allowed to flow through and light up the lighting device or the like by connecting the lighting device or the display device to the attachment 556.

Another arrangement inside the passenger compartment 502 will be described with reference to FIGS. 72 and 73. As shown in FIG. 72, a space can be formed at the rear of the passenger compartment 502 by folding the rear seats SR506 and sliding the rear seats SR506 forward. The expanded space may be used as a bedroom or a luggage storage place.

In addition, the expanded space may be used as a space such as a workplace by connecting the table 519 or the display device 536 to the second battery storage portion 552, and disposing a preferred chair 547 around the second battery storage portion 552. In addition, a hammock or the like may be hung from the roof 506, and the expanded space may be used as a bedroom.

### <Seventh example>

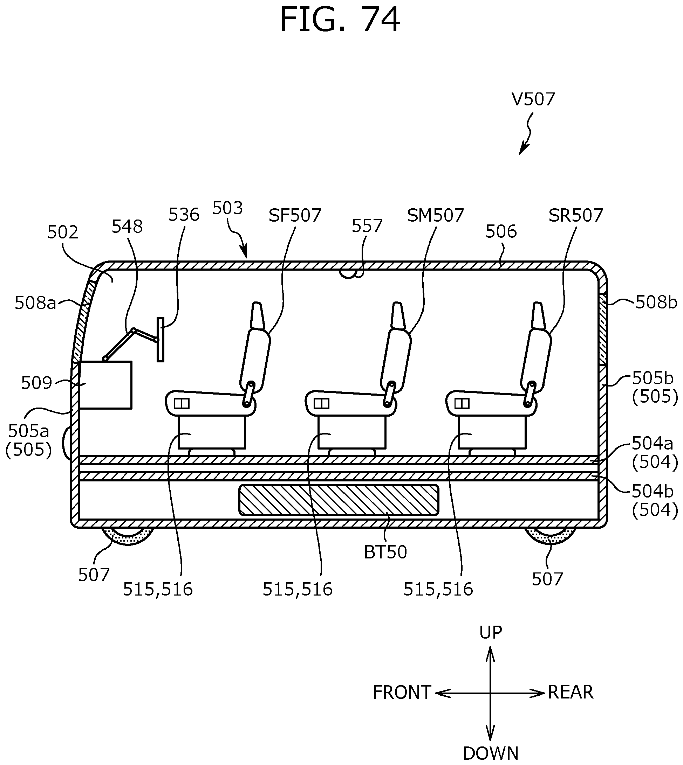

A vehicle V507 that is a seventh example of the present embodiment will be described with reference to FIG. 74. FIG. 74 is a view schematically showing a configuration of the vehicle V507, and is a view of the vehicle V507 when viewed from the side.

Conventionally, an instrument panel is provided with a display that displays map information from a car navigation system or the like. In the future, it is expected that a display with a larger screen or a plurality of displays are disposed to display more information. However, when the size of a display is increased or a plurality of displays are provided, portions of some displays are far away from a driver's seat or some displays are disposed far away therefrom, and therefore, simply tilting the display does not allow the hands or fingers of an occupant reach the display, and it is difficult for the occupant to perform a touch operation, which is a problem.

Therefore, in the vehicle V507, even when the display device 536 that is touch-operable is disposed at a position away from an operator, for example, on the instrument panel 509, the display device 536 is configured to be movable toward the seat of the operator and to be operable.

More specifically, as shown in FIG. 74, the instrument panel 509 capable of storing conveyance equipment is provided on the front side of the passenger compartment 502, and the display device 536 that is touch-operable is attached via an arm portion 548 that is extendable and retractable with respect to the instrument panel 509.

The instrument panel 509 corresponds to an equipment storage portion of the present invention, and the display device 536 corresponds to an image display device.

The display device 536 can be moved closer to the operator by the arm portion 548. For example, an occupant seated in a front seat SF507 that is a passenger seat or in a middle seat SM507 can touch-operate the display device 536.

Incidentally, a movable mechanism such as an actuator may be provided in the arm portion 548 to allow the display device 536 to automatically move closer to the operator. In this case, it is preferable that the display device 536 moves when the operator wishes to operate the display device 536. For example, when the driver presses an operating switch provided on a steering wheel, the display device 536 may be moved closer to the driver. In addition, an in-vehicle camera 557 may detect the line of sight or the like of the occupant H50, and when the occupant H50 looks at the display device 536, the display device 536 may be moved closer to the occupant H50. In addition, the voice of the occupant may be acquired by a microphone provided in the passenger compartment, and the display device 536 may be moved in response to an instruction by the voice of the occupant.

When the display device 536 is moved, the physique or position of the occupant may be detected by the in-vehicle camera 557 or a pressure sensor used in a seat belt reminder or the like, an appropriate position for operation may be obtained, and the display device 536 is moved to the position. Such calculation of the position of the display device 536 is performed by an ECU installed in the vehicle V507.

Incidentally, the display device 536 may be provided to be removable from the instrument panel 509, and the removed display device 536 may be wirelessly connected to the ECU in the vehicle and operable. In addition, the occupant H50 may use the display device 536 in the same manner as a tablet terminal.

In addition, a part or the entirety of the instrument panel 509 may be tilted for each display device 536 or the height of the instrument panel 509 may be changed, and the display device 536 may be configured to project toward the operator.

### <Eighth example>

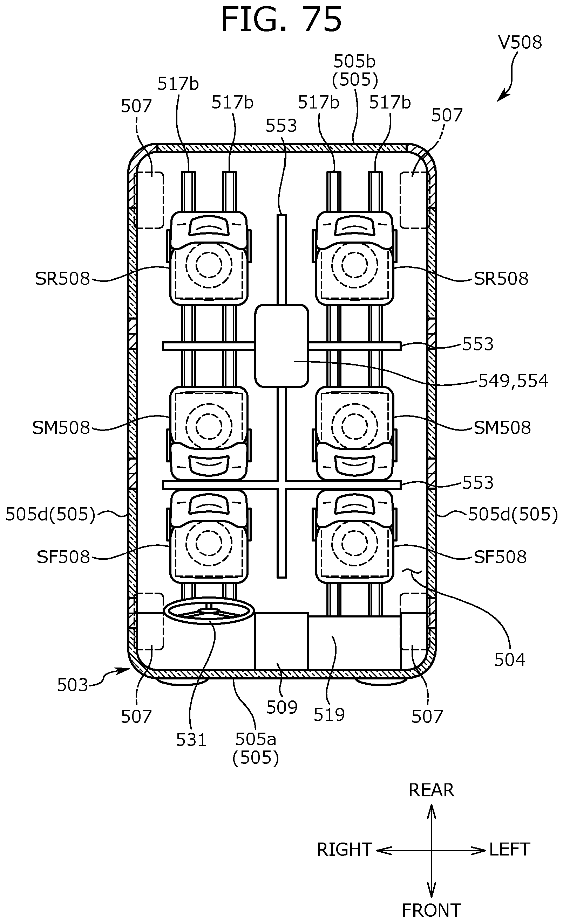

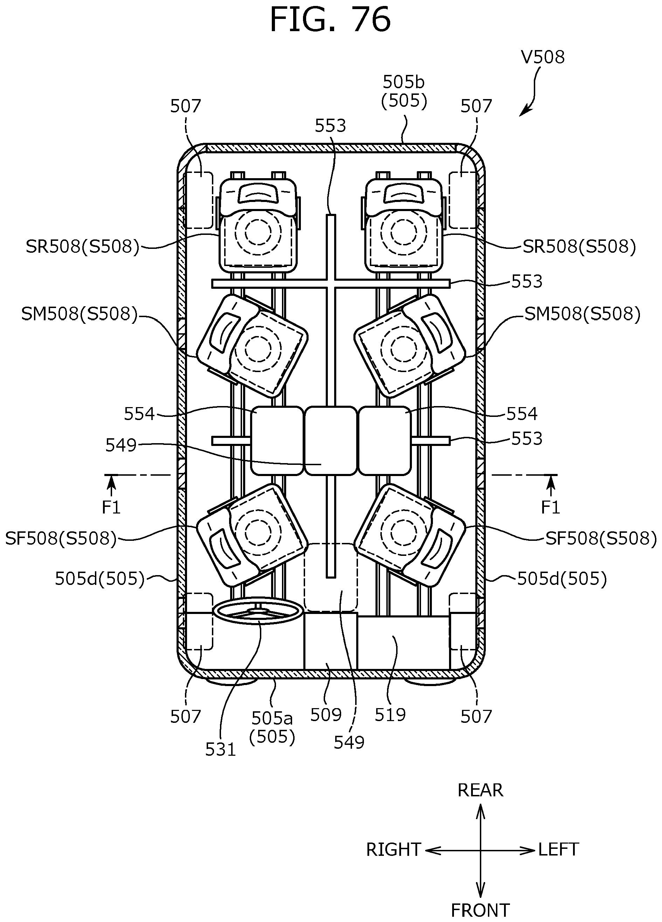

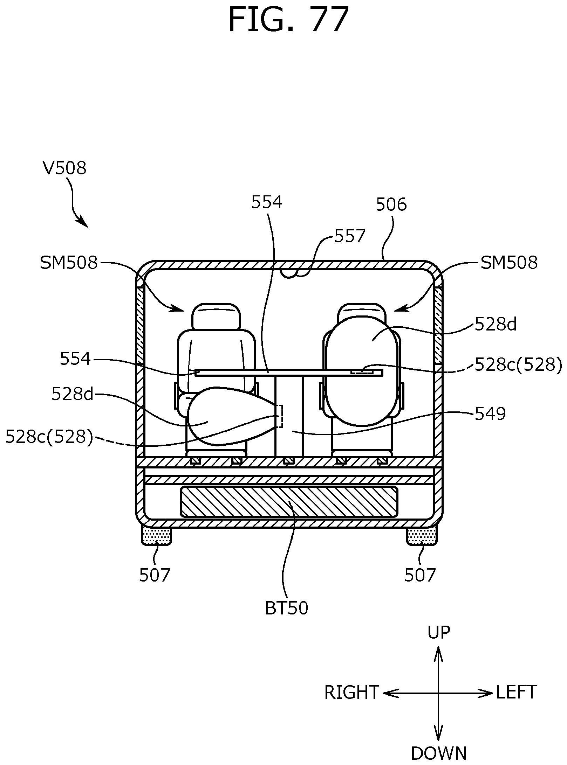

A vehicle V508 that is an eighth example of the present embodiment will be described with reference to FIGS. 75 to 77. FIG. 75 is a view schematically showing a configuration of the vehicle V508, and is a cross-sectional view of the vehicle V508 when viewed from above. FIG. 76 is a view showing an example of a seat arrangement of the vehicle V508, and is a cross-sectional view of the vehicle V508 when viewed from above. FIG. 77 is a cross-sectional view taken along line F1-F1 of FIG. 76, and is a view of the vehicle V508 when viewed from the front.

In a vehicle capable of autonomous driving, an occupant is freed from driving, and can freely spend her or his time at a preferred place in the vehicle. When the occupant performs a task or study in the vehicle, it is considered that having a large table similar to those used indoors is convenient and comfort is improved.

JP 2022-105207 A discloses a conveyance seat including a table provided on a back surface of the conveyance seat. However, since a table provided on a conventional conveyance seat is not designed for working (for example, studying or performing a task) in a vehicle, the table surface is small and it is difficult to work on the table, which is a problem. In addition, since the position of the table is fixed, the table can be used only at a designated seat, which is a problem.

The vehicle V508 that is the eighth example of the present embodiment includes the passenger compartment 502 (interior space) that the occupant H50 enters, and the wall portion 503 including the floor 504, the roof 506, and the side wall 505 that surround the passenger compartment 502. Furthermore, the vehicle V508 includes a table rail 553 provided on the floor 504, and a table 554 attached to be slidable on the table rail 553.

In the vehicle V508, a plurality of vehicle seats S508 (front seats SF508, middle seats SM508, and rear seats SR508) in which the occupants H50 are seated are provided and are slidable in the front to rear direction by seat rails (lower rails 517b) disposed on the floor surface. In addition, each of the vehicle seats S508 includes the reclining device 514, the height adjustment device 515, and the seat rotating device 516, and is configured such that the rearward tilt angle of the seat back or the height and orientation of the seat cushion can be changed.

As shown in FIG. 75, the table rail 553 is disposed to extend in the front to rear direction at a center of the vehicle V508 in the width direction. In addition, the table rail 553 is disposed to extend in the right to left direction (seat width direction) between the front seats SF508 and the middle seats SM508 and between the middle seats SM508 and the rear seats SR508. The table 554 is movable in the front to rear direction or the width direction along the table rail 553. Incidentally, the table 554 may be a center console 549.

When the occupant H50 wants to use the table 554 at the front seat SF508, the table 554 can be moved forward and used. In addition, when the occupant H50 wants to use the table 554 at the rear seat SR508, the table 554 is moved rearward and used.

Therefore, even when a table is not provided for each seat, the table 554 can be moved to a seat in which an occupant who wishes to use the table 554 is seated, and the table 554 can be used.

Conventionally, the need to provide a table on each seat is eliminated, so that unnecessary space can be eliminated and the weight can also be reduced. When the occupant H50 does not use the table 554, the table 554 may be disposed at a location that does not obstruct movement, for example, behind the rear seat SR508.

The table 554 may be detachably attached to the table rail 553. When the table 554 is not required, the table 554 may be removed from the table rail 553 and stored behind the rear seat SR508.

In addition, the table 554 may be detachable, and the detached table 554 may be attached to the instrument panel 509 or the door 505d. In addition, the table 554 may be attached to the seat rails (lower rails 517b) for moving the vehicle seat S508 forward and rearward, and may be slidably moved. In addition, the vehicle seat S508 may be removed from the seat rails, and the table 554 may be attached using the rails.

In addition, the table 554 may be attached to be storable in the instrument panel 509, and when in use, the occupant H50 may pull out the table 554 and attach the table 554 to the table rail 553 for use. In addition, the table 554 may be attached to be storable in the roof 506, and may be pulled out and used as necessary.

The table 554 that moves on the rail may move in response to the opening and closing of the door 505d. In addition, the table 554 may move in response to the movement of the vehicle seat S508.

In addition, the center console 549 provided with drink holders and the like may be made detachable, and the removed center console 549 may be used as the table 554 that moves on the rail.

In consideration of safety, it is preferable that the table 554 that is movable on the rail is configured to tip over in the event of a collision. In addition, as shown in FIG. 77, the airbag device 528c that is the occupant protection device 528 may be provided on the table 554, and a bag 528d of the airbag device 528c may be deployed in the event of a collision. In addition, when the vehicle seat S508 is rotated, it is preferable that the table 554 on the rail moves to a position where it is easy for the occupant H50 to use, according to the orientation of the seat.

In addition, as shown in FIG. 76, when the plurality of vehicle seats S508 rotate in the passenger compartment, it is preferable that the seats are controlled as follows. The following control is executed by, for example, a control unit (ECU) (not illustrated) installed in the vehicle V508 and connected to each of the vehicle seats S508.

First, biometric information is acquired by a biometric sensor (sensor unit) provided in the vehicle seat S508, and physique information such as the body type of the seated occupant, for example, the height of the occupant is estimated. The biometric sensor is, for example, a pressure sensor disposed on the surface of the seat cushion.

Next, the amounts of movement and the amounts of rotation of the plurality of vehicle seats S508 and the order of movements (operation order) of the vehicle seats S508 that prevent the feet of the occupants H50 from hitting each other when the vehicle seats S508 in the passenger compartment are rotated are automatically calculated.

When the body type of the occupant H50 is estimated, the body type may be estimated not only using the biometric sensor provided in the vehicle seat S508, but also using the in-vehicle camera 557 that captures an image of the interior of the passenger compartment.

In addition, information transmission means for providing rotation start information to the seated occupant H50 when the vehicle seat S508 rotates may be provided. As the information transmission means, for example, a light source located where the light source is visible to the occupant H50 may be caused to emit light. Alternatively, as the information transmission means, a vibration device provided in the vehicle seat S508 may be vibrated.

In addition, as the information transmission means, a speaker in the vehicle may be used to announce the start of rotation by voice, warning sound, or the like. For example, when the seat rotates, posture instruction means for instructing the occupant H50 to change the posture of the occupant H50 during operation of the seat may be provided, and the posture instruction means may notify the occupant H50 of a message such as "please raise the feet and stand by" by voice. Further, a similar message may be displayed on a display inside the vehicle.

In addition, when the vehicle seat S508 is rotationally moved, the table 554 or the center console 549 that is movably provided may be retracted to below the instrument panel 509 or the position of the rear seat SR508. Alternatively, an operation such as moving the table 554 or the center console 549 to the center of the passenger compartment 502 may be performed.

In addition, it is preferable that each of the vehicle seats S508 includes a distance sensor or an in-vehicle camera that measures a distance to the vehicle seat S508 adjacent thereto and a distance to the occupant. When the distance sensor or the in-vehicle camera 557 detects or predicts a danger such as the body of the occupant H50 being sandwiched between the vehicle seats S508 adjacent to each other during seat operation or movement of the vehicle seat, the movement of the vehicle seat S508 may be automatically stopped.

In addition, the seat operation and the rotation of the vehicle seat S508 may be performed in a state where the door 505d of the vehicle V508, for example, a power sliding door is opened.

### <Table occupant protection device>

As shown in FIG. 76, when a plurality of the vehicle seats in the passenger compartment face the center of the passenger compartment, a center console or a table may be disposed in the center of the passenger compartment. In such a state, even when occupant protection devices such as airbag devices are provided in the vehicle seats, sufficient protection may not be able to be provided.

Therefore, in the vehicle V508 of the present example, as shown in FIG. 76, the center console 549 or the table 554 disposed at the center of the passenger compartment is provided with the airbag device 528c that can be deployed outward. The airbag device 528c includes an airbag module including the bag 528d, an inflator, and the like. As shown in FIG. 77, it is preferable that the airbag device 528c is provided in a side wall portion of the center console 549, the side wall portion extending in the up to down direction.

In addition, when the table 554 extending outward from the center console 549 in a horizontal direction is provided, it is preferable that the airbag device 528c is provided at an outer end portion of the table 554.

A plurality of the airbag devices 528c may be disposed on the table 554, and when the bags 528d of the airbags are deployed, the bags 528d may surround the entire periphery of the table 554.

In addition, the airbag device 528c that is deployed downward may be installed on the roof 506 of the vehicle V508, and the bag 528d may be deployed to wrap the table 554 from above.

The conveyance according to the present embodiment has been described above with reference to the drawings. Incidentally, in the vehicles shown in the first to third embodiments, the seat backs are attached to the storage portions provided in any one of the mattress, the roof, and the side wall portion; however, the positions where the seat backs can be stored may be a selection and combination of the mattress, the roof, and the side wall portion. For example, the seat back of the front seat may be stored in the storage portion of the mattress, and the seat back of the rear seat may be stored in the storage portion of the roof. In addition, the support surface adjustment device or the occupant protection device installed in the vehicle V502 of the second example can be applied to the vehicle V501 of the first example or the vehicles of the other examples.

### REFERENCE SIGNS LIST

### <First embodiment>

- S:: vehicle seat
- P:: pad material
- T:: skin material
- BN1:: spine
- BN2:: pelvis
- MT:: mobile terminal
- AM:: airbag module
- 1:: seat cushion
- 2:: seat back
- 3:: headrest
- 4:: slide rail
- 10:: posture improvement assistance device
- 11:: air cell (movable body)
- 12:: shoulder air cell
- 12R:: right shoulder air cell
- 12L:: left shoulder air cell
- 13:: lumbar air cell
- 13U:: first lumbar air cell
- 13D:: second lumbar air cell
- 14:: pelvis air cell
- 15:: pressure sensor
- 16:: shoulder pressure sensor
- 16R:: right shoulder pressure sensor
- 16L:: left shoulder pressure sensor
- 17:: lumbar pressure sensor
- 17U:: first lumbar pressure sensor
- 17D:: second lumbar pressure sensor
- 18:: pelvis pressure sensor
- 20:: air supply pump
- 21:: valve unit
- 30:: control unit
- 31:: signal acquisition unit (acquisition unit)
- 32:: LC calculation unit (calculation unit)
- 33:: posture determination unit (determination unit)
- 34:: air cell control unit (movable body control unit)
- 35:: setting reception unit
- 36:: output control unit
- 40:: posture display screen
- 41:: posture display region
- 42:: schematic human body diagram
- 43:: posture specifying display
- 44:: lumbar curve value display
- 45:: posture improvement assistance information
- 46:: graph display region
- 47:: time-series graph
- 48:: history display region
- 49:: daily history information
- 50:: kneading execution screen
- 51:: kneading position display region
- 52:: schematic kneading diagram
- 53:: kneading auxiliary information
- 54:: kneading state display region
- 56:: mode selection button
- 57:: display switching button
- 100:: back frame
- 110:: side frame
- 111:: side frame plate
- 120:: upper frame
- 121:: horizontal portion
- 122:: vertical portion
- 123:: pillar support portion
- 130:: lower frame
- 140:: pressure-receiving member
- 141:: pressure-receiving portion
- 141A:: center recess
- 141B:: right recess
- 141C:: left recess
- 142:: support portion
- 143:: coupling wire
- 143A:: upper coupling wire
- 143B:: lower coupling wire
- 144:: bead portion
- 144A:: first bead portion
- 144B:: second bead portion
- 144C:: third bead portion
- 145:: engaging pawl
- 200:: back frame
- 210:: side frame
- 220:: upper frame
- 240:: pressure-receiving member
- 243:: coupling wire
- 243A:: upper coupling wire
- 243B:: lower coupling wire
- 244:: engaging pawl
- 245:: air cell fastener
- 246:: tube
- 300:: back frame
- 310:: side frame
- 311:: first side frame piece
- 312:: second side frame piece
- 312a:: uneven portion
- 312b:: flat portion
- 320:: upper frame
- 321:: upper wall portion
- 321a:: upper through-hole
- 321b:: upper reinforcing portion
- 321c:: upper bent portion
- 322:: lower wall portion
- 322a:: lower through-hole
- 322b:: lower reinforcing portion
- 322c:: lower bent portion
- 323:: front wall portion
- 323a:: center portion
- 324:: pillar guide
- 324a:: support hole
- 325:: headrest pillar

### <Second embodiment>

- 401:: vehicle
- 402:: vehicle door (opening/closing body)
- 402F:: front door
- 402R:: rear door
402a: door body portion
402b: door upper portion
402ba: inner end portion
402c: window portion
402d: sunroof
402e: door pocket
402f: lower end
- 403:: roof
403a: sunroof
403b: roof airbag device
- 404:: floor
- 405:: battery
- 406:: tire
- 407:: operating system box
407a: steering wheel
- 408:: window
408a: front window
408b: rear window
408c: side window
- 410, 410', 410'':: vehicle seat (seat)
- 410F:: front seat
- 410M:: middle seat
- 410R:: rear seat
- 411:: seat cushion
411a: bolster portion
- 412:: seat back
412a: bolster portion
412b: armrest
- 413:: headrest
413a: inner end portion
- 414:: reclining device
- 415:: height adjustment device
- 416:: seat rotating device
- 417:: seat rail
- 418, 418A:: side support (occupant support member)
418La, 418Ra: cushion air cell
418Lb, 418Rb: back air cell
418Lc, 418Rc: armrest air cell
- 419:: speaker
- 420, 420A, 420B:: instrument panel
- 421, 421B:: panel body
- 422, 422B:: pull-out portion
- 423:: airbag device
- 424:: storage part
- 425:: storage type table
- 427:: footrest portion
427a: upper rotating shaft
427b: lower rotating shaft
- 428:: recess
- 430:: center console
- 431:: recess
- 432:: odor sensor

### <Third embodiment>

V501, V501A to V501C, V502, V502B, V503, V504, V504A, V504A',
V505, V505A, V506 to V508: vehicles (conveyances)
S50, S50A, S505: vehicle seat
SF50: front seat
SM50: middle seat
SR50: rear seat
BT50, BT501, BT502: battery
M50: motor
H50: occupant
P50: pad
T50: skin
502: passenger compartment (interior space)
503: wall portion
504, 504A, 504G: floor
   504a, 504Aa: upper floor
   504b, 504Ab: lower floor
   504c: air layer
   504d: battery recess
505: side wall
   505a: front portion
   505b: tailgate
   505c: pillar
   505d: door
506: roof (upper wall)
   506a: sunroof
507: tire
508: window
   508a: front window
   508b: rear window
509: instrument panel (equipment storage portion)
510, 510A, 510B, 510B', 510C: mattress
   510a, 510Aa, 510Ba: support surface
   510Ab: floor clearance portion
511, 511A, 511D: seat cushion
512, 512B, 512B', 512C, 512D, 512E: seat back
   512a, 512Ba: support surface
   512b: back support member
   512Bc: through-hole
   512d: extension portion
513: headrest
   513a: through-hole
514: reclining device
515: height adjustment device
516: seat rotating device
517: rail device
   517a: upper rail
   517b: lower rail
   517c: side rail
518: occupant support member
519: table
520, 520B, 520B', 520C, 520D: storage portion
521, 521A, 521B, 521B', 521C, 521D: storage recess
522: coupling portion
   522a: rotating shaft
   522b: lock device
523, 523': foot recess
   523a to 523h: first recess to eighth recess
524: filling member
525: heater
526: notification device
527: support surface adjustment device
528: occupant protection device
   528a: side airbag
   528b: belt extraction portion
   528c: airbag device
   528d: bag
529: frame
530: ottoman
531: steering wheel
532: battery support wall
534: ventilation hole
535: gap
536: display device
537: end portion support member
538: armrest
539: movable member
540: coupling member
541: wire-shaped member
542: net-shaped member
543: planar member
544: attachment member
545: luggage
546: luggage holding member
547: chair
548: arm portion
549: center console
550: battery storage portion
551: first battery storage portion
552: second battery storage portion
553: table rail
554: table
555: motor compartment
556: attachment
557: in-vehicle camera

## Claims

1. A control unit (30) that determines a posture of a seated occupant seated in a chair (S), and that controls a display device (MT) to output a determination result, comprising:
an acquisition unit (31) that acquires pressure signals output by pressure sensors (15) provided in a plurality of respective movable bodies (11) built into the chair (S);
a calculation unit (32) that calculates a lumbar curve value, which is an index related to the posture of the seated occupant, by inputting a plurality of the pressure signals acquired by the acquisition unit (31) into a predetermined calculation equation;
a determination unit (33) that determines the posture of the seated occupant based on the lumbar curve value calculated by the calculation unit (32); and
an output control unit (36) that controls the display device (MT) to output a schematic human body diagram corresponding to a determination result of the determination unit (33), **characterized in that**
the control unit (30) further comprises a movable body control unit (34) that controls the movable bodies (11) to be displaced,
the calculation unit (32) calculates a lumbar curve value variation amount that is a statistical variation amount of the calculated lumbar curve value,
the determination unit (33) determines a lower back pain level of the seated occupant based on the lumbar curve value variation amount calculated by the calculation unit (32), and
the movable body control unit (34) controls the movable bodies (11) to be periodically displaced based on a determination result of the lower back pain level by the determination unit (33).

2. The control unit (30) according to claim 1,
wherein the determination unit (33) determines whether the posture of the seated occupant is at least one of a hunched posture, a standard posture, and an arched posture, based on the lumbar curve value, and
the output control unit (36) controls the display device (MT) to output a text indicating whether the posture of the seated occupant is the hunched posture, the standard posture, or the arched posture, together with the schematic human body diagram.

3. The control unit (30) according to claim 1,
wherein the output control unit (36) controls the display device (MT) to output a time-series graph showing a change over time in the lumbar curve value.

4. The control unit (30) according to claim 1, further comprising:
a setting reception unit that receives an input of a set value related to a physique of the seated occupant,
wherein the calculation unit (32) calculates the lumbar curve value by inputting the pressure signals and the set value into the predetermined calculation equation.

5. A posture improvement assistance (10) device, comprising:
the control unit (30) according to any one of claims 1 to 4; and
the chair (S) into which the plurality of movable bodies (11) provided with the pressure sensors (15) are built.

6. A control method for determining a posture of a seated occupant seated in a chair (S) and controlling a display device (MT) to output a determination result, the control method comprising a computer executing:
an acquisition step (S20) of acquiring pressure signals output by pressure sensors (15) provided in a plurality of respective movable bodies (11) built into the chair (S);
a calculation step (S21) of calculating a lumbar curve value that is an index related to the posture of the seated occupant, by inputting a plurality of the pressure signals acquired in the acquisition step into a predetermined calculation equation;
a determination step (S30) of determining the posture of the seated occupant based on the lumbar curve value calculated in the calculation step; and
an output control step (S31) of controlling the display device (MT) to output a schematic human body diagram corresponding to a determination result of the determination step, **characterized in that**
the control method further comprises a step (S13) of controlling the movable bodies (11) to be displaced,
wherein the movable bodies (11) are controlled to be displaced for correcting the posture of the seated occupant.

## Patentansprüche

1. Eine Steuereinheit (30), die die Körperhaltung eines auf einem Stuhl (S) sitzenden Insassen bestimmt und eine Anzeigevorrichtung (MT) so steuert, dass diese ein Bestimmungsergebnis ausgibt, mit:
einer Erfassungseinheit (31), die Drucksignale erfasst, die von Drucksensoren (15) ausgegeben werden, die in mehreren jeweiligen beweglichen Körpern (11) vorgesehen sind, die in den Stuhl (S) eingebaut sind;
einer Berechnungseinheit (32), die einen Lendenkurvenwert, bei dem es sich um einen Index handelt, der mit der Körperhaltung des sitzenden Insassen in Zusammenhang steht, berechnet, indem sie mehrere der von der Erfassungseinheit (31) erfassten Drucksignale in eine vorbestimmte Berechnungsgleichung einspeist;
einer Bestimmungseinheit (33), die die Körperhaltung des sitzenden Insassen auf der Grundlage des von der Berechnungseinheit (32) berechneten Lendenkurvenwerts bestimmt; und
einer Ausgabesteuereinheit (36), die die Anzeigevorrichtung (MT) so steuert, dass diese ein schematisches Diagramm eines menschlichen Körpers ausgibt, das einem Bestimmungsergebnis der Bestimmungseinheit (33) entspricht,
**dadurch gekennzeichnet, dass**
die Steuereinheit (30) ferner eine Steuereinheit für bewegliche Körper (34) umfasst, die die beweglichen Körper (11) so steuert, dass sie verschoben werden,
die Berechnungseinheit (32) einen Schwankungsbetrag des Lendenkurvenwerts berechnet, bei dem es sich um einen statistischen Schwankungsbetrag des berechneten Lendenkurvenwerts handelt,
die Bestimmungseinheit (33) einen Grad der Schmerzen im unteren Rücken des sitzenden Insassen auf der Grundlage des von der Berechnungseinheit (32) berechneten Schwankungsbetrags des Lendenkurvenwerts bestimmt, und
die Steuereinheit für bewegliche Körper (34) die beweglichen Körper (11) so steuert, dass sie auf der Grundlage eines von der Bestimmungseinheit (33) bestimmten Grads der Schmerzen im unteren Rücken periodisch verschoben werden.

2. Die Steuereinheit (30) nach Anspruch 1,
wobei die Bestimmungseinheit (33) auf der Grundlage des Lendenkurvenwerts bestimmt, ob die Körperhaltung des sitzenden Insassen mindestens eine von einer gekrümmten Haltung, einer Standardhaltung und einer gewölbten Haltung ist, und
die Ausgabesteuereinheit (36) die Anzeigevorrichtung (MT) so steuert, dass sie einen Text zusammen mit dem schematischen Diagramm eines menschlichen Körpers ausgibt, der angibt, ob die Körperhaltung des sitzenden Insassen die gekrümmte Haltung, die Standardhaltung oder die gewölbte Haltung ist.

3. Die Steuereinheit (30) nach Anspruch 1,
wobei die Ausgabesteuereinheit (36) die Anzeigevorrichtung (MT) so steuert, dass diese ein Zeitreihendiagramm ausgibt, das eine zeitliche Veränderung des Lendenkurvenwerts zeigt.

4. Die Steuereinheit (30) nach Anspruch 1, ferner umfassend:
eine Einstellungsempfangseinheit, die eine Eingabe eines Einstellwerts empfängt, der sich auf den Körperbau des sitzenden Insassen bezieht,
wobei die Berechnungseinheit (32) den Lendenkurvenwert berechnet, indem sie die Drucksignale und den Einstellwert in die vorbestimmte Berechnungsgleichung einspeist.

5. Eine Haltungsverbesserungsunterstützungsvorrichtung (10), umfassend:
die Steuereinheit (30) nach einem der Ansprüche 1 bis 4; und
den Stuhl (S), in den die mehreren beweglichen Körper (11), die mit den Drucksensoren (15) versehen sind, eingebaut sind.

6. Ein Steuerungsverfahren zum Bestimmen einer Körperhaltung eines in einem Stuhl (S) sitzenden Insassen und zum Steuern einer Anzeigevorrichtung (MT), um ein Bestimmungsergebnis auszugeben, wobei das Steuerungsverfahren die Ausführung folgender Schritte durch einen Computer umfasst:
einen Erfassungsschritt (S20) zum Erfassen von Drucksignalen, die von Drucksensoren (15) ausgegeben werden, die in mehreren jeweiligen beweglichen Körpern (11) vorgesehen sind, die in den Stuhl (S) eingebaut sind;
einen Berechnungsschritt (S21) zum Berechnen eines Lendenkurvenwerts, der ein Index in Bezug auf die Körperhaltung des sitzenden Insassen ist, durch Eingabe einer Vielzahl der im Erfassungsschritt erfassten Drucksignale in eine vorbestimmte Berechnungsgleichung;
einen Bestimmungsschritt (S30) zum Bestimmen der Körperhaltung des sitzenden Insassen auf der Grundlage des im Berechnungsschritt berechneten Lendenkurvenwerts; und
einen Ausgabesteuerungsschritt (S31) zum Steuern der Anzeigevorrichtung (MT), um ein schematisches Diagramm eines menschlichen Körpers auszugeben, das einem Bestimmungsergebnis des Bestimmungsschritts entspricht,
**dadurch gekennzeichnet, dass**
das Steuerungsverfahren ferner einen Schritt (S13) zum Steuern der beweglichen Körper (11) umfasst, so dass diese verschoben werden,
wobei die beweglichen Körper (11) so gesteuert werden, dass sie verschoben werden, um die Körperhaltung des sitzenden Insassen zu korrigieren.

## Revendications

1. Unité de commande (30) qui détermine la posture d'un occupant assis sur une chaise (S) et qui commande un dispositif d'affichage (MT) de manière à délivrer en sortie un résultat de détermination, comprenant :
une unité d'acquisition (31) qui acquiert des signaux de pression délivrés en sortie par des capteurs de pression (15) prévus dans une pluralité de corps mobiles respectifs (11) intégrés à la chaise (S) ;
une unité de calcul (32) qui calcule une valeur de courbure lombaire, qui est un indice lié à la posture de l'occupant assis, en introduisant une pluralité des signaux de pression acquis par l'unité d'acquisition (31) dans une équation de calcul prédéterminée ;
une unité de détermination (33) qui détermine la posture de l'occupant assis sur la base de la valeur de courbure lombaire calculée par l'unité de calcul (32) ; et
une unité de commande de sortie (36) qui commande le dispositif d'affichage (MT) de manière à délivrer en sortie un schéma du corps humain correspondant à un résultat de détermination de l'unité de détermination (33), **caractérisée en ce que**
l'unité de commande (30) comprend en outre une unité de commande de corps mobiles (34) qui commande les corps mobiles (11) de manière à les déplacer,
l'unité de calcul (32) calcule une quantité de variation de valeur de courbure lombaire qui est une quantité de variation statistique de la valeur de courbure lombaire calculée,
l'unité de détermination (33) détermine un niveau de douleur dans le bas du dos de l'occupant assis sur la base de la quantité de variation de valeur de courbure lombaire calculée par l'unité de calcul (32), et
l'unité de commande de corps mobiles (34) commande les corps mobiles (11) de manière à les déplacer périodiquement sur la base d'un résultat de détermination du niveau de douleur dans le bas du dos par l'unité de détermination (33).

2. Unité de commande (30) selon la revendication 1,
dans laquelle l'unité de détermination (33) détermine si la posture de l'occupant assis est au moins l'une parmi une posture voûtée, une posture standard et une posture cambrée, sur la base de la valeur de courbure lombaire, et
l'unité de commande de sortie (36) commande le dispositif d'affichage (MT) de manière à délivrer en sortie un texte indiquant si la posture de l'occupant assis est la posture voûtée, la posture standard ou la posture cambrée, en même temps que le schéma du corps humain.

3. Unité de commande (30) selon la revendication 1,
dans laquelle l'unité de commande de sortie (36) commande le dispositif d'affichage (MT) de manière à délivrer en sortie un graphique chronologique montrant l'évolution dans le temps de la valeur de courbure lombaire.

4. Unité de commande (30) selon la revendication 1, comprenant en outre :
une unité de réception de réglage qui reçoit une entrée d'une valeur de consigne liée à la morphologie de l'occupant assis,
dans laquelle l'unité de calcul (32) calcule la valeur de courbure lombaire en introduisant les signaux de pression et la valeur de consigne dans l'équation de calcul prédéterminée.

5. Dispositif d'aide à l'amélioration de posture (10), comprenant :
l'unité de commande (30) selon l'une quelconque des revendications 1 à 4 ; et
le siège (S) dans lequel sont intégrés la pluralité de corps mobiles (11) pourvus des capteurs de pression (15).

6. Procédé de commande pour déterminer la posture d'un occupant assis sur une chainse (S) et commander un dispositif d'affichage (MT) de manière à délivrer en sortie un résultat de détermination, le procédé de commande comprenant un ordinateur exécutant :
une étape d'acquisition (S20) consistant à acquérir des signaux de pression délivrés en sortie par des capteurs de pression (15) prévus dans une pluralité de corps mobiles respectifs (11) intégrés à la chaise (S) ;
une étape de calcul (S21) consistant à calculer une valeur de courbure lombaire qui est un indice lié à la posture de l'occupant assis, en introduisant une pluralité des signaux de pression acquis lors de l'étape d'acquisition dans une équation de calcul prédéterminée ;
une étape de détermination (S30) consistant à déterminer la posture de l'occupant assis sur la base de la valeur de courbure lombaire calculée lors de l'étape de calcul ; et
une étape de commande de sortie (S31) consistant à commander le dispositif d'affichage (MT) de manière à délivrer en sortie un schéma du corps humain correspondant à un résultat de détermination de l'étape de détermination, **caractérisé en ce que**
le procédé de commande comprend en outre une étape (S13) consistant à commander les corps mobiles (11) de manière à les déplacer,
dans lequel les corps mobiles (11) sont commandés de manière à les déplacer afin de corriger la posture de l'occupant assis.
